(19) Europäisches Patentamt
European Patent Office
Office européen des brevets

(11) Publication number: **0 320 984 A2**

(12) **EUROPEAN PATENT APPLICATION**

(21) Application number: 88121142.9

(22) Date of filing: 16.12.88

(51) Int. Cl.4: C07D 401/12 , C07D 401/14 , A61K 31/47 , A61K 31/44 , C07D 405/14 , C07D 409/14 , C07D 413/14

(30) Priority: 18.12.87 JP 318680/87
07.06.88 JP 138355/88

(43) Date of publication of application: 21.06.89 Bulletin 89/25

(84) Designated Contracting States: CH DE FR GB IT LI

(71) Applicant: FUJIREBIO INC.
6-7, Shimoochiai 4-chome Shinjuku-ku
Tokyo 161(JP)

(72) Inventor: Ikawa, Hiroshi
1-26-10 Miyasaka
Setagaya-ku Tokyo(JP)
Inventor: Kadoiri, Akiyoshi
1458-5-303 Hazama-cho
Hachiouji-shi Tokyo(JP)
Inventor: Kobayashi, Nobuo
1228 Kotta
Tama-shi Tokyo(JP)
Inventor: Yamaura, Tetsuaki
2-17-8 Niiza
Niiza-shi Saitama-ken(JP)
Inventor: Kase, Noriko
3-19-2 Nukuiminami-cho
Koganei-shi Tokyo(JP)
Inventor: Kita, Hiromi
1-15-9-303 Yamato-cho
Nakano-ku Tokyo(JP)

(74) Representative: Dipl.-Ing. Schwabe, Dr. Dr. Sandmair, Dr. Marx
Stuntzstrasse 16
D-8000 München 80(DE)

(54) **1,4-Dihydropyridine derivatives.**

(57) 1,4-Dihydropyridine derivatives of the formula (I) having vasodilative activity and inhibitory activity on platelet aggregation:

EP 0 320 984 A2

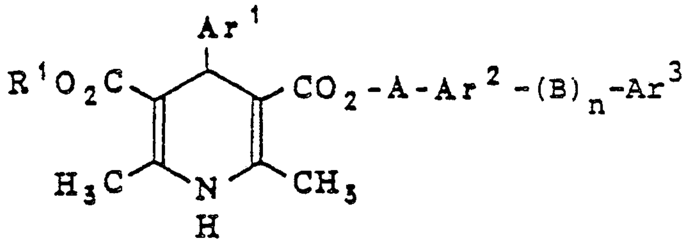

... (I)

wherein $Ar^1$ represents an aromatic hydrocarbon group or heterocyclic group which may have at least one substituent; $R^1$ represents a hydrocarbon group which may have at least one substituent; A and B each represent a hydrocarbon group which may have at least one substituent, in which any of the carbon atoms in the main chain thereof may be replaced by a nitrogen atom or an oxygen atom; $Ar^2$ represents an aromatic hydrocarbon group or heterocyclic group; $Ar^3$ represents a heterocyclic group which may have at least one substituent; and n is 0 or 1.

# 1,4-DIHYDROPYRIDINE DERIVATIVES

## BACKGROUND OF THE INVENTION

The present invention relates to 1,4-dihydropyridine derivatives having superior vasodilative and platelet aggregation inhibiting activities, which are represented by the following general formula:

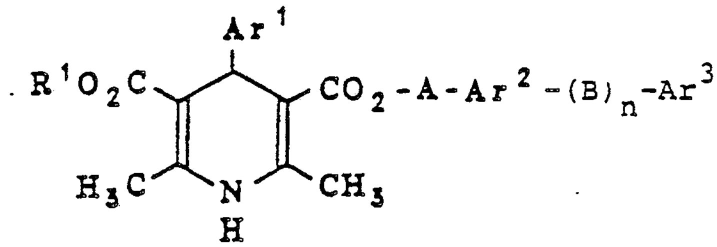

... (I)

Many derivatives of 1,4-dihydropyridine are known, which are remedies for diseases of circulatory system such as remedies for ischemic heart disease, cerebral circulatory disease and hypertension. For example, 1,4-dihydro-2,6-dimethyl-4-(2-nitrophenyl)pyridine-3,5-dicarboxylic acid dimethyl ester (U.S Patent No. 3,644,627; hereinafter referred to as "NIFEDIPINE") and 1,4-dihydro-2,6-dimethyl-4-(3-nitrophenyl)-pyridine-3,5-dicarboxylic acid-3-[2-(N-benzyl-N-methylamino)ethyl]ester-5-methyl ester hydrochloride (Japanese Patent Application Publication No. 45075/1980; hereinafter referred to as "NICARDIPINE") have already been utilized as hypotensor and coronary vasodilator. Moreover, other derivatives of 1,4-dihydropyridine having vasodilative activity together with inhibitory activity on platelet aggregation have been disclosed in Japanese Patent Application as listed below: Japanese Patent Application Laid-Open Nos. 140989/1981, 215684/1985, 226876/1985, 5076/1986, 10576/1986, 47477/1986, 197578/1986, 212581/1986, 148481/1987 and 187468/1987.

## SUMMARY OF THE INVENTION

It is an object of the present invention to provide medicines having higher inhibitory activity on platelet aggregation together with vasodilative activity.

Another object of the present invention is to provide medicines for vasodilation and inhibition of platelet aggregation, which have a low toxicity and do not exhibit significant side effects.

These objects of the present invention can be achieved by 1,4-dihydropyridine derivatives having the following general formula (I):

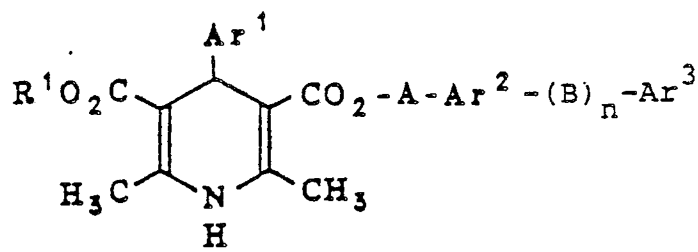

... (I)

wherein $Ar^1$ represents an aromatic hydrocarbon group or heterocyclic group which may have one or more substituents; $R^1$ represents a hydrocarbon group which may have one or more substituents; A and B each represent a hydrocarbon group which may have one or more substituents, in which any of the carbon atoms in the main chain thereof may be replaced by a nitrogen atom or an oxygen atom; $Ar^2$ represents an aromatic hydrocarbon group; $Ar^3$ represents a heterocyclic group which may have one or more substituents; and n is 0 or 1.

DETAILED DESCRIPTION OF THE PREFERRED EMBODIMENTS

In the 1,4-dihydropyridine derivatives having the above general formula (I), $Ar^1$ may be an aromatic hydrocarbon group which is selected from the group consisting of a phenyl group and a naphthyl group. Of these groups, a phenyl group is preferable as the aromatic hydrocarbon group represented by $Ar^1$.

Furthermore, $Ar^1$ may be a heterocyclic group selected from the group consisting of a pyridyl group, a benzoxazolyl group, a quinolyl group, a furyl group, a thienyl group, an isoquinolyl group, a benzthiazolyl, a benzoxadiazolyl and a benzthiadiazolyl. Of these groups, a pyridyl group, a benzoxazolyl group, a quinolyl group, a furyl group, and a thienyl group are preferable as the heterocyclic group represented by $Ar^1$.

The aromatic hydrocarbon groups or heterocyclic groups represented by $Ar^1$ may have one or more substituents selected from the group consisting of a nitro group, a trihalomethyl group such as trifluoromethyl and trichloromethyl, a cyano group, a halogen such as fluorine, chlorine, bromine and iodine, a phenoxy group, an amido group, a lower alkoxyl group having 1 to 4 carbon atoms, a lower alkyl group having 1 to 4 carbon atoms, a lower alkylthio group, a lower alkoxycarbonyl group, and a lower alkylcarbonyl group. Of these substituents, a nitro group, a trihalomethyl group such as trifluoromethyl and trichloromethyl, a cyano group, a halogen such as fluorine, chlorine, bromine and iodine, a phenoxy group are preferable as the substituents of the aromatic hydrocarbon groups or heterocyclic groups represented by $Ar^1$.

Specific preferable examples of the aromatic hydrocarbon group or heterocyclic group represented by $Ar^1$ are 2-nitrophenyl group, 3-nitrophenyl group, 4-nitrophenyl group, 2-cyanophenyl group, 2,3-dichlorophenyl group, 2-furyl group, 2-thienyl group, 3-thienyl group, 1-naphthyl group, 4-quinolyl group, 3-azidophenyl group, 3-trifluoromethylphenyl group, 4-methoxyphenyl group, 4-methylthiophenyl group, 3-pyridyl group, 4-methylphenyl and 3-trichloromethylphenyl group.

$R^1$ is a straight chain, branched chain or cyclic, saturated or unsaturated hydrocarbon group having 1 to 6 carbon atoms, which may contain oxygen, sulfur or nitrogen therein, and further may have a substituent such as a halogen, a cyano group, a nitro group, a nitrato group, a hydroxyl group, a phenoxy group, a disubstituted amino group, a phenylthio group, a piperidino group, a pyrrolidino group, a substituted piperazinyl group, a morpholino group and a phenyl. The phenyl substituent may further have one or two substituents selected from the group consisting of a halogen, a cyano group, a trihalomethyl group, an azido group, a disubstituted amino group, a lower alkyl group having 1 to 4 carbon atoms, a lower alkoxy group having 1 to 4 carbon atoms, a lower alkylthio group, a lower alkylcarbonyl group, a lower alkoxycarbonyl group, a carbamoyl group, a halomethyl group and a hydroxymethyl group. As the hydrocarbon group containing oxygen represented by $R^1$, an alkoxyalkyl group is preferable, and as the substituent of the hydrocarbon group represented by $R^1$, a disubstituted amino group is preferable for use in the present invention.

The nitrogen atom contained in the hydrocarbon group represented by $R^1$ may link with a group selected from the group consisting of a lower alkyl having 1 to 3 carbon atoms, an aryl group or an aralkyl group.

Further, $R^1$ may be a group represented by $-A-Ar^2-(B)n-Ar^3$, in which A, $Ar^2$, $Ar^3$, and n are respectively the same as those defined previously in the general formula of the 1,4-dihydropyridine derivatives.

Preferred examples of the group represented by $R^1$ are a methyl group, an ethyl group, a propyl group, a n-butyl group, a n-pentyl group, a n-hexyl group, an isopropyl group, an isobutyl group, a cyclopentyl group, a cyclohexyl group, a propenyl group, a 2-butenyl group, a 3-butenyl group, a 2-pentynyl group, a 2,4-hexadienyl group, a 2,4-hexadiynyl group, a hexa-2-yn-4-ene group and a hexa-2-en-4-yne group.

Preferred examples of the group represented by $R^1$ containing therein an oxygen atom, a sulfur atom or a nitrogen atom are a methoxyethyl group, an ethoxyethyl group, a methoxypropyl group, a ethoxypropyl group, a methylthioethyl group, an ethylthioethyl group, a methylthiopropyl group, an ethylthiopropyl group, a phenylthioethyl group, a 2-(N-methylamino)ethyl group, a 2-(N,N-dimethylamino)ethyl group, a 2-(N-phenyl-N-methylamino)ethyl group, a 2-(N-benzyl-N-methylamino)ethyl group, a 2-(4-phenylpiperazin-1-yl)-ethyl group, a 2-{4-(diphenylmethyl)-piperazinyl}ethyl group, a 2-morpholinoethyl group, a N-benzylpyrrolidin-3-yl group and an N-benzylpiperidin-3-yl group.

A represents a straight chain or branched chain unsaturated hydrocarbon group having 3 to 12 carbon atoms, or a cyclic, unsaturated hydrocarbon group having 4 to 12 carbon atoms, in which unsaturated hydrocarbon groups, at least an unsaturated bond thereof is conjugated with the aromatic hydrocarbon or heterocyclic group represented by $Ar^2$. The hydocarbon groups represented by $Ar^2$ may have a substituent selected from the group consisting of a halogen, a phenyl group, a pyridyl group, a thienyl group, a furyl group, a cyano group, an alkylcarbonyl group and an alkoxycarbonyl group, and may contain in the chain thereof a group selected from the group consisting of -O-N=CH-, -CH=N-O-, -N=N-, -N=CH- and

-CH = N-.

Preferred examples of the group represented by A are as follows:

$-CH_2-CH{=}CH-$ , $-CH_2\text{(}CH{=}CH\text{)}_2$ ,

$-CH_2-C{\equiv}C-$ , $-CH_2\text{(}C{\equiv}C\text{)}_2$ , $-CH_2\text{(}CH{=}CH\text{)}_3$ ,

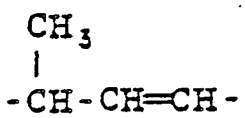 , 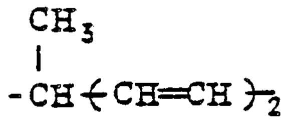 , 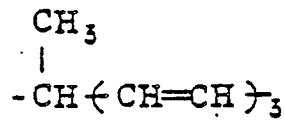 ,

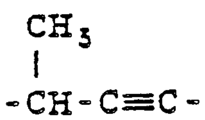 , 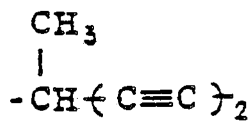 , 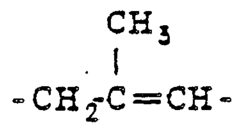 ,

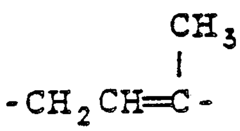 , 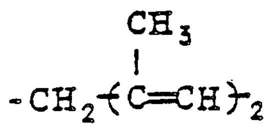 , 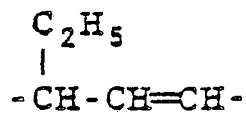 ,

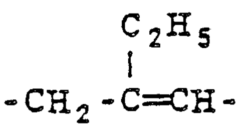 , 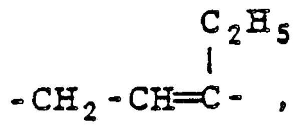 , 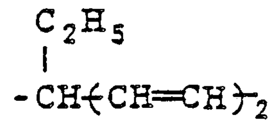 ,

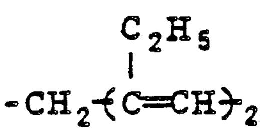 , $-CH_2-CF{=}CH-$ , 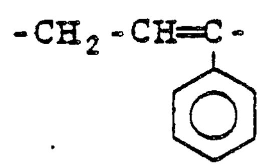 ,

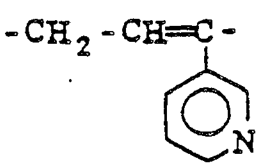 , $-CH_2-CH{=}CH-C{\equiv}C-$ ,

$-CH_2CH_2-CH{=}CH-$ , $-CH_2CH_2-O-N{=}CH-$ ,

$-CH_2CH_2-N{=}CH-$ , $-CH_2CH_2CH{=}N-$ , $-CH_2CH_2-N{=}N-$ , and

$-CH_2-C{\equiv}C-CH{=}CH-$ ,

$Ar^2$ may be o-, m- or p-phenylene group, a furandiyl group, a thiophenediyl group, a pyridinediyl group or a pyrrolediyl group, or $Ar^2$ may be linked with A by a methylene group, an ethylene group, a vinyl group, an oxygen atom, a sulfur atom or an amino group to form a 5- or 6-membered condensed ring.

Preferred examples of the group represented by $Ar^2$ are as follows:

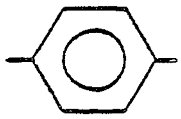 , 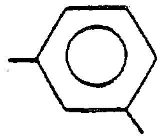 , 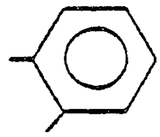 , 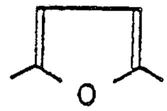 ,

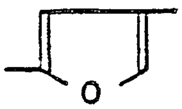 , 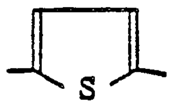 , 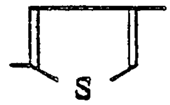 , 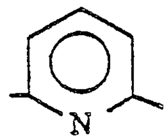 ,

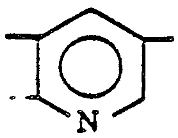 , 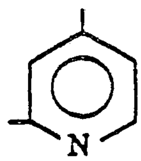 , 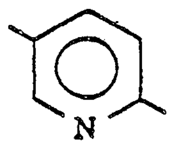 , 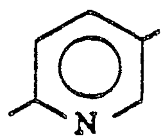 ,

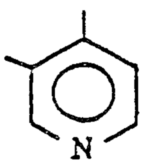 , 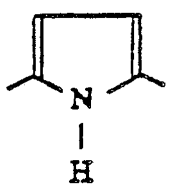 , 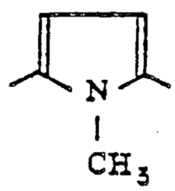 ,

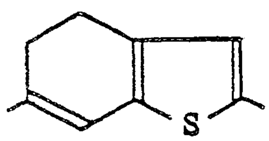 , 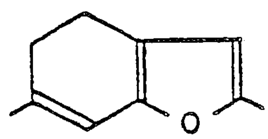 , 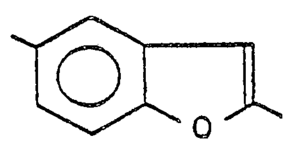 ,

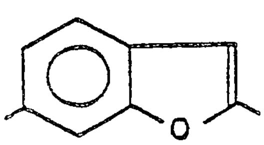 , 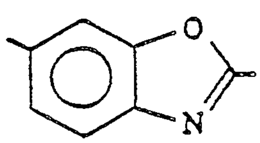 , 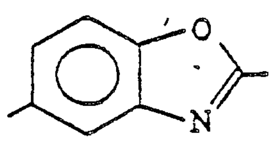 , and

,

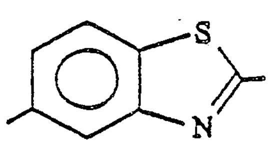 , 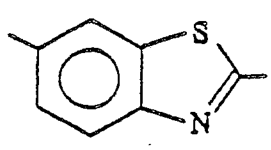 ,

B may be an alkylene or alkenylene group having 1 to 3 carbon atoms, which may have a substituent selected from the group consisting of a lower alkyl group having 1 to 4 carbon atoms, a cyclic alkyl group, an aryl and an aralkyl group.

Preferred examples of the group represented by B are a methylene group, an ethylene group, a trimethylene group, an ethylidene group, a benzylidene group, a vinylene group, a cyclohexylmethylene group and a 2-phenylethylidene group.

$Ar^3$ may be a heterocyclic group with five or six members containing therein at least one atom selected

from the group consisting of nitrogen, oxygen and sulfur. Preferred examples of the group represented by $Ar^3$ are a 2-pyrrolyl group, a 2-pyridyl group, a 3-pyridyl group, a 4-pyridyl group, an imidazolyl group, a 2-oxazolyl group, a 4-oxazolyl group, a 2-thiazolyl group, a triazolyl group and a tetrazolyl group.

B may be linked with $Ar^2$ or $Ar^3$ by a methylene group, an ethylene group, a vinylene group, an oxygen atom, a sulfur atom or a nitrogen atom to form a ring with five or six members, such as

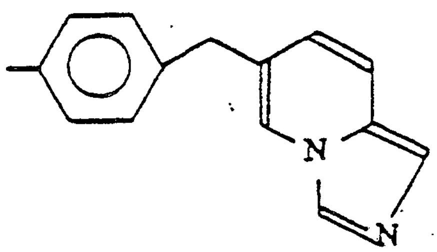
,
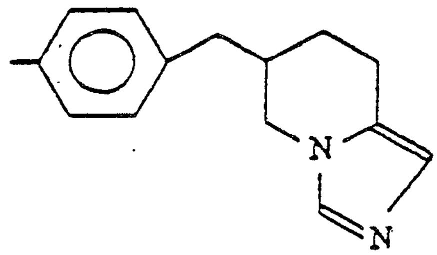
,

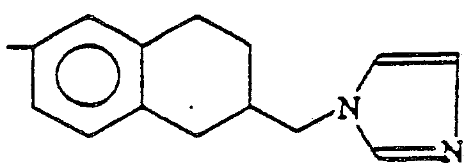

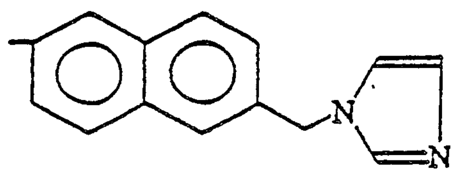

Process for preparation of 1,4-dihydropyridine derivatives

1,4-dihydropyridine derivatives having the above general formula (I) are prepared by the processes 1 or 2 as shown below:

[Process 1]

Esters having the general formula (II):

$CH_3COCH_2COOR^1$ (II)

are allowed to react with aldehydes having the general formula (III):

$Ar^1$-CHO (III)

and then are allowed to react with $\beta$-aminoacrylic acid ester having the general formula (IV):

$CH_3(NH_2)C = CH\text{-}COOA\text{-}Ar^2\text{-}B\text{-}Ar^3$ (IV)

wherein $R^1$, $Ar^1$, A, B, $Ar^2$ and $Ar^3$ are the same as those as shown in the above.

Esters having the general formula (II) include methyl acetoacetate, ethyl acetoacetate, isopropyl acetoacetate, 2-methoxyethyl acetoacetate, 2-cyanoethyl acetoacetate, 3-phenyl-2-propen-1-yl acetoacetate, 2,4-hexadiene acetoacetate, 3-{4-(1-imidazolylmethyl)phenyl}-2-propen-1-yl acetoacetate, 2-(N-benzyl-N-methylamino)ethyl acetoacetate, N-benzylpiperizin-3-yl acetoacetate and N-benzyl-pyrrolidin-3-yl acetoacetate.

Aldehydes having the general formula (III) include 2-nitrobenzaldehyde, 3-nitrobenzaldehyde, 4-nitrobenzaldehyde, 3-trifluoromethylbenzaldehyde, 3-azidobenzaldehyde, 3-pyridinecarboxaldehyde, 4-quinolinecarboxaldehyde, 4-cyanobenzaldehyde, 2,3-dichlorobenzaldehyde, furfural, 2-thiophenecarboxaldehyde, 3-thiophenecarboxaldehyde, 4-methoxybenzaldehyde, 4-methylthiobenzaldehyde and 4-tolualdehyde.

$\beta$-aminoacrylic esters include 3-{4-(1-imidazolylmethyl)phenyl}-2-propen-1-yl aminocrotonate, 3-{4-pyridine-3-yl-methyl)phenyl}-2-propen-1-yl aminocrotonate, 3-{4-(1,2,4-triazol-1-yl-methyl)phenyl}-2-propen-yl aminocrotonate, 3-{4-(1,2,3,4-tetrazol-1-yl-methyl)phenyl}-2-propen-1-yl aminocrotonate, 3-{2-(1-imidazolylmethyl)phenyl}-2-propen-1-yl aminocrotonate, 3-{3-(1-imidazolylmethyl)phenyl}-2-propen-1-yl aminocrotonate, 3-{5-(1-imidazolylmethyl)furan}-2-propen-1-yl aminocrotonate, 3-{5-(1-imidazolylmethyl)-thiophene}-2-propen-1-yl aminocrotonate, 3-{5-(1-imidazolylmethyl)pyridine}-2-propen-1-yl aminocrotonate,

3-{2-(1-imidazolylmethyl)-1-methylpyrrol-5-yl}-2-propen-1-yl aminocrotonate, 3-[4-{1-(1-imidazolyl)-ethyl}phenyl]-2-propen-1-yl aminocrotonate, 3-{4-(1-imidazolyl)phenyl}-2-propen-1-yl aminocrotonate, 3-{4-(1-imidazolylmethyl)phenyl}-2-propyn-1-yl aminocrotonate, 5-{4-(1-imidazolylmethyl)phenyl}-2,4-pentadien-1-yl aminocrotonate, 4,5-dihydro-2-(1-imidazolyl)methyl thianaphten-6-yl methyl aminocrotonate, 4,5-dihydro-2-{α-(1-imidazolyl)benzyl}thianaphten-6-yl methyl aminocrotonate and 3 {4-(imidazo-[1,2-a]pyridin-6-yl-methyl)phenyl}-2-propen-1-yl aminocrotonate.

The reactions are carried out in an inactive solvent such as methanol, ethanol, propanol, isopropanol, benzene, toluene, dioxane, tetrahydrofuran, dimethyl sulfoxide and dimethylformamide at temperatures of 50 °C to 150 °C.

[Process 2]

Carboxylic acid or carboxylic acid derivatives having the following general formula (V)

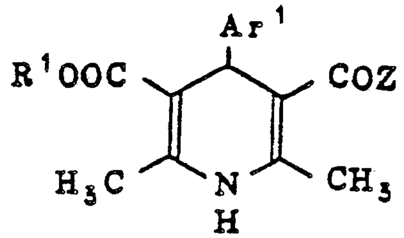

... (V)

are allowed to react with alcohol derivatives having the following general formula (VI)
HO-A-$Ar^2$-B-$Ar^3$ (VI)
wherein $R^1$, $Ar^1$, A, B, $Ar^2$ and $Ar^3$ are the same as those as shown in the above, and Z is hydroxyl group, halogen atom, methylsulfonyloxy group or benzotriazol-1-oxy group.

Carboxylic acid and derivatives thereof having the general formula (V) can be easily obtained by the conventional process (for instance, described in Chemical Pharmaceutical Bulletin, 28 2809 (1980)).

The carboxylic acid derivatives include 1,4-dihydro-2,6-dimethyl-5-methoxycarbonyl-4-(3-nitrophenyl)-pyridine-3-carboxylic acid, 1,4-dihydro-2,6-dimethyl-5-methoxycarbonyl-4-(3-nitrophenyl)pyridine-3-carboxylic acid chloride, 1,4-dihydro-2,6-dimethyl-5-ethoxycarbonyl-4-(3-nitrophenyl)pyridine-3-carboxylic acid chloride, 1,4-dihydro-2,6-dimethyl-5-isopropyloxycarbonyl-4-(3-nitrophenyl)pyridine-3-carboxylic acid chloride, 1,4-dihydro-2,6-dimethyl-5-(2,4-hexadien-1-yl)oxycarbonyl-4-(3-nitrophenyl)pyridine-3-carboxylic acid chloride, 1,4-dihydro-2,6-dimethyl-5-(3-phenyl-2-propen-1-yl)oxycarbonyl-4-(3-nitrophenyl)pyridine-3-carboxylic acid chloride and 1,4-dihydro-2,6-dimethyl-5-[3-{4-(1-imidazolylmethyl)phenyl}-2-propen-1-yl]-oxycarbonyl-4-(3-nitrophenyl)pyridine-3-carboxylic acid chloride.

Alcohol derivatives having the general formula (VI) are easily available and such alcohol derivatives include 3-{4-(1-imidazolylmethyl)phenyl}-2-propen-1-ol, 3-{4-(3-pyridylmethyl)phenyl}-2-propen-1-ol, 3-{4-(1,2,4-triazol-1-yl-methyl)phenyl}-2-propen-1-ol, 3-{4-(1,2,3,4-tetrazol-1-yl-methyl)phenyl}-2-propen-1-ol, 3-{2-(1-imidazolylmethyl)phenyl}-2-propen-1-ol, 3-{3-(1-imidazolylmethyl)phenyl}-2-propen-1-ol, 3-{5-(1-imidazolylmethyl)furan}-2-propen-1-ol, 3-{5-(1-imidazolylmethyl)thiophene}-2-propen-1-ol, 3-{2-(1-imidazolylmethyl)-1-methylpyrrol-5-yl}-2-propen-1-ol, 3-[4- {1-(1-imidazolyl)ethyl}phenyl]-2-propen-1-ol, 3-{4-(1-imidazolyl)phenyl}-2-propen-1-ol, 3-{4-(1-imidazolylmethyl)phenyl}-2-propyn-1-ol, 5-{4-(1-imidazolylmethyl)-phenyl}-2,4-pentadien-1-ol, 4,5-dihydro-2-(1-imidazolyl)methylthia naphten-6-yl-methanol, 4,5-dihydro-2-{α-(1-imidazolyl)benzyl}thianaphten-6-yl-methanol and 3-{4-(imidazo-[1,2-a]pyridin-6-yl-methyl)phenyl}-2-propen-1-ol.

In the above-mentioned processes. When "Z" of the general formula (V) is hydroxyl group, the reaction is carried out in the presence of acids (inorganic acid such as HCl or $H_2SO_4$, or Lewis acid such as $BF_3$) or condensing agents such as dicyclohexylcarbodiimide (DDC). The acids are used in an amount of 0.1 to 10 equivalents for carboxylic acids or derivatives thereof represented by the general formula (V), and the condensing agents are used in an amount of one to two equivalents for the carboxylic acid or derivatives thereof represented by the formula (V). When "DDC" is used as a condensing agent, a base such as N,N-dimethylaminopyridine may be used in an amount of 0.1 to one equivalent. The reaction is carried out in inactive solvents such as benzene, toluene, dichloromethane, chloroform, acetonitrile, dichloroethane or acetone.

Starting compounds for preparing 1,4-dihydropyridine derivatives of the present invention can be

synthesized as follows:

1. Synthesis of (E)-3-{4-(1-imidazolylmethyl)phenyl}-2-propen-1-ol:

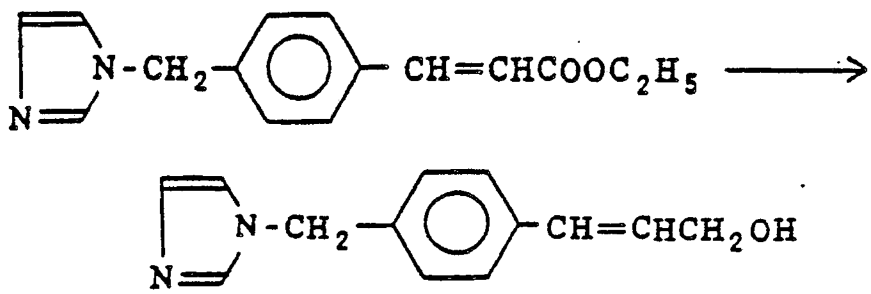

5.12 g (50 mM) of ethyl (E)-{4-(1-imidazolylmethyl)phenyl}acrylate was dissolved in 30 ml of tetrahydrofuran, and to this solution was added dropwise 77 ml of 1.5 M toluene solution of diisobutylaluminum hydride over one hour, while stirring under cooling with ice-cold water, and the reaction mixture was further stirred for one hour. Thereafter, to the mixture was added 1.5 ml of saturated brine, and after the mixture was stirred for two hours, the insoluble matter was filtered off and the filtrate was distilled under reduced pressure to remove the solvent 7.75 g of the captioned compound was obtained. Yield: 72.3% of theory

NMR $\delta CDCl_3$ 4.33 (d, 2H, J = 5Hz), 5.10 (s, 2H), 6.37 (dt, 1H, J = 16Hz, 5Hz), 6.61 (d, 1H, J = 16Hz), 6.89 (s, 1H), 7.09-7.12 (d like, 3H), 7.35-7.38 (d like, 2H), 7.54 (s, 1H).

2. Synthesis of (E)-3-{4-(1-imidazolylmethyl)phenyl}-2-propen-1-yl acetoacetate

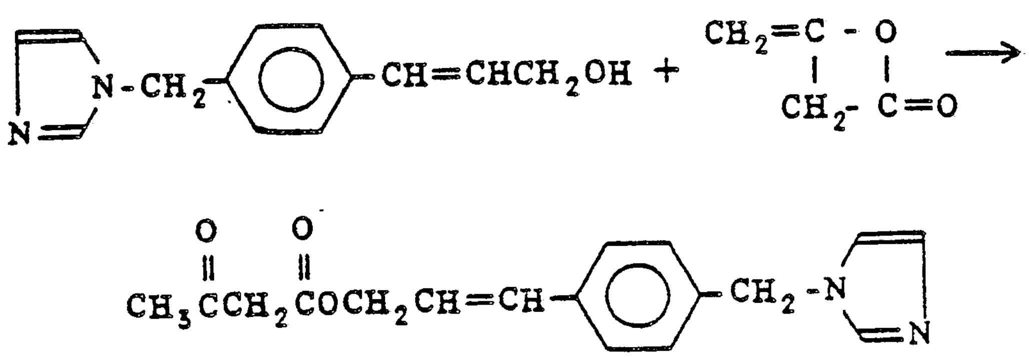

6.42 g (30 mM) of (E)-3-{4-(1-imidazolylmethyl)phenyl}-2-propen-1-ol was dissolved in 30 ml of benzene, and to this solution was added 0.305 g (0.3 mM) of triethylamine. The mixture was heated at a temperature of 70 °C. To this solution was added dropwise 3.03 g of diketene over 30 minutes and the mixture was stirred for one hour. The solvent was distilled off and the residue was chromatographed on a silica gel column. 8.95 g of the captioned compound was obtained.

NMR $\delta CDCl_3$ 2.28 (s, 3H), 3.51 (s, 2H), 4.80 (d, 2H, J = 6Hz), 5.11 (s, 2H), 6.28 (dt, 1H, J = 6Hz, 15Hz), 6.66 (d, 1H, J = 15Hz), 6.89 (s, 1H), 7.09 (s, 1H), 7.11 (d, 2H, J = 7Hz), 7.38 (d, 2H, J = 7Hz), 7.54 (s, 1H).

3. Synthesis of (E)-3-{4-(1-imidazolylmethyl)phenyl}-2-propen-1-yl 3-aminocrotonate

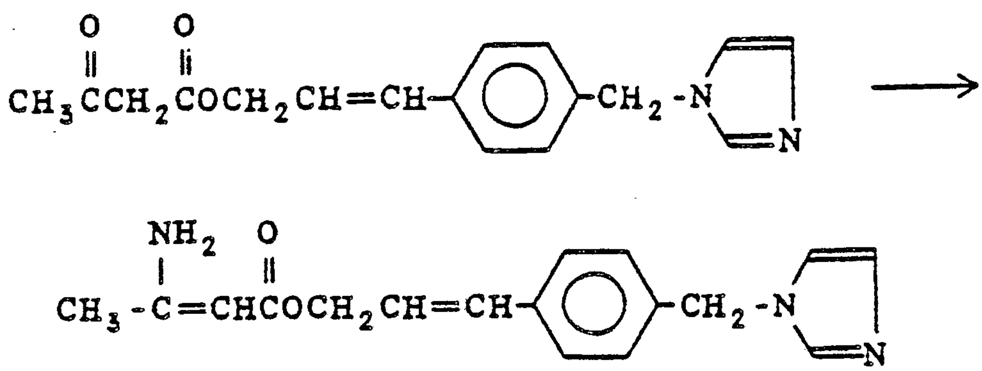

5.97 g (20 mM) of (E)-3-{4-(1-imidazolylmethyl)phenyl}-2-propen-1-yl acetoacetate was dissolved in 50 ml of methanol, and to the solution was added 7.71 g (160 mM) of ammonium acetate at room temperatures, while stirring, and the mixture was stirred for six hours. To the solution were added 100 ml of dichloromethane and a saturated aqueous solution of sodium hydrogencarbonate to alkalize the solution. After the dichloromethane phase was separated from the aqueous solution, washed repeatedly with water, and then dried over anhydrous sodium sulfate, the solvent was distilled off to obtain 5.32 g of the captioned compound. Yield: 89.4% of theory

NMR $\delta CDCl_3$ 1.92 (s, 3H), 4.58 (s, 1H), 4.73 (d, 2H, J=6Hz), 5.09 (s, 2H), 6.32 (dt, 1H, J=16, 6Hz), 6.62 (d, 1H, J=16Hz), 6.89 (s, 1H), 7.10 (d like 3H), 7.37 (d, 2H, J=8.4Hz), 7.54 (s, 1H).

Examples of 1,4-dihydropyridine derivatives of the present invention will be shown below:

Example 1-1

(E)-3-{4-(1-imidazolylmethyl)phenyl}-2-propen-1-yl methyl 1,4-dihydro-2,6-dimethyl-4-(3-nitrophenyl)-pyridine-3,5-dicarboxylate

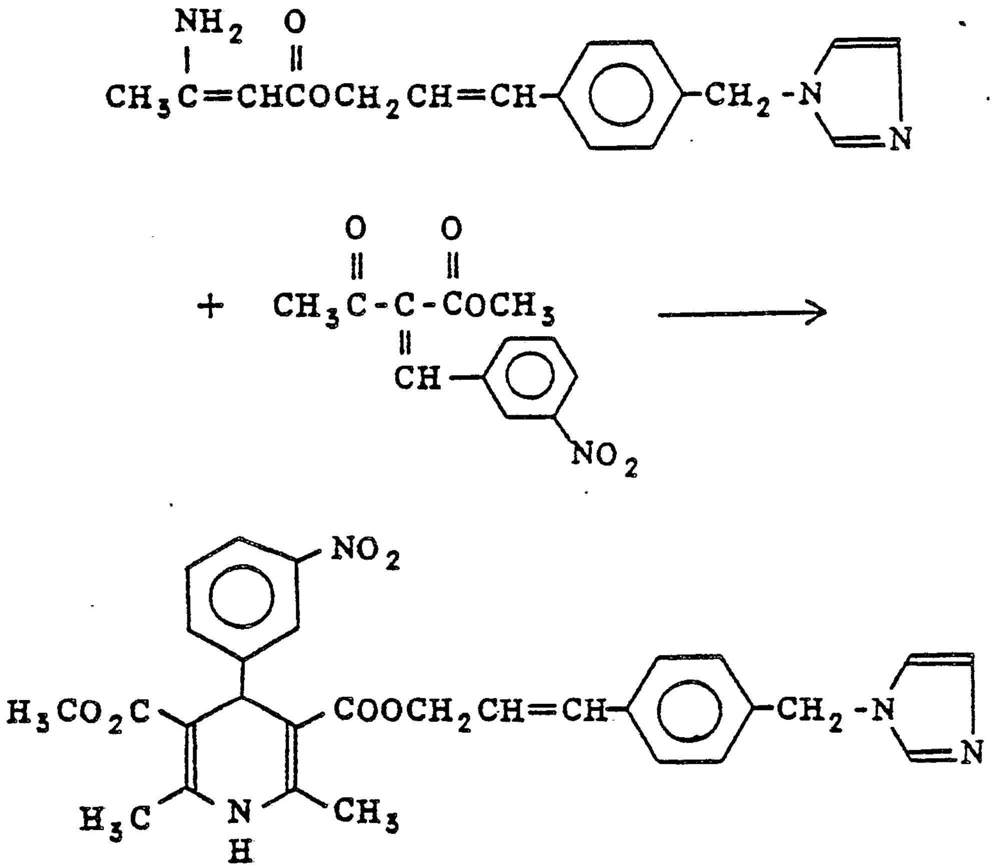

2.97 g (10 mM) of 3-{4-(1-imidazolylmethyl)phenyl}-2-propen-1-yl 3-aminocrotonate and 2.49 g (10 mM) of methyl 2-(3-nitrobenzylidene)acetoacetate were dissolved in 50 ml of toluene and then the solution was refluxed for eight hours. After cooling, the crystals were filtered off and then recrystallized from methanol to obtain 4.01 g of the captioned compound. Yield: 76% of theory

Melting point (°C) 176 ~ 178.4 (dec.)

NMR $\delta CDCl_3$ 2.37 (s, 3H), 2.39 (s, 3H), 3.63 (s, 3H), 4.6~4.8 (m, 2H), 5.12 (s, 2H), 6.23 (dt, 1H, J=16Hz), 6.40 (brs, 1H), 6.50 (d, 1H, J=16Hz), 6.92 (s, 1H), 7.1~7.13 (m, 3H), 7.31~7.37 (m, 3H), 7.57 (s, 1H), 7.58 (d, 1H, J=8,2Hz), 8.13 (t, 1H, J=2Hz).

IR ($cm^{-1}$) $\nu_{c=o}$ 1696

$\nu_{no_2}$ 1530 1352

Elemental analysis $C_{29}H_{28}N_4O_6$

| | C (%) | H (%) | N (%) |
|---|---|---|---|
| Found | 65.78 | 5.29 | 10.76 |
| Calcd. | 65.90 | 5.34 | 10.60 |

Example 1-2

(E)-3-{4-(1-imidazolylmethyl)phenyl}-2-propen-1-yl methyl 1,4-dihidro-2,6-dimethyl-4-(3-nitrophenyl)-pyridine-3,5-dicarboxylate

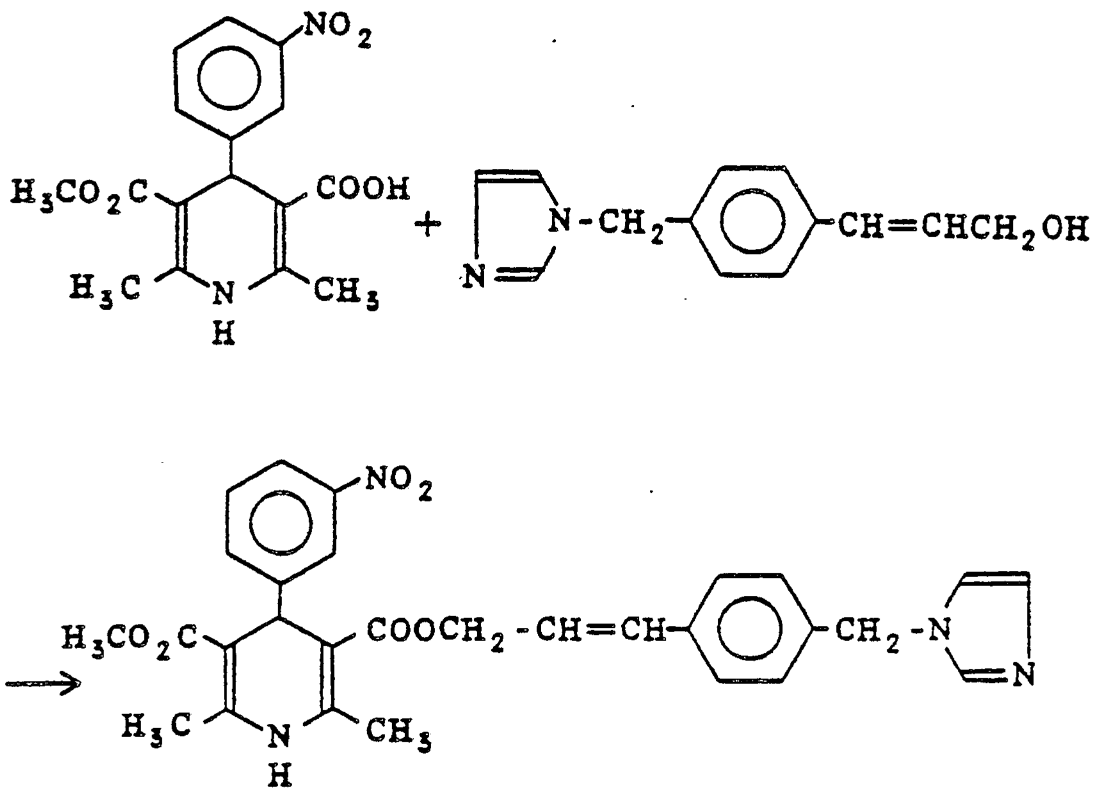

1. 66 g (5 mM) of 1,4-dihydro-2,6-dimethyl-5-methoxycarbonyl-4-(3-nitrophenyl)pyridine-3-carboxylic acid together with 1.09 g (5 mM) of 3-{4-(1-imidazolylmethyl)phenyl}-2-propen-1-ol, 1.03 g (5 mM) of dicyclohexylcarbodiimide and 0.61 g (5 mM) of 4-N,N-dimethylaminopyridine were dissolved in 20 ml of toluene, while heating, and refluxed for eight hours. The hot solution was cooled to a room temperature and the crystals produced were filtered off. The filtrate was washed with water and dried over anhydrous sodium sulfate. After the solvent was distilled off, the residue was purified by chromatography on a silica gel column to obtain 2.58 g of the captioned compound. Yield: 97.6% of theory

This compound is identical as the compound of Example 1-1 in melting point, NMR and IR values.

Example 2

Methyl (E)-3-{4-(3-pyridylmethyl)phenyl}-2-propen-1-yl 1,4-dihydro-2,6-dimethyl-4-(3-nitrophenyl)pyridine-3,5-dicarboxylate

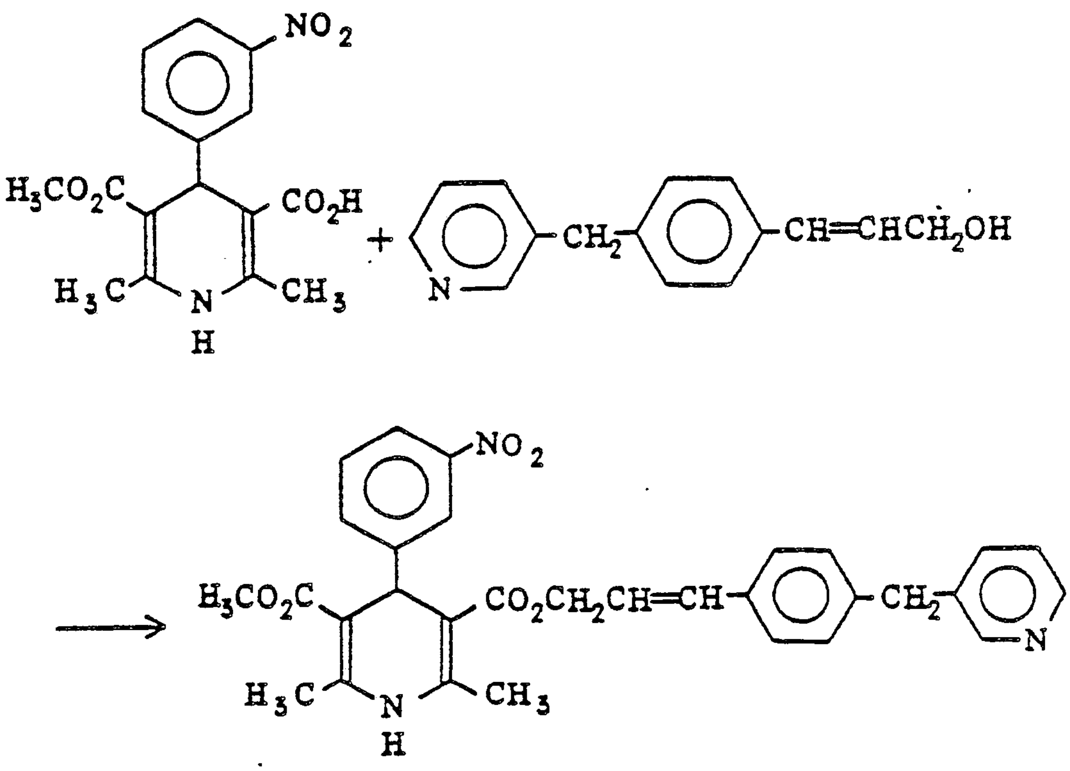

332 mg (1 mM) of 1,4-dihydro-2,6-dimethyl-5-methoxycarbonyl-4-(3-nitrophenyl)pyridine-3-carboxylic acid together with 225 mg (1 mM) of (E)-3-{4-(3-pyridylmethyl)phenyl}-2-propen-1-ol, 248 mg (1.2 mM) of dicyclohexylcarbodimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 481 mg of the captioned compound.

Yield: 89% of theory

Melting point (°C) 140.9 ~ 142.2

NMR $\delta CDCl_3$ 2.37 (s, 3H), 2.38 (s, 3H), 3.63 (s, 3H), 3.97 (s, 2H), 4.67 (ddd, 1H, J=13Hz, 6Hz, 1Hz), 4.74 (ddd, 1H, J=13Hz, 6Hz, 1Hz), 5.13 (s, 1H), 5.76 (bs, 1H), 6.20 (dt, 1H, J=16Hz, 6Hz), 6.51 (d, 1H, J=16Hz), 7.14 (d, 2H, J=8Hz), 7.21 (dd, 1H, J=8Hz, 5Hz), 7.29 (d, 2H, J=8Hz), 7.34 (t, 1H, J=8Hz), 7.46 (d, 1H, J=8Hz), 7.63 (d, 1H, J=8Hz), 7.98 (ddd, 1H, J=8Hz, 3Hz, 2Hz), 8.12 (t, 1H, J=2Hz), 8.47 (dd, 1H, J=5Hz, 2Hz), 8.50 (d, 1H, J=2Hz).

IR ($cm^{-1}$) $\nu$co 1700

$\nu no_2$ 1525, 1350

High mass Molecular formula $C_{31}H_{29}N_3O_6$

| | |
|---|---|
| Found | 539.20623 |
| Calcd. | 539.20560 |

Example 3

(E)-3-{4-(1-imidazolyl)phenyl}-2-propen-1-yl methyl 1,4-dihydro-2,6-dimethyl-4-(3-nitrophenyl)pylidine-3,5-dicarboxylate

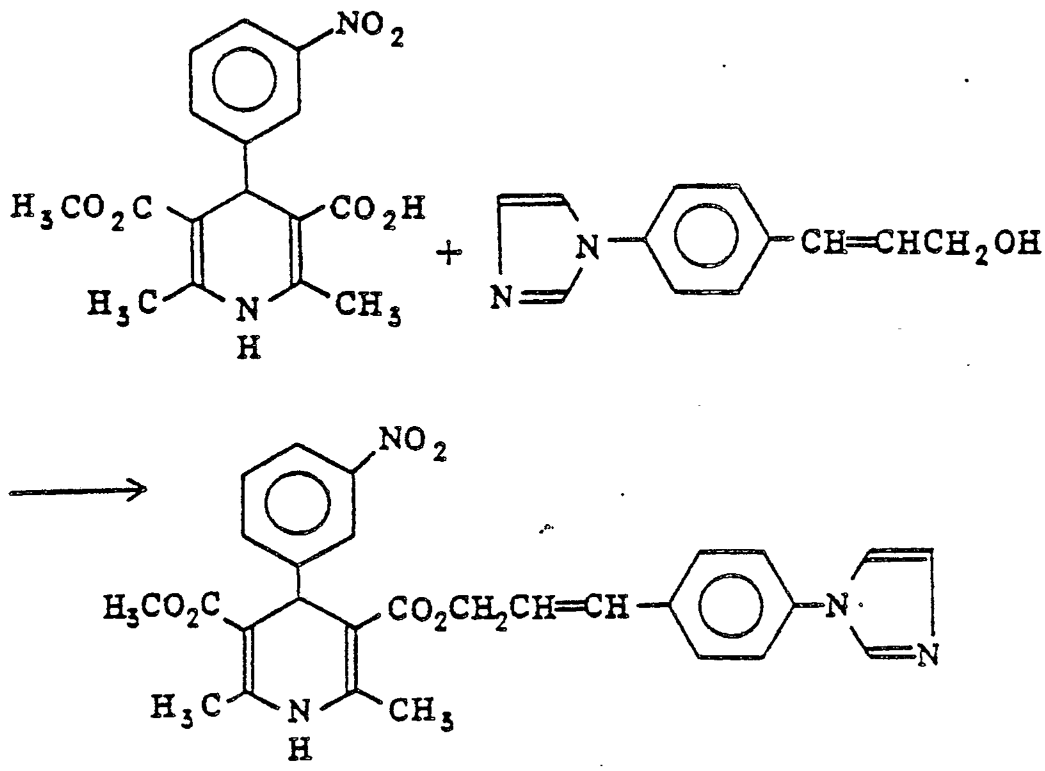

332 mg (1 mM) of 1,4-dihydro-2,6-dimethyl-5-methoxycarbonyl-4-(3-nitrophenyl)pyridine-3-carboxylic acid together with 200 mg (1 mM) of (E)-3-{4-(1-imidazolyl)phenyl}-2-propen-1-ol, 248 mg (1.2 mM) of dicyclohexyl carbodiimide and 134 mg (1.1 mM) of 4-N.N-dimethylaminopyridine were dissolved in 5 ml of toluene, while heating, and refluxed for six hours. The solution was cooled to room temperatures, and the crystals produced was filtered off. The filtrate was washed with water and dried over anhydrous sodium sulfate. After the solvent was distilled off, the residue was purified by chromatography on a silica gel column to obtain 494 mg of the captioned compound. Yield: 96% of theory

Melting point (°C) 220.2 ~ 221.7

NMR $\delta CDCl_3$ 2.38 (s, 3H), 2.41 (s, 3H), 3.65 (s, 3H), 4.69 (dd, 1H, J=13Hz, 6Hz), 4.78 (dd, 1H, J=13Hz, 6Hz), 5.15 (s, 1H), 5.81 (s, 1H), 6.27 (dt, 1H, J=16Hz, 6Hz), 6.54 (d, 1H, J=16Hz), 7.22 (s, 1H), 7.30 (s, 1H), 7.36 (d, 2H, J=8Hz), 7.37 (t, 1H, J=8Hz), 7.45 (d, 2H, J=8Hz), 7.65 (d, 1H, J=8Hz), 7.88 (s, 1H), 7.99 (dt, 1H, J=8Hz, 2Hz), 8.15 (t, 1H, J=2Hz).

IR ($cm^{-1}$) $\nu co$ 1705

$\nu no_2$ 1530, 1350

High mass Molecular formula $C_{28}H_{26}N_4O_6$

| | |
|---|---|
| Found | 514.18454 |
| Calcd. | 514.18520 |

Example 4

(E)-3-{5-(1-imidazolylmethyl)-2-furan}-2-propen-1-yl methyl 1,4-dihydro-2,6-dimethyl-4-(3-nitrophenyl)-pyridine-3,5-dicarboxylate

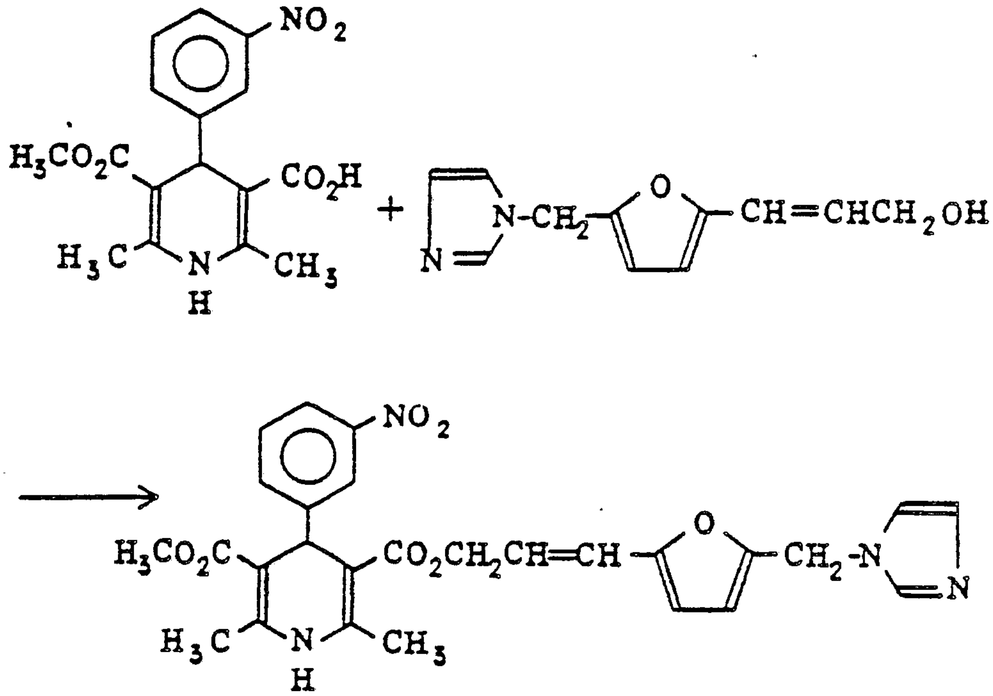

332 mg (1 mM) of 1,4-dihydro-2,6-dimethyl-5-methoxycarbonyl-4-(3-nitrophenyl)pyridine-3-carboxylic acid together with 204 mg (1 mM) of (E)-3-(5-(1-imidazolylmethyl}-2-furan]-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 430 mg of the captioned compound. Yield: 83% of theory

Melting point (°C) 169.1 ~ 170.0

NMR $\delta CDCl_3$ 2.37 (s, 3H), 2.38 (s, 3H), 3.64 (s, 3H), 4.59 (dd, 1H, J=13Hz, 6Hz), 4.74 (dd, 1H, J=13Hz, 6Hz), 5.09 (s, 2H), 5.12 (s, 1H), 6.09 (dt, 1H, J=16Hz, 6Hz), 6.16 (1H, J=3Hz), 6.20 (d, 1H, J=16Hz), 6.24 (bs, 1H), 6.27 (d, 1H, J=3Hz), 6.99 (s, 1H), 7.08 (s, 1H), 7.34 (t, 1H, J=8Hz), 7.58 (s, 1H), 7.64 (d, 1H, J=8Hz), 7.97 (dd, 1H, J=8Hz, 2Hz), 8.13 (t, 1H, J=2Hz).

IR ($cm^{-1}$) $\nu co$ 1695

$\nu no_2$ 1525, 1345

High mass Molecular formula $C_{27}H_{26}N_4O_7$

| | |
|---|---|
| Found | 518.18130 |
| Calcd. | 518.18011 |

Example 5

(E)-3-{5-(1-imidazolylmethyl)-2-thiophene}-2-propen-1-yl methyl 1,4-dihydro-2,6-dimethyl-4-(3-nitrophenyl) pyridine-3,5-dicarboxylate

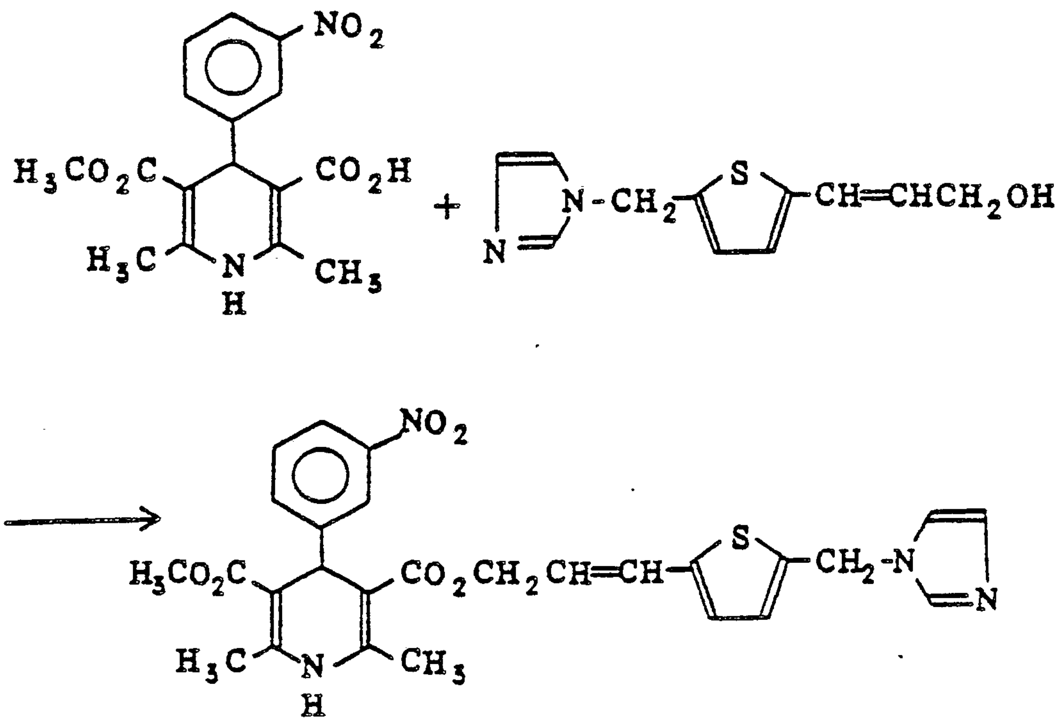

332 mg (1 mM) of 1,4-dihydro-2,6-dimethyl-5-methoxycarbonyl-4-(3-nitrophenyl)pyridine-3-carboxylic acid together with 220 mg (1 mM) of (E)-3-{5-(1-imidazolylmethyl)-2-thiophene}-2-propen-1-ol, 248 mg (1.2 mM) of dicyclohexylcarbodiimide and 134 mg (1.1 mM) of 4-N,N-dimethylaminopyridine were disssolved in 5 ml of toluene, while heating, and refluxed for six hours. The solution was cooled to room tempertures, and the crystals produced were filtered off. The filtrate was washed with water and dried over anhydrous sodium sulfate. After the solvent was distilled off, the residue was purified by chromatography on a silica gel column to obtain 514 mg of the capitioned compound. Yield: 96.1% theory

Melting point (°C) 169.5 ~ 172.3

NMR $CDCl_3$ 2.37 (s, 3H), 2.38 (s, 3H), 3.64 (s, 3H), 4.58 (ddd, 1H, J=13Hz, 6Hz, 1Hz), 4.71 (ddd, 1H, J=13Hz, 6Hz• Hz), 5.11 (s, 1H), 5.22 (s, 1H), 5.95 (dt, 1H, J=16Hz, 6Hz), 6.08 (bs, 1H), 6.53 (d, 1H, J=4Hz), 6.83 (d, 1H, J=4Hz), 6.97 (s, 1H), 7.09 (s, 1H), 7.35 (t, 1H, J=8 Hz), 7.57 (s, 1H), 7.63 (d, 1H, J=8Hz), 7.97 (dt, 1H, J=8Hz, 2Hz), 8.11 (t, 1H, J=2Hz).

IR ($cm^{-1}$) $\nu$co 1695

$\nu$no2 1525, 1350

High mass Molecular formule $C_{27}H_{26}N_4O_6S$

| | |
|---|---|
| Found | 534.15771 |
| Calcd. | 534.15727 |

Example 6

(E)-3-{4-(1-imidazolylmethyl)phenyl-2-buten-1-yl methyl 1,4-dihydro-2,6-dimethyl-4-[3-nitrophenyl) pyridine-3,5-dicarboxylate

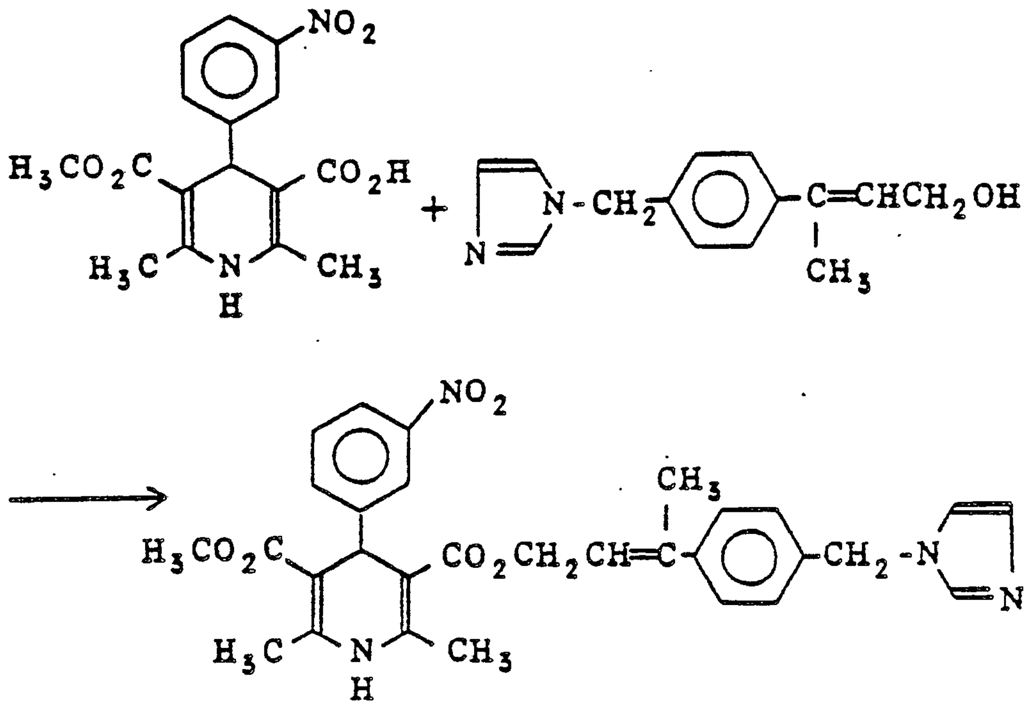

332 mg (1 mM) of 1,4-dihydro-2,6-dimethyl-5-methoxycarbonyl-4-(3-nitrophenyl)pyridine-3-carboxylic acid together with 228 mg (1 mM) of (E)-3-{4-(1-imidazolylmethyl)phenyl}-2-buten-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the cyrstals produced were filtered off. The filtrate was washed with water and dried over anhydrous sodium sulfate. After the solvent was distilled off, the residue was purified by chromatography on a silica gel column to obtain 539 mg of the captioned compound. Yield: 100% of theory

Melting point (°C) 201.9 ~ 202.9

NMR $\delta CDCl_3$ 2.03 (s, 3H), 2.37 (s, 3H), 2.39 (s, 3H), 3.64 (s, 3H), 4.71 (dd, 1H, J=13Hz, 6Hz), 4.78 (dd, 1H, J=13Hz, 6Hz), 5.11 (s, 1H), 5.12 (s, 2H), 5.77 (s, 1H), 5.82 (t, 1H, J=6Hz), 6.92 (s, 1H), 7.11 (s, 1H), 7.12 (d, 2H, J=8Hz), 7.34 (t, 1H, J=8Hz), 7.34 (d, 2H, J=8Hz), 7.56 (s, 1H), 7.63 (d, 1H, J=8Hz), 7.98 (dt, 1H, J=8Hz, 2Hz), 8.12 (t, 1H, J=8Hz).

IR (cm$^{-1}$) $\nu$co 1695

$\nu no_2$ 1530, 1350

High mass Molecular formula $C_{30}H_{30}N_4O_6$

| | |
|---|---|
| Found | 542.21719 |
| Calcd. | 542.21650 |

Exmaple 7

Ethyl (E)-3-{4-(1-imidazolylmethyl)phenyl}-2-propen-1-yl 1,4-dihydro-2,6-dimethyl-4-(3-nitrophenyl)pyridine-3,5-dicarboxylate

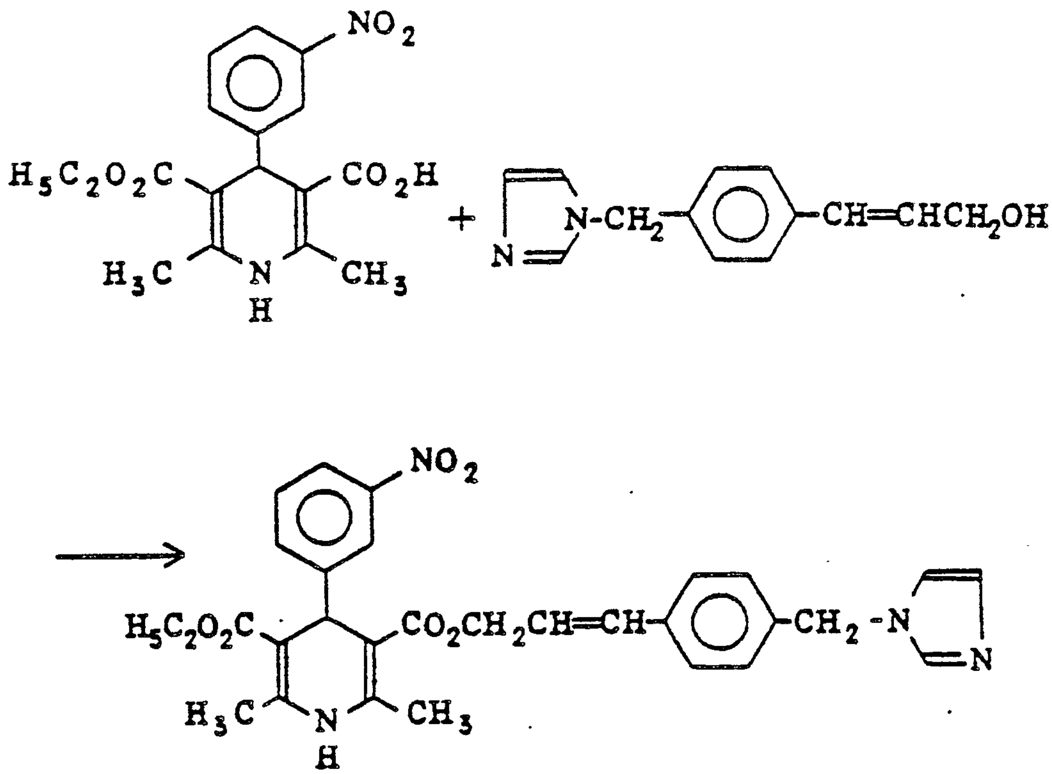

346 mg(1 mM) of 1,4-dihydor-2,6-dimethyl-5-etoxycarbonyl-4-(3-nitrophenyl)pyridine-3-caboxylic acid together with 215 mg(1 mM) of (E)-3-{4-(1-imidazolylmethyl)phenyl}-2-propen-1-ol, 248 mg (1.2 mM) of dicyclohexylcarbodiimide and 134 mg(1.1 mM) of 4-N,N-dimethylaminopyridine were dissolved in 5 ml of toluene, while heating, and refluxed for six hours, The solution was cooled to room temperatures and the crystals produced were filtered off. The filtrate was washed with water and dried over anhydrous sodium sulfate. After the solvent was distilled off, the residue was purified by chromatography on a silica gel column to obtain 474 mg of the captioned compund. Yield: 87.4% of theory

Melting point (°C) 171.8 ~ 172.5

NMR $\delta CDCl_3$ 1.21 (t, 3H, J=6HZ), 2.40 (s, 3H), 2.39 (s, 3H), 4.01-4.17 (m, 2H), 4.65 (ddd, 1H, J=13Hz, 6Hz, 1Hz), 4.75 (ddd, 1H, J=13Hz, 6Hz, 1Hz), 5.11 (s, 2H), 5.13 (s, 1H), 5.74 (s, 1H), 6.21 (dt, 1H, J=16Hz, 6Hz), 6.49 (d, 1H, J=16Hz), 6.91 (s, 1H), 7.10 (s, 1H), 7.11 (d, 2H, J=8Hz), 7.31 (d, 2H, J=8Hz), 7.35 (t, 1H, J=8Hz), 7.55 (s, 1H), 7.64 (d, 1H, J=8Hz), 7.98 (dd, 1H, J=8Hz, 2Hz), 8.14 (t, 1H, J=2Hz)

IR($cm^{-1}$) $\nu co$ 1695

$\nu no_2$ 1525, 1350

High mass Molecular formula $C_{30}H_{30}N_4O_6$

| | |
|---|---|
| Found | 542.21698 |
| Colcd. | 542.21650 |

Example 8

(E)-3-{4-(1-imidazolylmethyl)phenyl}-2-methyl-2-propen-1-yl methyl 1,4-dihydro-2,6-dimethyl-4-(3-nitro-phenyl)pyridine-3,5-dicarboxylate

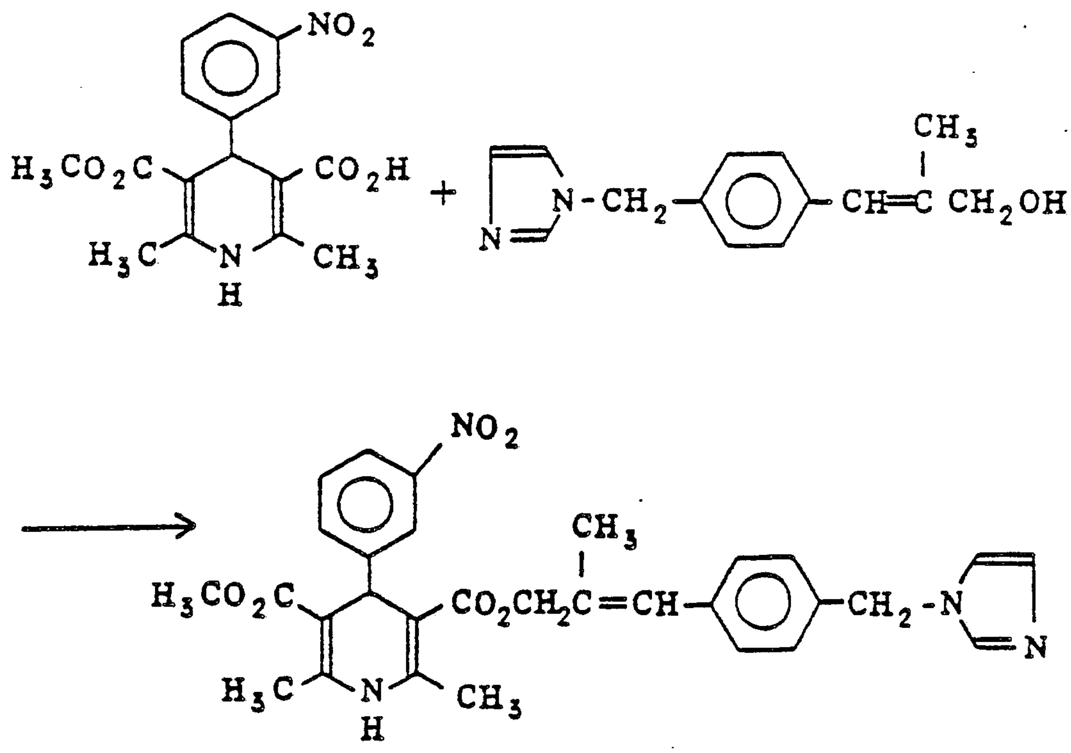

332 mg (1 mM) of 1,4-dihydro-2,6-dimethyl-5-methoxycabonyl-4-(3-nitrophenyl)pyridine-3-caboxylic acid together with 228 mg (1 mM) of (E)-3-{4-(1-imidazolylmethyl)phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled in room tempertures, and the cyrstals produced were filtered off. The filtrate was washed with water and dried over anbydrous sodium sulfate. After the solvent was distilled off, the residue was purified by chromatography on a silica gel column to obtain 523 mg of the captioned compound. Yield: 96.3% of theory

Melting point (°C) 166.1 ~ 167.1

NMR $\delta CDCl_3$ 1.75 (s, 3H), 2.36 (s, 3H), 2.41 (s, 3H), 3.65 (s, 3H), 4.55 (d, 1H, J=13Hz), 4.66 (d, 1H, J=13Hz), 5.11 (s, 2H), 5.15 (s, 1H), 5.98 (s, 1H), 6.33 (s, 1H), 6.92 (s, 1H), 7.10 (s, 1H), 7.12 (d, 2H, J=8Hz), 7.19 (d, 2H, J=8Hz), 7.35 (t, 1H, J=8Hz), 7.56 (s, 1H), 7.65 (d, 1H, J=8Hz), 7.98 (dd, 1H, J=8Hz, 2Hz), 8.12 (t, 1H, J=2Hz)

IR ($cm^{-1}$) $\nu co$ 1695

$\nu no_2$ 1530, 1350

High mass Molecular formula $C_{30}H_{30}N_4O_6$

| | |
|---|---|
| Found | 542.21735 |
| Calcd. | 542.21650 |

Example 9

(E)-3-{4-(1,3,4-triazol-1-ylmethyl)phenyl}-2-propen-1-yl 1,4-dihydro-2,6-dimethyl-4-(3-nitrophenyl)-pyridine3,5-dicarboxylate

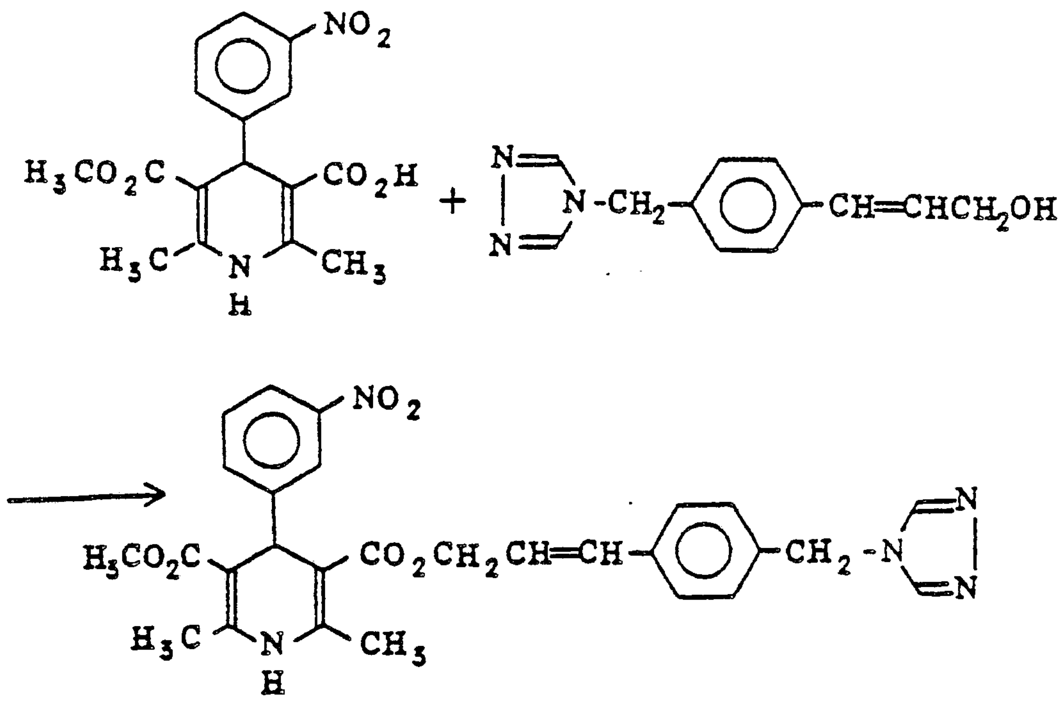

332 mg (1 mM) of 1,4-dihydro-2,6-dimethyl-5-methoxycarbonyl-4-(3-nitrophenyl)pyridine-3-carboxylic acid together with 215 mg (1 mM) of (E)-3-{4-(1,3,4-triazol-1-ylmethyl)phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography in a silica gel column to obtain 522 mg of the captioned compound. Yield: 98.6% of theory

Melting point (°C) 226.2 ~ 227.8

NMR $\delta CDCl_3$ 2.37 (s, 3H), 2.39 (s, 3H), 3.64 (s, 3H), 4.66 (dd, 1H, J = 13Hz, 6Hz), 4.77 (dd, 1H, J = 13Hz, 6Hz), 5.13 (s, 1h), 5.17 (s, 2H), 5.86 (s, 1H), 6.24 (dt, 1H, J = 16Hz, 6Hz), 6.49 (d, 1H, J = 16Hz), 7.15 (d, 2H, J = 8Hz), 7.35 (t, 1H, J = 8Hz), 7.97 (dd, 1H, J = 8Hz, 2Hz), 8.13 (t, 1H, J = 2H), 8.19 (S, 2H)

IR ($cm^{-1}$) $\nu$co 1695

$\nu no_2$ 1530, 1350

High mass Molecular formula $C_{28}H_{27}N_5O_6$

| | |
|---|---|
| Found | 529.19689 |
| Calcd. | 529.19610 |

Example 10

Methyl (E)-3-{4-(1,2,4-triazol-1-ylmethyl)phenyl}-2propen-1-yl 1,4-dihydro-2,6-dimethyl-4-(3-nitrophenyl)-pyridine-3,5-dicarboxylate

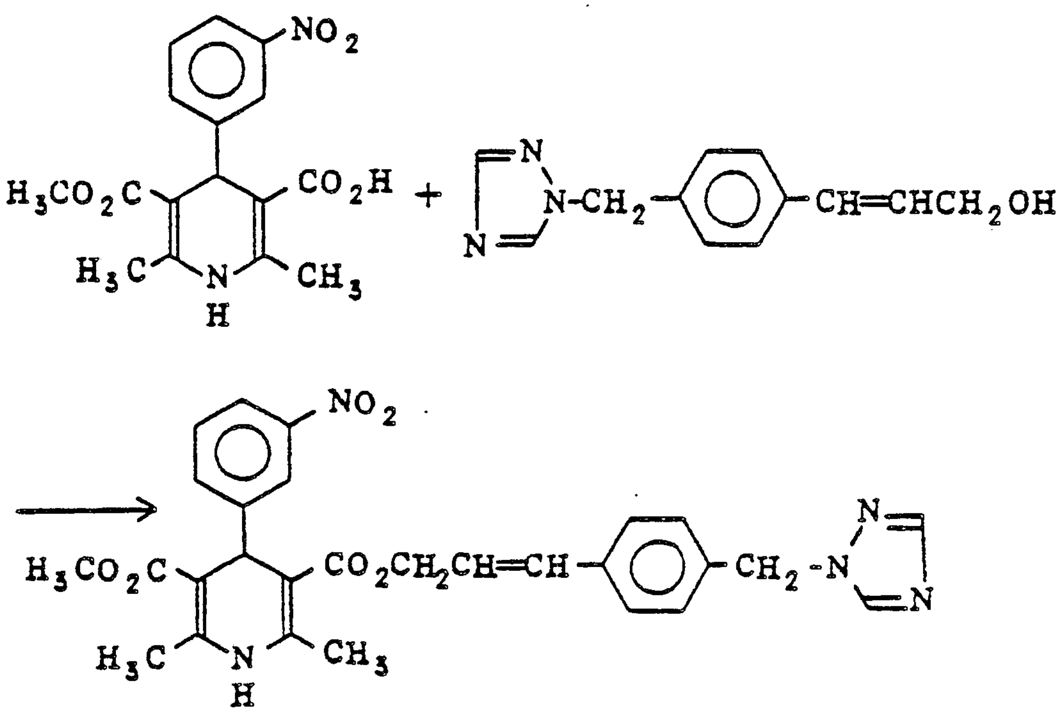

332 mg (1 mM) of 1,4-dihydro-2,6-dimethyl-5-methoxycarbonyl-4-(3-nitrophenyl)pyridine-3-carboxylic acid together with 215 mg (1 mM) of (E)-3-{4-(1,2,4-triazol-1-ylmethyl)phenyl}-2-propen-1-ol, 248 mg (1.2 mM) of dicyclohexylcarbodiimide and 134 mg (1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 522 mg of the captioned compound. Yield: 98.6% of theory

Melting point (°C) 140.6 ~ 142.3

NMR $\delta CDCl_3$ 2.37 (s, 3H), 2.39 (s, 3H), 3.64 (s, 3H), 4.66 (dd, 1H, J=13Hz, 6Hz), 4.75 (dd, 1H, J=13H, 6Hz), 5.13 (s, 1H), 5.34 (s, 2H), 5.73 (s, 1H), 6.23 (dt, 1H, J=16H, 6H), 6.51 (d, 1H, J=16Hz), 7.23 (d, 2H, J=8Hz), 7.34 (d,2H, J=8Hz), 7.34 (t, 1H, J=7Hz), 7.63 (d, 1H, J=7Hz), 7.98 (dd, 1H, J=7Hz, 2Hz), 7.99 (s, 1H), 8.07 (s, 1H), 8.12 (t, 1H, J=2Hz).

IR ($cm^{-1}$) $\nu co$ 1695

$\nu no_2$ 1530, 1350

High mass Molcular formula $C_{28}H_{27}N_5O_6$

| | |
|---|---|
| Found | 529.19641 |
| Calcd. | 529.19610 |

Example 11

Methyl (E)-3-{4-(1,2,3,4-tetrazol-1-ylmethyl)phenyl}-2-propen-1-yl 1,4-dihydro-2,6-dimethyl-4-(3-nitrophenyl)-pyridine-3,5-dicarboxylate

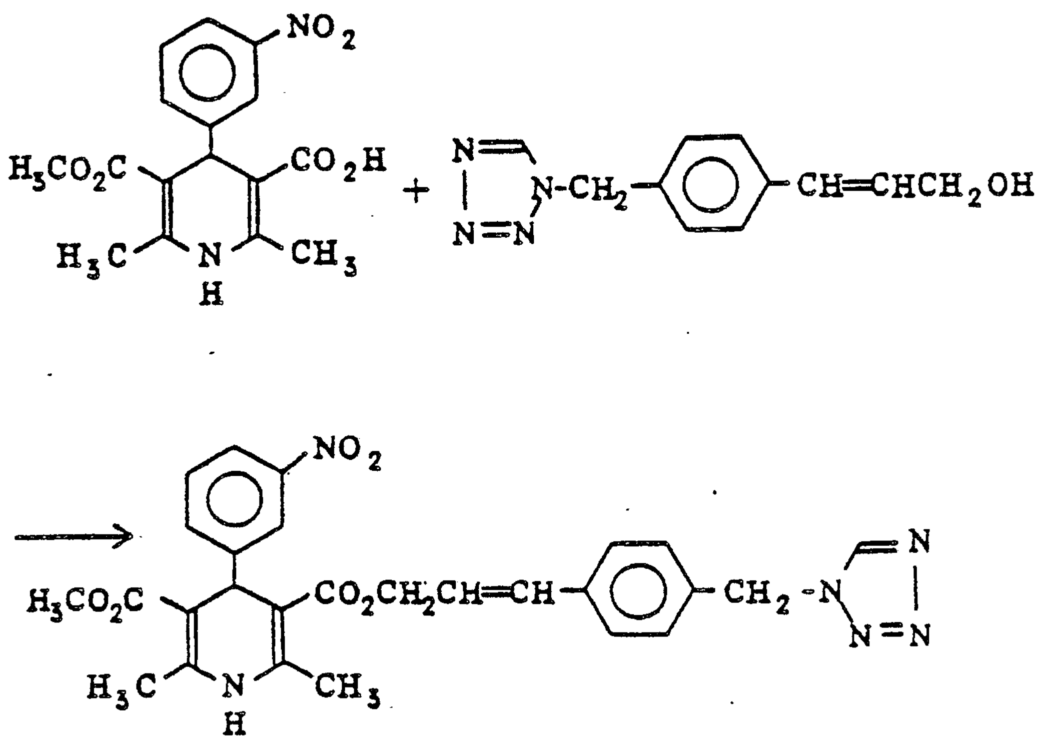

332 mg (1 mM) of 1,4-dihydro-2,6-dimethyl-5-methoxycarbonyl-4-(3-nitrophenyl)pyridine-3-carboxylic acid together with 217 mg (1 mM) of (E)-3-{4-(1,2,3,4-tetrazol-1-ylmethyl)phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 442 mg of the captioned compound. Yield: 83.4% of theory

Melting point (°C) 108.2 ~ 109.9

NMR $\delta CDCl_3$ 2.37 (s, 3H), 2.39 (s, 3H), 3.64 (s, 3H), 4.65 (dd, 1H, J=13Hz, 6Hz), 4.77 (dd, 1H, J=13Hz, 6Hz), 5.13 (s, 1H), 5.58 (s, 2H), 5.77 (s, 1H),6.24 (dt, 1H, J=16Hz, 6Hz), 6.50 (d, 1H, J=16Hz), 7.26 (d, 2H, J=8Hz), 7.35 (t, 1H, J=8Hz), 7.37 (d, 2H, J=8Hz), 7.63 (d, 1H, J=8Hz), 7.97 (dd, 1H, J=8Hz, 2Hz), 8.12 (t, 1H, J=2Hz), 8.52 (s, 1H).

IR ($cm^{-1}$) $\nu co$ 1700

$\nu no_2$ 1530, 1355

mass (m/e) 530 ($M^+$), 499, 408, 331, 287

Example 12

(E)-3-{4-(1-imidazolylmethyl)phenyl}-2-propen-1-yl methyl 1,4-dihydro-2,6-dimethyl-4-(2,3-dichlorophenyl)-pyridine-3,5-dicarboxylate

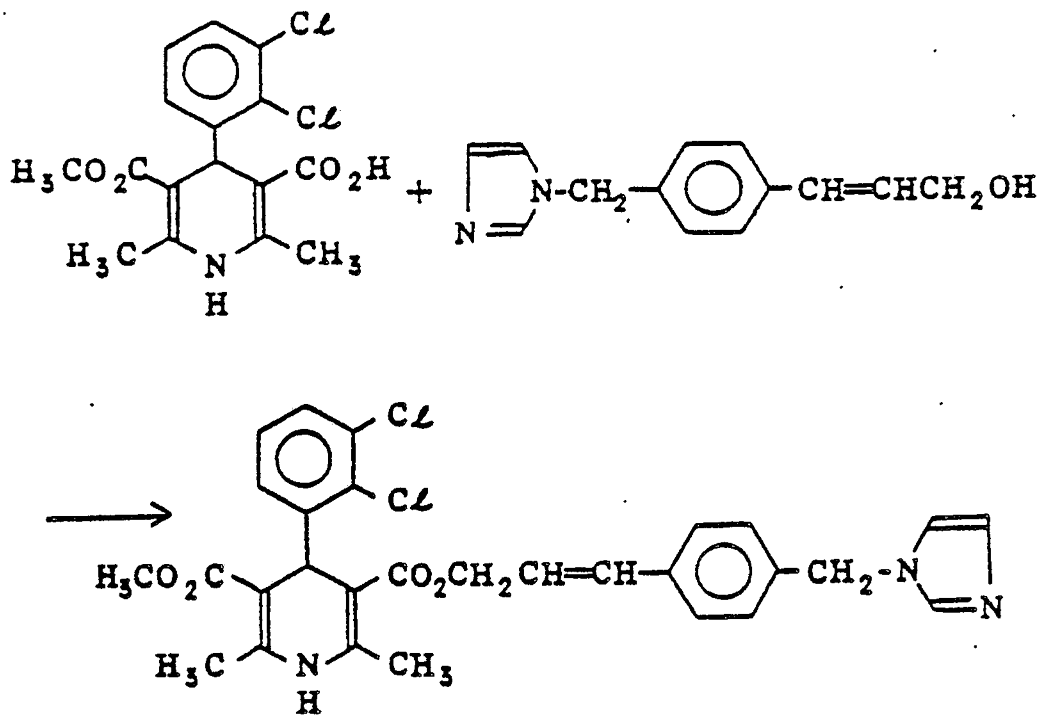

356 mg (1 mM) of 1,4-dihydro-2,6-dimethyl-5-methoxycarbonyl-4-(2,3-dichlorophenyl)pyridine-3-carboxylic acid together with 215 mg (1 mM) of (E)-3-{4-(1-imidazolylmethyl)-phenyl}-2-propen-1-ol, 248 mg (1.2 mM) of dicyclohexyl-carbodiimide and 134 mg (1.1 mM) of 4-N,N-dimethylamino-pyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off,the residue was purified by chromatography on a silica gel column to obtain 481 mg of the captioned compound. Yield: 87% of theory

Melting point (°C) 121.1 ~ 122.5

NMR $\delta CDCl_3$ 2.32 (s, 3H), 2.34 (s, 3H), 3.60 (s, 3H), 4.63 (ddd, 1H, J=13Hz, 6Hz, 2Hz), 4.72 ddd, 1H, J=13Hz, 6Hz, 2Hz), 5.10 (s, 2H), 5.49 (s, 1H), 5.70 (s, 1H), 6.18 (dt, 1H, J=16Hz, 6Hz), 6.41 (d, 1H, J=16Hz), 6.90 (s, 1H), 7.05 (t, 1H,J=8Hz), 7.09 (d, 2H, J=8Hz), 7.10 (s, 1H), 7.21 (dd, 1H, J=8Hz, 2Hz), 7.30 (d, 3H, J=8Hz), 7.55 (s, 1H).

IR ($cm^{-1}$) $\nu co$ 1695

High mass Molecular formula $C_{29}H_{27}Cl_2N_3O_4$

| | |
|---|---|
| Found | 551.13765 |
| Calcd. | 551.13783 |

Example 13

(Z)-3-{4-(1-imidazolylmethyl)phenyl}-2-fluoro-2-propen-1-yl methyl 1,4-dihydro-2,6-dimethyl-4-(3-nitrophenyl)pyridine-3,5-dicarboxylate

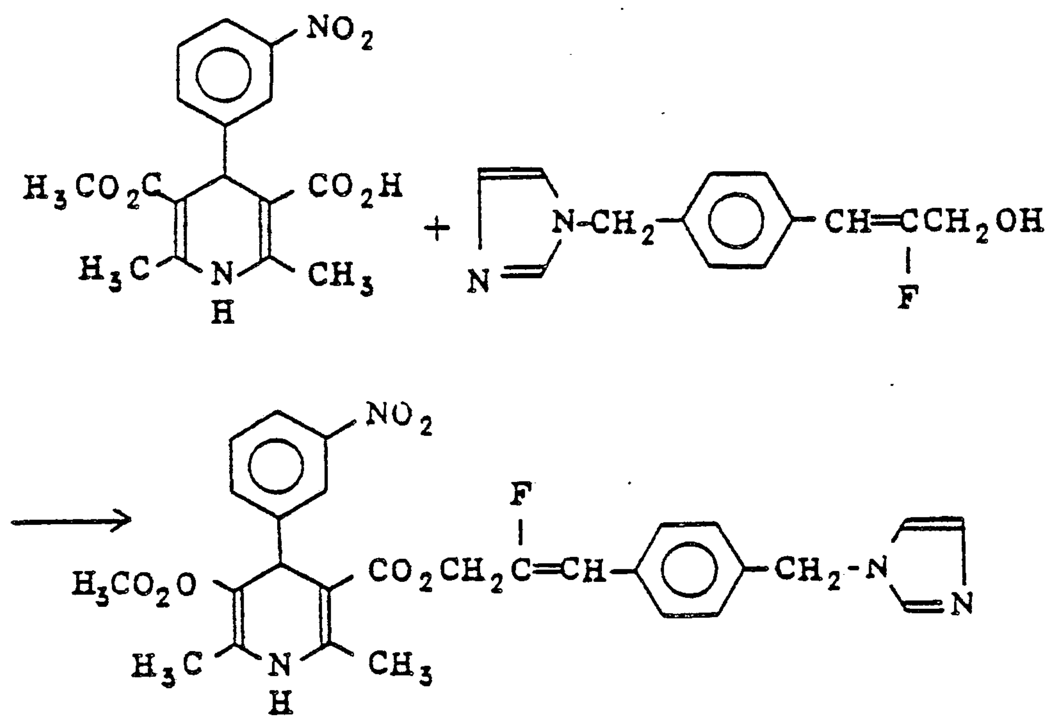

332 mg (1 mM) of 1,4-dihydro-2,6-dimethyl-5-methoxycarbonyl-4-(3-nitrophenyl)pyridine-3-carboxylic acid together with 219 mg (1 mM) of (Z)-3-{4-(1-imidazolylmethyl)phenyl}-2-fluoro-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 506 mg of the captioned compound. Yield: 92.5% of theory

Melting point (°C) 186.2 ~ 188.3

NMR $\delta CDCl_3$ 2.38 (s, 3H), 2.39 (s, 3H), 3.63 (s, 3H), 4.63 (dd, 1H, J=13Hz, 18Hz), 4.75 (dd, 1H, J=13Hz, 18Hz), 5.12 (s, 3H), 5.71 (s, 1H, 37Hz), 5.86 (s, 1H), 6.91 (s, 1H), 7.10 (s, 1H), 7.13 (d, 2H, J=8Hz), 7.34 (t, 1H, J=8Hz), 7.45 (d, 2H, J=8Hz), 7.56 (s, 1H), 7.64 (d, 1H, J=8Hz), 7.97 (dd, 1H, J=8Hz, 2Hz), 8.11 (t, 1H, J=2Hz)

IR ($cm^{-1}$) $\nu co$ 1700

$\nu no_2$ 1530, 1350

High mass Molecular formula $C_{29}H_{27}FN_4O_6$

| | |
|---|---|
| Found | 546.19285 |
| Calcd. | 546.19143 |

Example 14

(E)-cinnamyl (E)-3-{4-(1-imidazolylmethyl)phenyl}-2-propen-1-yl 1,4-dihydro-2,6-dimethyl-4-(3-nitrophenyl)-pyridine-3,5-dicarboxylate

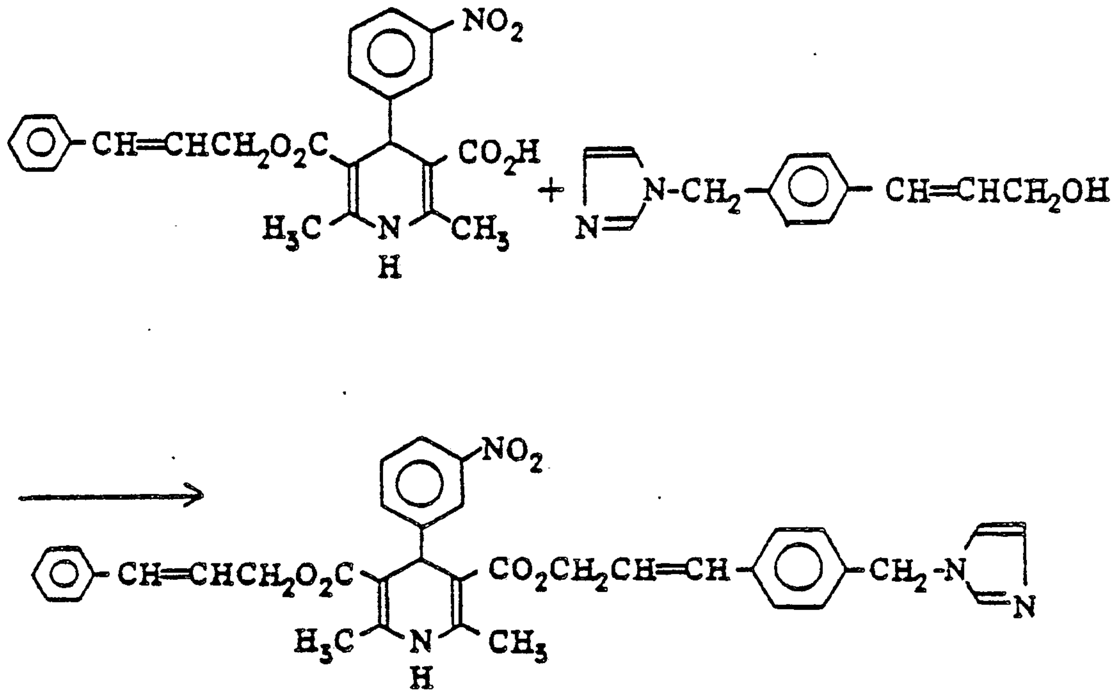

434 mg (1 mM) of 5-(E)-cinnamyloxycarbonyl-1,4-dihydro-2,6-dimethyl-4-(3-nitrophenyl)pyridine-3-carboxylic acid together with 215 mg (1 mM) of (E)-3-{4-(1-imidazolylmethyl)phenyl}-2-propen-1-ol, 248 mg (1.2 mM) of dicyclo hexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 588 mg of the captioned compound. Yield: 93.2% of theory

Melting point (°C) 96.8 ~ 98.5

NMR $\delta CDCl_3$ 2.39 (s, 6H), 4.61 - 4.78 (m, 4H), 5.09 (s, 2H), 5.18 (s, 1H), 5.79 (s, 1H), 6.14 - 6.26 (m, 2H), 6.48 (d, 1H, J=16Hz), 6.53 (d, 1H, J=16Hz), 6.90 (s, 1H), 7.07 (d, 2H, J=8Hz), 7.11 (s, 1H), 7.24 - 7.34 (m, 8H), 7.56 (s, 1H), 7.65 (d, 1H, J=8Hz), 7.95 (dd, 1H, J=8Hz, 2Hz), 8.16 (t, 1H, J=2Hz)

IR ($cm^{-1}$) $\nu$co 1695

$\nu no_2$ 1530, 1345 .

High mass Molecular formula $C_{37}H_{34}N_4O_6$

| | |
|---|---|
| Found | 630.24661 |
| Calcd. | 630.24779 |

Example 15

(E)-2,(E)-4-hexadien-1-yl (E)-3-{4-(1-imidazolylmethyl)phenyl}2-propen-1-yl 1,4-dihydro-2,6-dimethyl-4-(3-nitrophenyl)pyridine-3,5-dicarboxylate

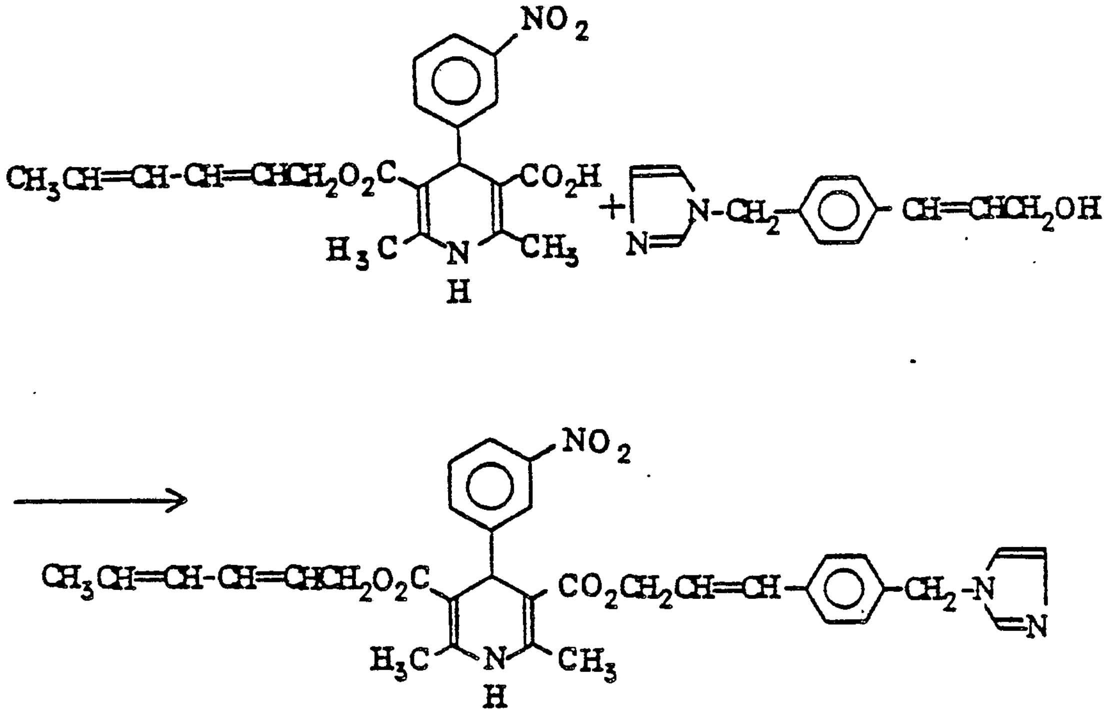

398 mg (1 mM) of 1,4-dihydro-2,6-dimethyl-5-{(E)-2,(E)-4-hexadien-1-yl}oxycarbonyl-4-(3-nitrophenyl)-pyridine-3-carboxylic acid together with 215 mg (1 mM) of (E)-3-{4-(1-imidazolylmethyl)phenyl}-2-propen-1-ol, 248 mg (1,2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel solumn to obtain 544 mg of the captioned compound. Yield: 91.4% of theory

Melting point (°C) 138.6 ~ 140.2

NMR $\delta CDCl_3$ 1.73 (d, 3H, J=7Hz), 2.37 (s, 3H), 2.39 (s, 3H), 4.50 (dd, 1H, J=13Hz, 7Hz), 4.58 (dd, 1H, J=13Hz, 7Hz), 4.65 (dd, 1H, J=13Hz, 6Hz), 4.75 (dd, 1H, J=13Hz, 6Hz), 5.11 (s, 2H), 5.14 (s, 1H), 5.55 (dt, 1H, J=15Hz, 7Hz), 5.67 (dq, 1H, J=15Hz, 7Hz), 5.82 (bs, 1H), 5.99 (dd, 1H, J=15Hz, 11Hz), 6.12 (dd, 1H, J=15Hz, 11Hz), 6.20 (dt, 1H, J=16Hz, 6Hz), 6.49 (d, 1H, J=16Hz), 6.90 (s, 1H), 7.10 (s, 1H), 7.11 (d, 2H, J=8Hz), 7.31 (d, 2H, J=8Hz), 7.34 (t, 1H, J=8Hz), 7.56 (s, 1H), 7.64 (d, 1H, J=8Hz), 7.97 (dd, 1H, J=8Hz, 2Hz), 8.13 (s, 1H).

IR ($cm^{-1}$) $\nu co$ 1700

$\nu no_2$ 1530, 1350

High mass Molecular formula $C_{34}H_{34}N_4O_6$

| | |
|---|---|
| Found | 594.24765 |
| Calcd. | 594.24779 |

Example 16

(E)-3-{4-(1-imidazolylmethyl)phenyl}-2-propen-1-yl 2-methoxyethyl 1,4-dihydro-2,6-dimethyl-4-(3-nitrophenyl)pyridine-3,5-dicarboxylate

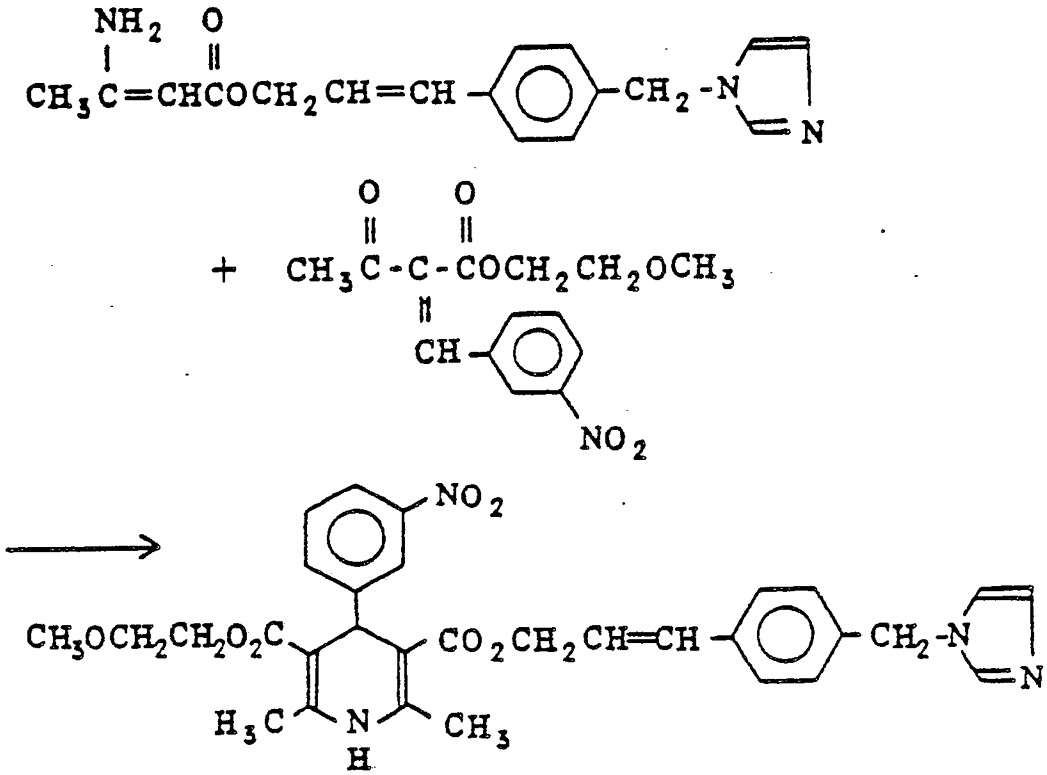

297 mg (1 mM) of 3-{4-(1-imidazolylmethyl)phenyl}-2-propen-1-yl 3-aminocrotonate and 293 mg (1 mM) of 2-methoxyethyl 2-(3-nitrobenzylidene)acetoacetate were dissolved in 5 ml of toluene and refluxed for six hours. After cooling, the crystals produced were filtered off and recrystallized from methanol to obtain 502 mg of the captioned compound. Yield: 87.6% of theory

Melting point (°C) 115.1 ~ 116.9

NMR δ$CDCl_3$ 2.37 (s, 3H), 2.39 (s, 3H), 3.29 (s, 3H), 3.46 - 3.58 (m, 2H), 4.09 - 4.24 (m, 2H), 4.64 (dd, 1H, J = 13Hz, 6Hz), 4.73 (dd, 1H, J = 13Hz, 6Hz), 5.11 (s, 2H), 5.15 (s, 1H), 5.85 (s, 1H), 6.21 (dt, 1H, J = 15Hz, 6Hz), 6.49 (d, 1H, J = 15Hz), 6.91 (s, 1H), 7.11 (d, 2H, J = 8Hz), 7.32 (d, 2H, J = 8Hz), 7.35 (t, 1H, J = 8Hz), 7.55 (s, 1H), 7.67 (d, 1H, J = 8Hz), 7.97 (dd, 1H, J = 8Hz, 2Hz), 8.14 (t, 1H, J = 2Hz).

IR ($cm^{-1}$) $\nu$co 1700

$\nu no_2$ 1530, 1350

High mass Molecular formula $C_{31}H_{32}N_4O_7$

| | |
|---|---|
| Found | 572.22791 |
| Calcd. | 572.22706 |

Example 17

(E)-3-{4-(1-imidazolylmethyl)phenyl}-2-propen-1-yl methyl 1,4-dihydro-2,6-dimethyl-4-(3-trifluoromethylphenyl)pyridine-3,5-dicarboxylate

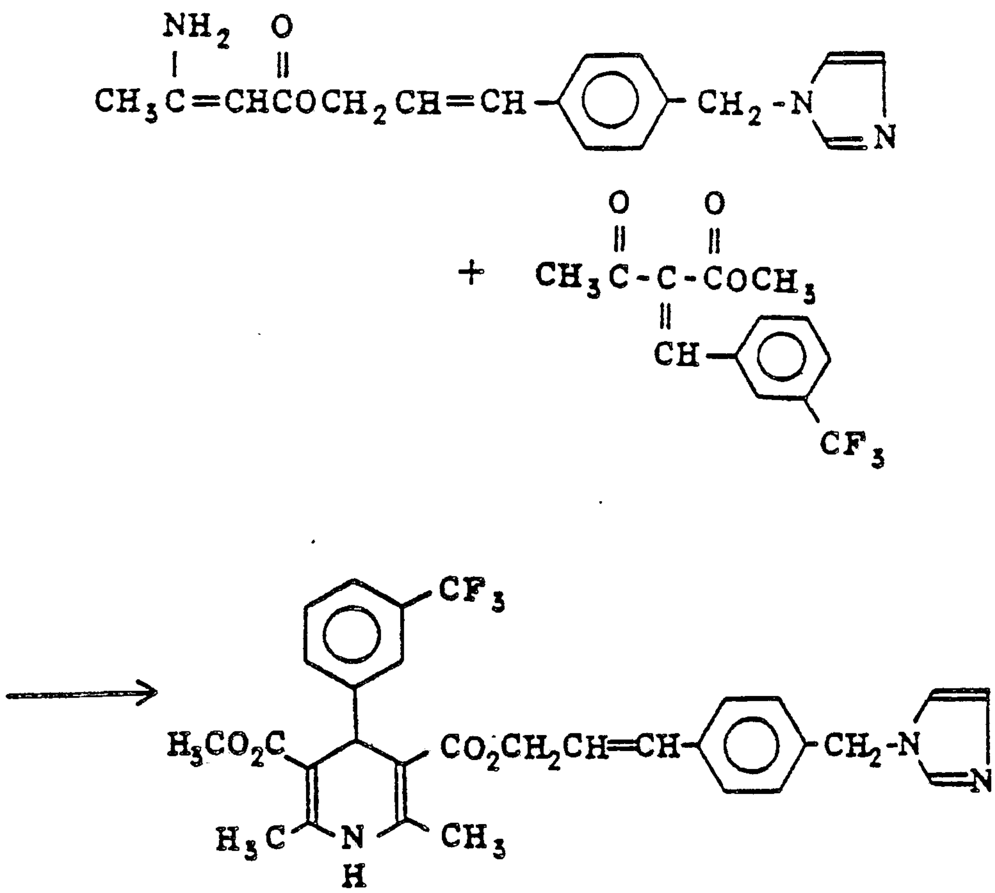

297 mg (1 mM) of 3-{4-(1-imidazolylmethyl)phenyl}-2-propen-1-yl 3-aminocrotonate and 272 mg (1 mM) of methyl 2-(3-trifluoromethylbenzylidene)acetoacetate were dissolved in 5 ml of toluene and refluxed for six hours. After cooling, the crystals produced were filtered off and recrystallized from methanol to obtain 394 mg of the captioned compound. Yield: 71.5% of theory

Melting point (°C) 170.9 ~ 171.5

NMR $\delta CDCl_3$ 2.36 (s, 3H), 2.38 (s, 3H), 3.63 (s, 3H), 4.65 (dd, 1H, J=13Hz, 6Hz), 4.74 (dd, 1H, J=13Hz, 6Hz), 5.08 (s, 1H), 5.10 (s, 2H), 5.71 (s, 1H), 6.20 (dt, 1H, J=16Hz, 6Hz), 6.47 (d, 1H, J=16Hz), 6.90 (s, 1H), 7.09 (s, 1H), 7.10 (d, 2H, J=8Hz), 7.30 (t, 1H, J=8Hz), 7.31 (d, 2H, J=8Hz), 7.38 (d, 1H, J=8Hz), 7.46 (d, 1H, J=8Hz), 7.53 (s, 1H), 7.55 (s, 1H).

IR ($cm^{-1}$) $\nu co$ 1695

High mass Molecular formula $C_{30}H_{28}F_3N_3O_4$

| | |
|---|---|
| Found | 551.20243 |
| Calcd. | 551.20315 |

Example 18

(E)-3-{4-(1-imidazolylmethyl)phenyl}-2-propen-1-yl methyl 1,4-dihydro-2,6-dimethyl-4-(2-cyanophenyl)-pyridine-3,5-dicarboxylate

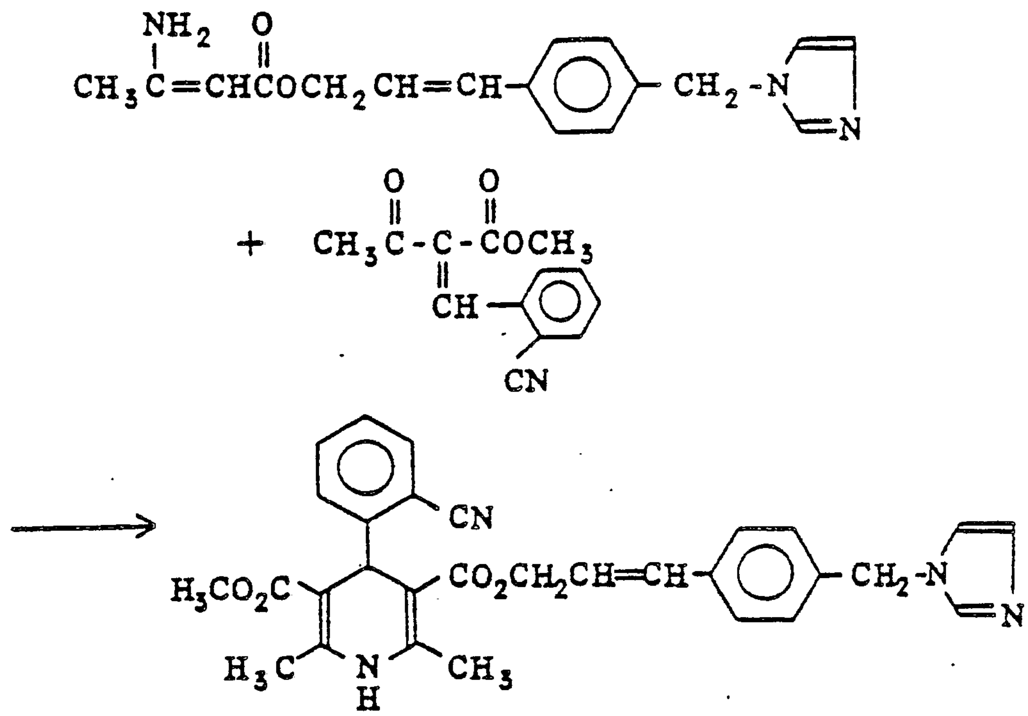

297 mg (1 mM) of 3-{4-(1-imidazolylmethyl)phenyl}propen-1-yl 3-aminocrotonate and 229 mg (1 mM) of methyl 2-(2-cyanobenzylidene)acetoacetate were dissolved in 5 ml of toluene and refluxed for six hours. After cooling, the crystals produced were filtered off and recrystallized from methanol to obtain 402 mg of the captioned compound. Yield: 79.1% of theory

Melting point (°C) 166.9 ~ 169.5

NMR $\delta CDCl_3$ 2.35 (s, 3H), 2.37 (s, 3H), 3.64 (s, 3H), 4.67 (dd, 1H, J=13Hz, 6Hz), 4.73 (dd, 1H, J=13Hz, 6Hz), 5.10 (s, 2H), 5.35 (s, 1H), 5.77 (s, 1H), 6.26 (dt, 1H, J=16Hz, 6Hz), 6.38 (d, 1H, J=16Hz), 6.90 (s, 1H), 7.08 (d, 2H, J=8Hz), 7.10 (s, 1H), 7.17 (td, 1H, J=8Hz, 2Hz), 7.30 (d, 2H, J=8Hz), 7.41-7.50 (m, 3H), 7.54 (s, 1H).

IR ($cm^{-1}$) $\nu_{cn}$ 2230

$\nu_{co}$ 1700

High mass Molecular formula $C_{30}H_{28}N_4O_4$

| | |
|---|---|
| Found | 508.21187 |
| Calcd. | 508.21102 |

Example 19

5-{4-(1-imidazolylmethyl)phenyl}-(E)-2,(E)-4-pentadien-1-yl methyl 1,4-dihydro-2,6-dimethyl-4-(3-nitrophenyl)pyridine-3,5-dicarboxylate

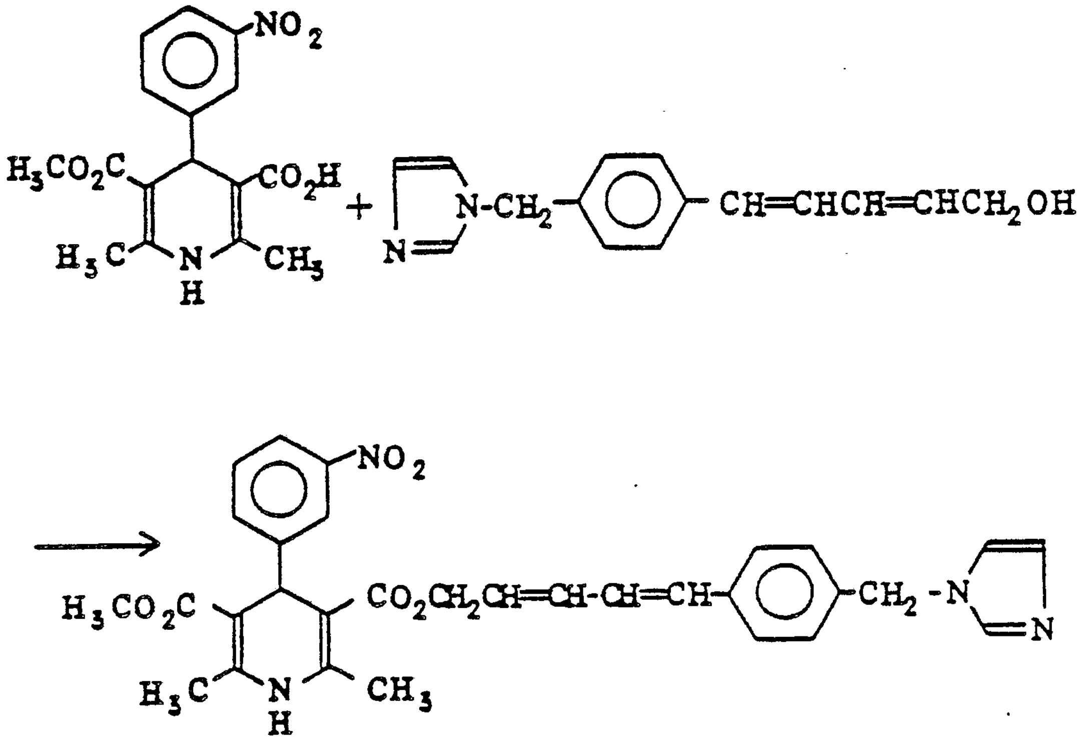

332 mg (1 mM) of 1,4-dihydro-2,6-dimethyl-5-methoxycarbonyl-4-(3-nitrophenyl)pyridine-3-carboxylic acid together with 240 mg (1 mM) of 5-{4-(1-imidazolylmethyl)phenyl}-(E)-2, (E)-4-pentadien-1-ol, 248 mg (1,2 mM) of dicyclohexylcarbodiimide and 134 mg (1.1 mM) of 4-N,N-dimethylaminopyridine were dissolved in 5 ml of toluene, while heating and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 387 mg of the captioned compound. Yield: 68.4% of theory

Melting point (°C) 179 (dec.)

NMR $\delta CDCl_3$ 2.377 (s, 3H), 2.388 (s, 3H), 3.644 (s, 3H), 4.580 (dd, 1H, J=14Hz, 7Hz), 4.700 (dd, 1H, J=14Hz, 7Hz), 5.129 (s, 1H), 5.155 (s, 2H), 5.825 (s, 1H), 5.850 (dt, 1H, J=15Hz, 7Hz), 6.280 (dd, 1H, J=15Hz, 13Hz), 6.470 (d, 1H, J=16Hz), 6.735 (dd, 1H, J=16Hz, 13Hz), 6.955 (s, 1H), 7.142 (d, 2H, J=8.4Hz), 7.167 (s, 1H), 7.377 (t, 1H, J=7.5Hz), 7.396 (d, 2H, J=8.4Hz), 7.635 (dt, 1H, J=7.5Hz, 2Hz), 7.820 (s, 1H), 8.000 (ddd, 1H, J=7.5Hz, 2Hz, 1Hz), 8.130 (dd, 1H, J=2Hz, 1Hz).

IR ($cm^{-1}$) $\nu_{co}$ 1700

$\nu_{no2}$ 1528, 1350 High mass Molecular formula $C_{31}H_{30}N_4O_6$

| | |
|---|---|
| Found | 554.21709 |
| Calcd. | 554.21650 |

Example 20

(E)-3-{2-(1-imidazolylmethyl)phenyl}-2-propen-1-yl methyl 1,4-dihydro-2,6-dimethyl-4-(3-nitrophenyl)-pyridine-3,5-dicarboxylate

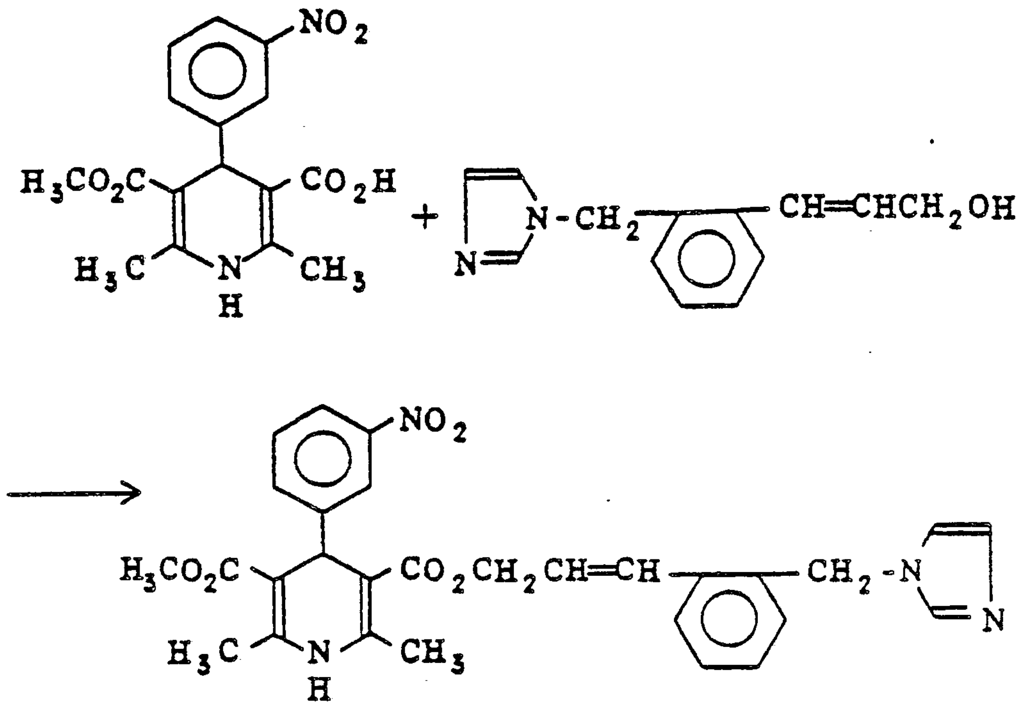

332 mg (1 mM) of 1,4-dihydro-2,6-dimethyl-5-methoxycarbonyl-4-(3-nitrophenyl)pyridine-3-carboxylic acid together with 215 mg (1 mM) of (E)-3-{2-(1-imidazolylmethyl)-phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 528 mg of the captioned compound. Yield: 100% of theory

Melting point (°C) 182 ~ 182.5

NMR $\delta CDCl_3$ 2.38 (s, 3H), 2.39 (s, 3H), 3.64 (s, 3H), 4.72 (d, 2H, J=6Hz), 5.16 (s, 2H), 5.16 (s, 1H), 6.07 (s, 1H), 6.13 (dt, 1H, J=16Hz, 6Hz), 6.66 (d, 1H, J=16Hz), 6.90 (s, 1H), 7.03 (dd, 1H, J=8Hz, 1Hz), 7.13 (s, 1H), 7.26-7.40 (m, 2H), 7.35 (t, 1H, J=7Hz), 7.45 (dd, 1H, J=8Hz, 1Hz), 7.64 (dt, 1H, J=7Hz, 2Hz), 7.65 (s, 1H), 7.96 (ddd, 1H, J=7Hz, 2Hz, 1Hz), 8.13 (dd, 1H, J=2Hz, 1Hz).

IR ($cm^{-1}$) $\nu$co 1702

$\nu no_2$ 1530, 1348

High mass Molecular formula $C_{29}H_{28}N_4O_6$

| | |
|---|---|
| Found | 528.20097 |
| Calcd. | 528.20085 |

Example 21

(E)-3-{3-(1-imidazolylmethyl)phenyl}-2-propen-1-yl methyl 1,4-dihydro-2,6-dimethyl-4-(3-nitrophenyl)-pyridine-3,5-dicarboxylate

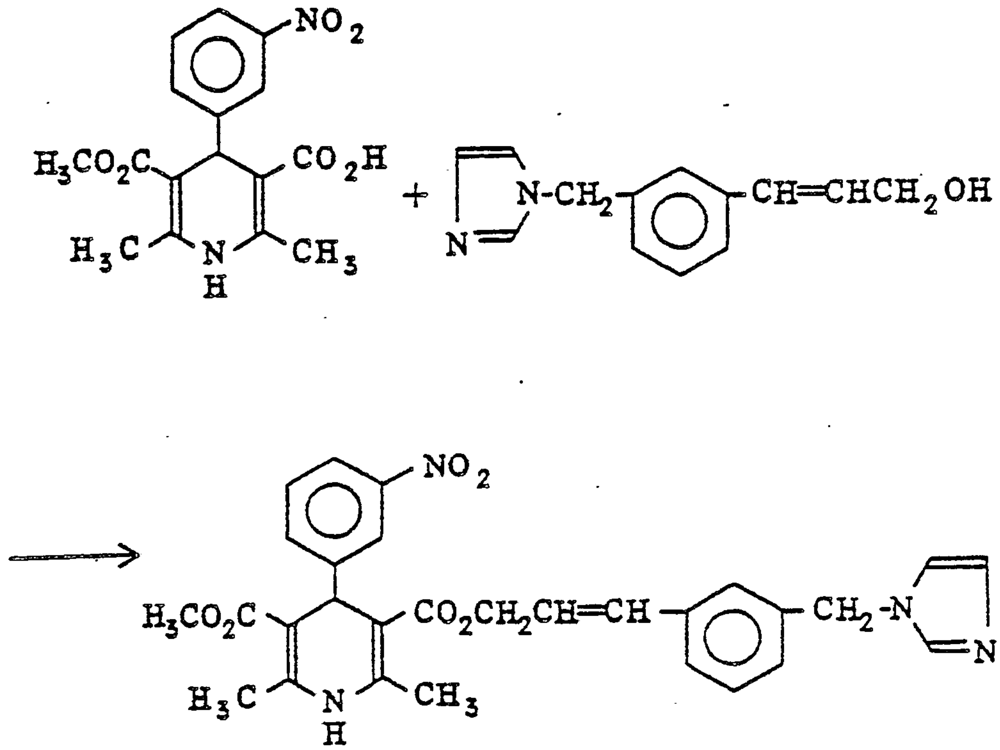

332 mg (1 mM) 1,4-dihydro-2,6-dimethyl-5-methoxycarbonyl-4-(3-nitrophenyl)pyridine-3-carboxylic acid together with 215 mg (1 mM) of (E)-3-{3-(1-imidazolylmethyl)phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the resiude was purified by chromatography on a silica gel column to obtain 481 mg of the captioned compound. Yield: 87% of theory

Melting point (°C) 190 ~ 191.5

NMR $\delta CDCl_3$ 2.38 (s, 3H), 2.40 (s, 3H), 3.64 (s, 3H), 4.63 (dd, 1H, J=13.2Hz, 7Hz), 4.79 (dd, 1H, J=13.2Hz, 7Hz), 5.13 (s, 1H), 5.19 (s, 1H), 5.84 (s, 1H), 6.22 (dt, 1H, J=16Hz, 7Hz), 6.45 (d, 1H, J=16Hz), 7.03 (s, 1H), 7.08 (dt, 1H, J=7Hz, 3Hz),. 7.16 (s, 1H), 7.21 (s, 1H), 7.31 (dt, 1H, J=7Hz, 3Hz), 7.34 (t, 1H, J=7Hz), 7.35 (t, 1H, J=7.8Hz), 7.63 (dt, 1H, J=7.8Hz, 2Hz), 7.91 (s, 1H), 7.98 (ddd, 1H, J=7.8Hz, 2Hz, 1Hz), 8.15 (dd, 1H, J=2Hz, 1Hz).

IR ($cm^{-1}$) $\nu$co 1702

$\nu no_2$ 1532, 1352

High mass Molecular formula $C_{29}H_{28}N_4O_6$

| | |
|---|---|
| Found | 528.20191 |
| Calcd. | 528.20085 |

Example 22

(E)-3-{4-(1-imidazolylmethyl)phenyl}-2-propen-1-yl methyl 1,4-dihydro-2,6-dimethyl-4-(3-pyridyl)pyridine-3,5-dicarboxylate

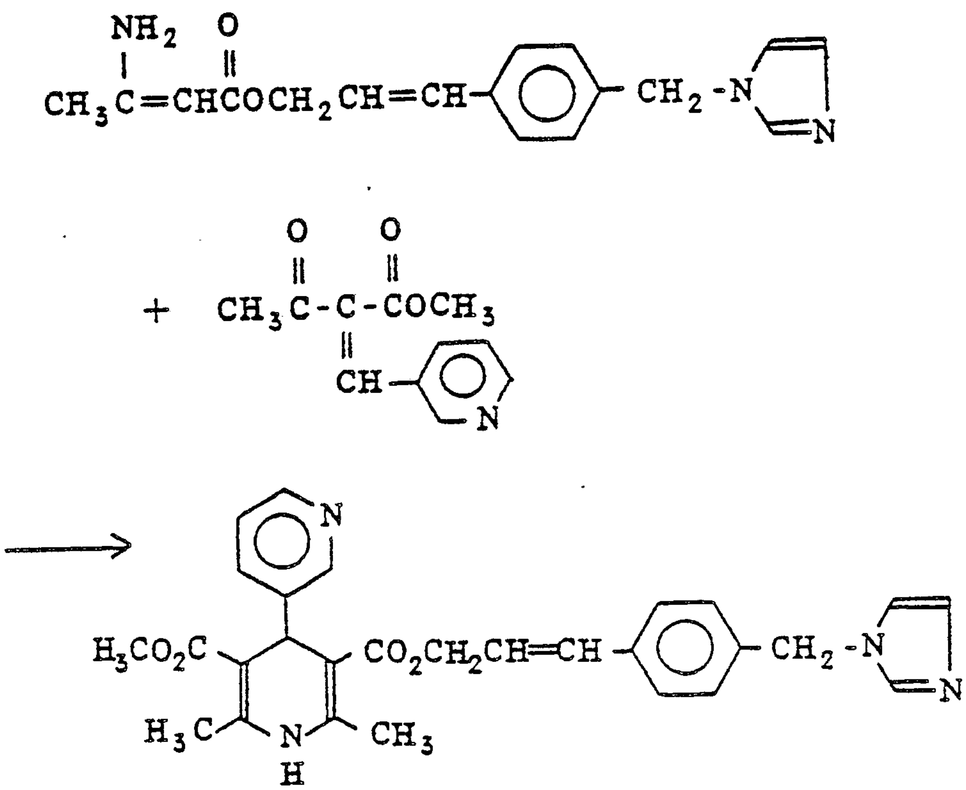

297 mg (1 mM) of 3-{4-(1-imidazolylmethyl)phenyl}-2-propen-1-yl 3-aminocrotonate and 205 mg (1 mM) of methyl 2-(3-pyridylidene)acetoacetate were dissolved in 5 ml of toluene and refluxed for six hours. After cooling, the crystals produced were filtered off and recrystallized from methanol to obtain 378 mg of the captioned compound. Yield: 78% of theory

Melting point (°C) 146 (dec.)

NMR δ$CDCl_3$ 2.35 (s, 3H), 2.37 (s, 3H), 3.64 (s, 3H), 4.68 (dd, 1H, J=15Hz, 6Hz), 4.74 (dd, 1H, J=15Hz, 6Hz), 5.03 (s, 1H), 5.12 (s, 2H), 6.22 (dt, 1H, J=16Hz, 6Hz), 6.31 (s, 1H), 6.48 (d, 1H, J=16Hz), 6.92 (s, 1H), 7.11 (s, 1H), 7.11 (d, 2H, J=8.7Hz), 7.16 (dd, 1H, J=7.5Hz, 4.5Hz), 7.33 (d, 2H, J=8.7Hz), 7.62 (s, 1H), 7.63 (dt, 1H, J=7.5Hz, 1.8Hz), 8.37 (dd, 1H, J=4.5Hz, 1.8Hz), 8.55 (d, 1H, J=1.8Hz).

IR ($cm^{-1}$) νco 1694

High mass Molecular formula $C_{28}H_{28}N_4O_4$

| | |
|---|---|
| Found | 484.21145 |
| Calcd. | 484.21102 |

Example 23

(E)-3-{4-(1-imidazolylmethyl)phenyl}-2-propen-1-yl methyl 1,4-dihydro-2,6-dimethyl-4-(4-quinolyl)pyridine-3,5-dicarboxylate

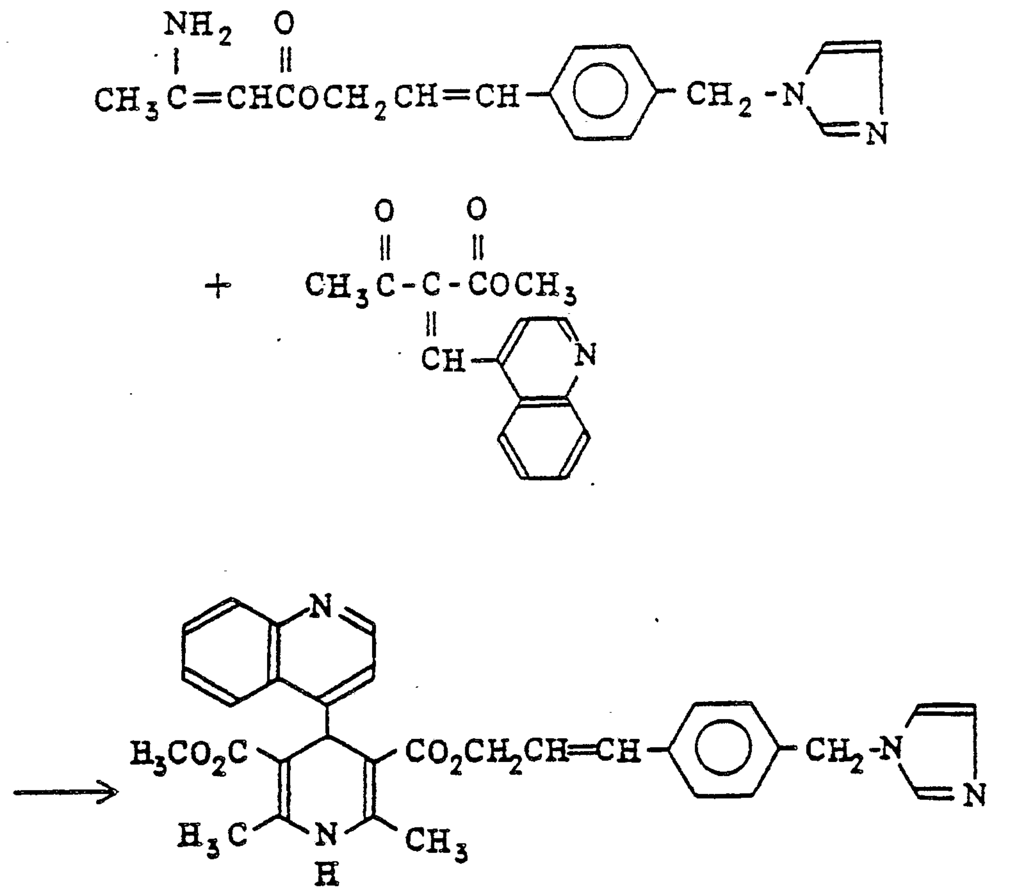

297 mg (1 mM) of 3-{4-(1-imidazolylmethyl)phenyl}-2-propen-1-yl 3-aminocrotonate and 255 mg (1 mM) of 2-(4-quinolylidene)acetoacetate were dissolved in 5 ml of toluene and refluxed for 15 hours. After cooling, the crystals produced were filtered off and recrystallized from methanol to obtain 371 mg of the captioned compound. Yield: 69% of theory

Melting point (°C) 122.5 (dec.)

NMR δ$CDCl_3$ 2.40 (s, 3H), 2.43 (s, 3H), 3.41 (s, 3H), 4.47 (dd, 1H, J=13.5Hz, 6Hz), 4.48 (dd, 1H, J=13.5Hz, 6Hz), 5.16 (s, 2H), 5.78 (dt, 1H, J=15.6Hz, 6Hz), 5.87 (s, 1H), 6.09 (s, 1H), 6.19 (d, 1H, J=15.6Hz), 6.97 (s, 1H), 7.11 (d, 2H, J=8.7Hz), 7.15 (d, 2H, J=8.7Hz), 7.17 (s, 1H), 7.51 (dt, 1H, J=7.8Hz, 1.8Hz), 7.52 (d, 1H, J=3.9Hz), 7.63 (dt, 1H, J=7.8Hz, 1.8Hz), 7.77 (s, 1H), 8.12 (dd, 1H, J=7.8Hz, 1Hz), 8.60 (dd, 1H, J=7.8Hz, 1Hz), 8.81 (d, 1H, J=3.9Hz), IR ($cm^{-1}$) $\nu$co 1700

High mass Molecular formula $C_{32}H_{30}N_4O_4$

| | |
|---|---|
| Found | 534.22834 |
| Calcd. | 534.22667 |

Example 24

(E)-3-{4-{1-(1-imidazolyl)ethyl}phenyl}-2-propen-1-yl methyl 1,4-dihydro-2,6-dimethyl-4-(3-nitrophenyl)-pyridine-3,5-dicarboxylate

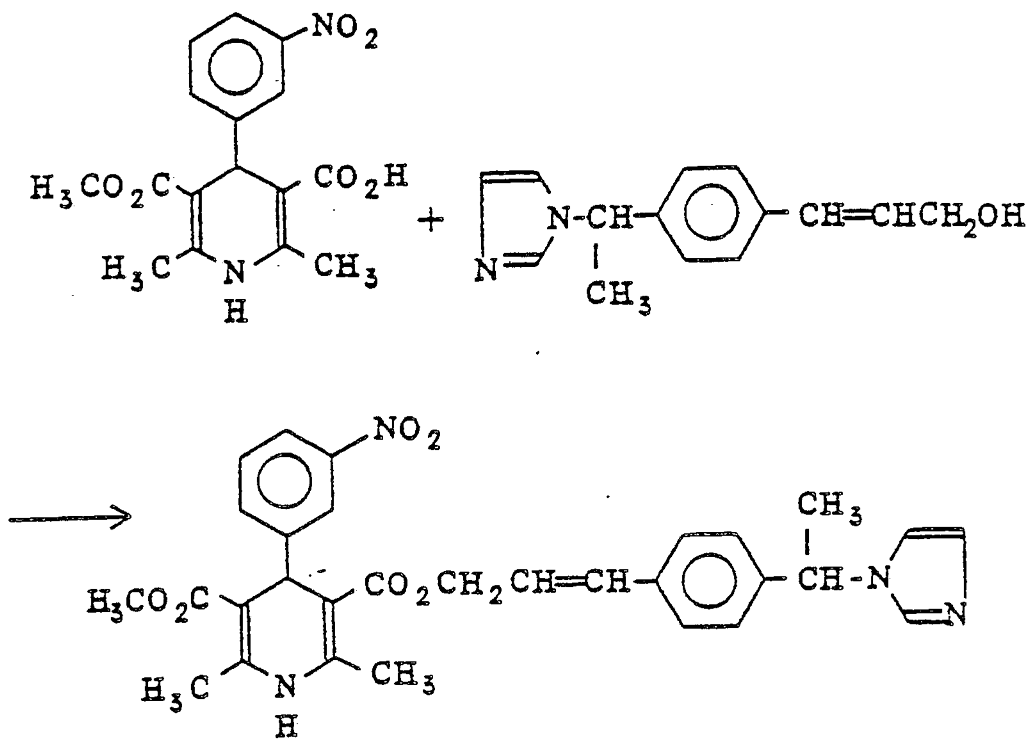

332 mg (1 mM) of 1,4-dihydro-2,6-dimethyl-5-methoxycarbonyl-4-(3-nitrophenyl)pyridine-3-carboxylic acid together with 228 mg (1 mM) of (E)-3-[4-{1-(1-imidazolyl) ethyl}phenyl]-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 519 mg of the captioned compound. Yield: 95.7% of theory

Melting point (°C) 107 ~ 109

NMR $\delta CDCl_3$ 1.87 (d, 3H, J=7.8Hz), 2.37 (s, 3H), 2.39 (s, 3H), 3.63 (s, 3H), 4.65 (dd, 1H, J=13Hz, 6Hz), 4.75 (dd, 1H, J=13Hz, 6Hz), 5.13 (s, 1H), 5.30 (s, 1H), 5.35 (q, 1H, J=7.8Hz), 6.22 (dt, 1H, J=15Hz, 6Hz), 6.50 (d, 1H, J=15Hz), 6.95 (s, 1H), 7.09 (s, 1H), 7.11 (d, 2H, J=8.4Hz), 7.31 (d, 2H, J=8.4Hz), 7.34 (t, 1H, J=8.8Hz), 7.63 (s, 1H), 7.64 (dt, 1H, J=8.8Hz, 2Hz), 7.98 (ddd, 1H, J=8.8Hz, 2Hz, 1Hz), 8.13 (dd, 1H, J=2Hz, 1Hz).

IR ($cm^{-1}$) $\nu co$ 1698

$\nu no_2$ 1528, 1350

High mass Molecular formula $C_{30}H_{30}N_4O_6$

| | |
|---|---|
| Found | 542.21549 |
| Calcd. | 542.21650 |

Example 25

(E)-3-{6-(1-imidazolylmethyl)pyridine-2-yl}-2-propen-1-yl methyl 1,4-dihydro-2,6-dimethyl-4-(3-nitrophenyl)-pyridine-3,5-dicarboxylate

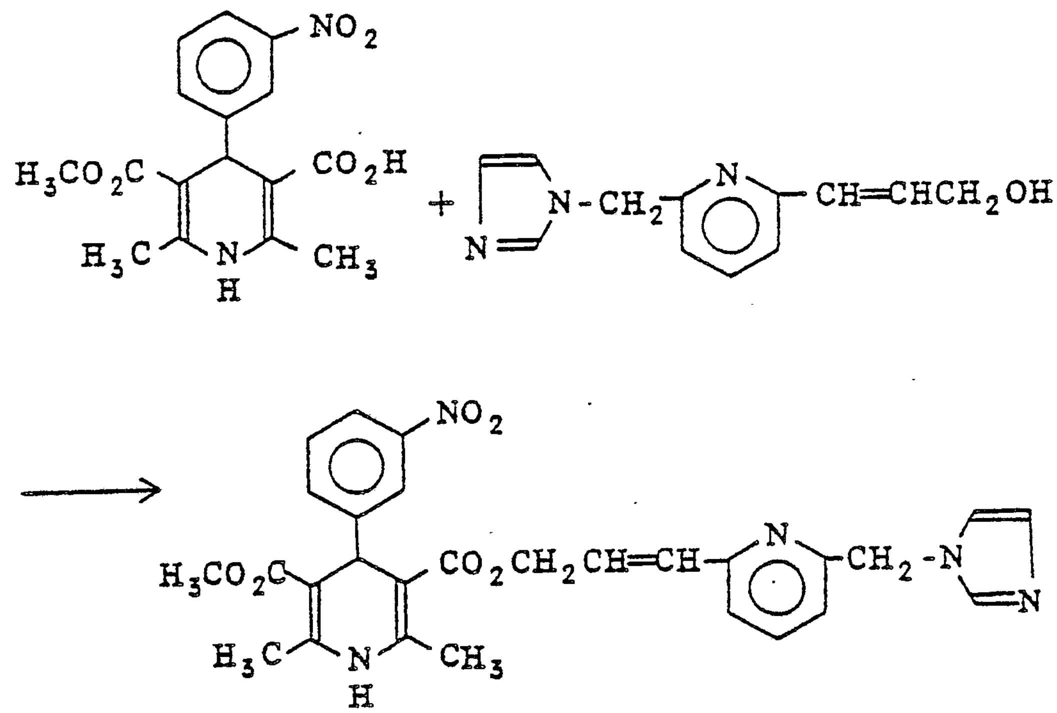

332 mg (1 mM) of 1,4-dihydro-2,6-dimethyl-5-methoxycarbonyl-4-(3-nitrophenyl)pyridine-3-carboxylic acid together with 215 mg (1 mM) of (E)-3-{6-(1-imidazolylmethyl)pyridin-2-yl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 506 mg of the captioned compound. Yield: 95.7% of theory

Meleting point (°C) 198.5 ~ 199.1

NMR $\delta CDCl_3$ 2.38 (s, 3H), 2.42 (s, 3H), 3.65 (s, 3H), 4.72 (dd, 1H, J=14Hz, 6Hz), 4.84 (dd, 1H, J=14Hz, 6Hz), 5.16 (s, 1H), 5.26 (s, 1H), 6.10 (s, 1H), 6.50 (d, 1H, J=15.6Hz), 6.74 (dt, 1H, J=15.6Hz, 6Hz), 6.84 (d, 1H, J=8Hz), 7.06 (s, 1H), 7.14 (d, 1H, J=8Hz), 7.16 (s, 1H), 7.37 (t, 1H, J=8Hz), 7.61 (t, 1H, J=8Hz), 7.66 (dt, 1H, J=8Hz, 1Hz), 7.80 (s, 1H), 7.98 (ddd, 1H, J=8.1Hz, 2Hz, 1Hz), 8.15 (dd, 1H, J=2Hz, 1H).

IR ($cm^{-1}$) $\nu co$ 1706

$\nu no_2$ 1534, 1354

High mass Molecular formula $C_{28}H_{27}N_5O_6$

| | |
|---|---|
| Found | 529.19715 |
| Calcd. | 529.19610 |

Example 26

2-[{4-(1-imidazolylmethyl)benzylideneamino}oxy]ethyl methyl 1,4-dihydro-2,6-dimethyl-4-(3-nitrophenyl)-pyridine3,5-dicarboxylate

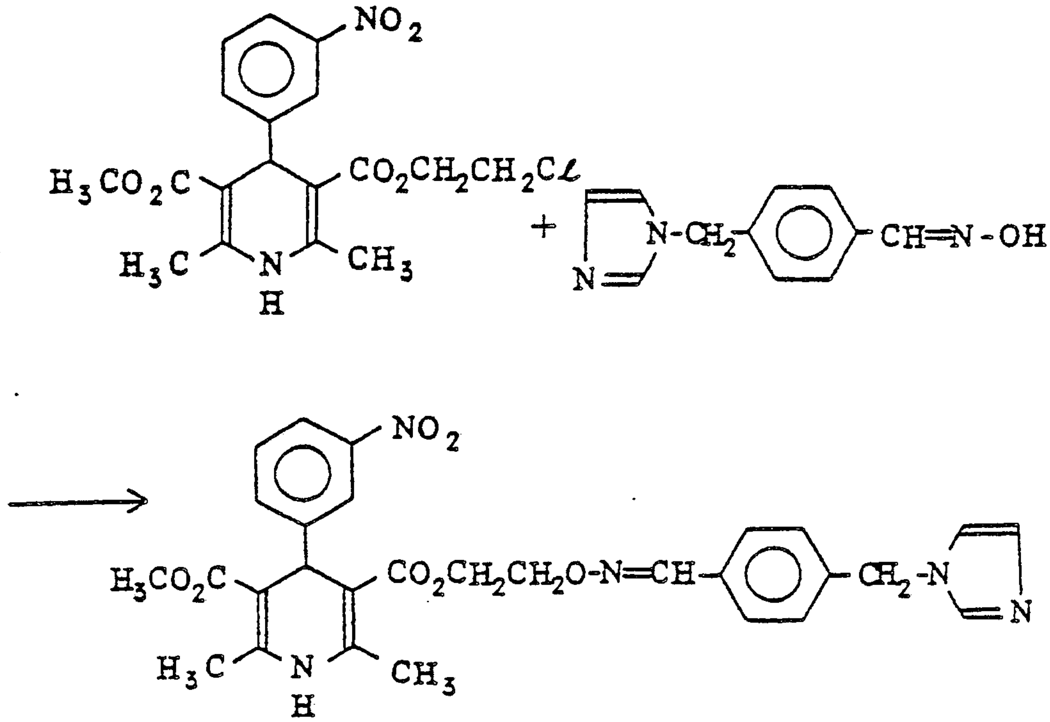

301 mg (0.763 mM) of 2-chloroethyl methyl 1,4-dihydro2,6-dimethyl-4-(3-nitrophenyl)pyridine-3,5-dicarboxylate and 155 mg (0.763 mM) of 4-(1-imidazolylmethyl)benzaldoxime were dissolved in 5 ml of dried acetonitrile, and then to the solution 5 mg (0.42 mM) of anhydrous potassium carbonate was added. After the mixture was heated under reflux for six hours, acetonitrile was distilled off and dichloromethane was added to the residue. After washing with water, the residue was dried over anhydrous sodium sulfate and dichloromethane was distilled off, and then the crystals were recrystallized from methanol to obtain 400 mg of the captioned compound. Yield: 94% of theory

Melting point (°C) 149 ~ 151

NMR $\delta CDCl_3$ 2.35 (s, 3H), 2.37 (s, 3H), 3.61 (s, 3H), 4.26-4.40 (m, 4H), 5.10 (s, 1H), 5.18 (s, 2H), 6.00 (s, 1H), 6.95 (s, 1H), 7.15 (s, 1H), 7.16 (d, 2H, J = 8.4Hz), 7.33 (t, 1H, J = 7.8Hz), 7.55 (d, 2H, J = 8.4Hz), 7.66 (dt, 1H, J = 7.8Hz, 2Hz), 7.78 (s, 1H), 7.96 (ddd, 1H, J = 7.8Hz, 2Hz, 1Hz), 8.00 (s, 1H), 8.10 (dd, 1H, J = 2Hz, 1Hz).

IR ($cm^{-1}$) $\nu co$ 1690

$\nu no_2$ 1532, 1354

High mass Molecular formula $C_{29}H_{29}N_5O_7$

| | |
|---|---|
| Found | 559.20644 |
| Calcd. | 559.20666 |

Example 27

di-(E)-3-{4-(1-imidazolylmethyl)phenyl}-2-propen-1-yl 1,4-dihydro-2,6-dimethyl-4-(3-nitrophenyl)pyridine-3,5-dicarboxylate

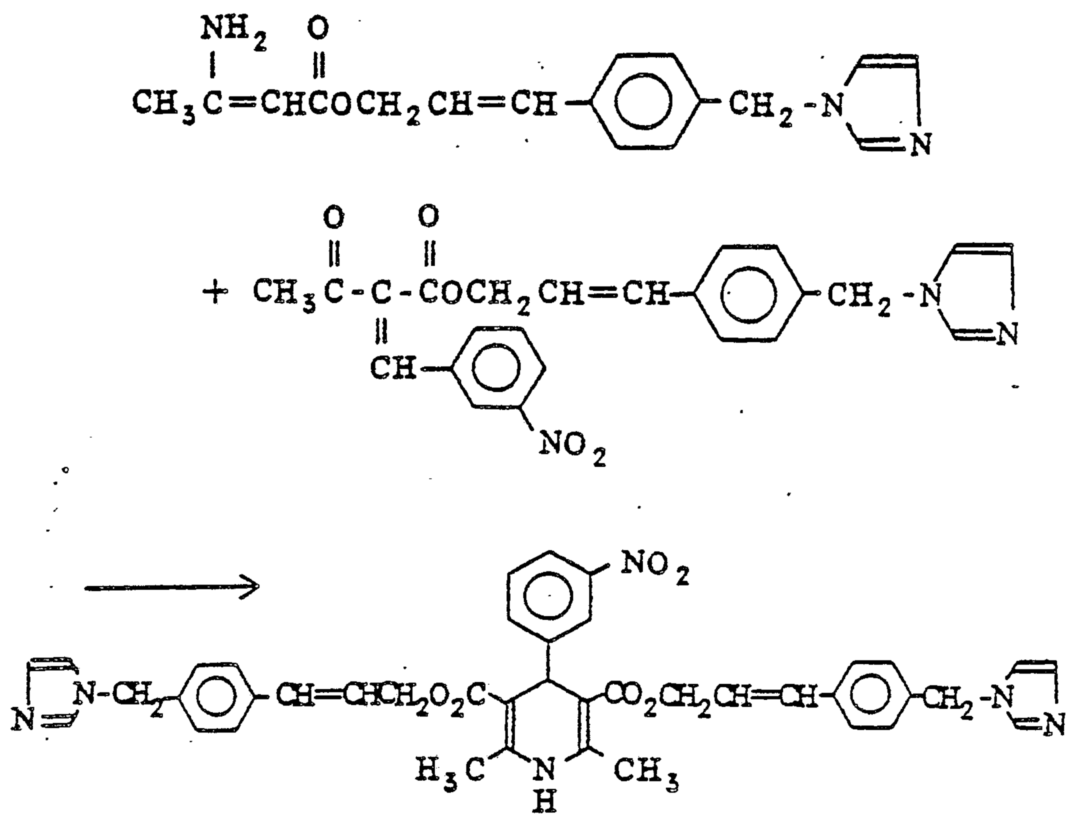

297 mg (1 mM) of 3-{4-{1-imidazolylmethyl)phenyl}-2-propen-1-yl 3-aminocrotonate and 431 mg (1 mM) of 3-{4-(1-imidazolylmethyl)phenyl}-propen-1-yl 2-(3-nitrobenzylidene)acetoacetate were dissolved in 5 ml of toluene and refluxed for six hours. After cooling, the crystals produced were filtered off and recrystallized from methanol to obtain 571 mg of the captioned compound. Yield: 80.4% of theory

Melting point (°C) 124.4 ~ 125.9

NMR $\delta CDCl_3$ 2.39 (s, 6H), 4.65 (ddd, 2H, J = 13Hz, 6Hz, 2Hz), 4.75 (ddd, 2H, J = 13Hz, 6Hz, 2Hz), 5.10 (s, 4H), 5.17 (s, 1H), 6.08 (bs, 1H), 6.20 (dt, 2H, J = 16Hz, 6Hz), 6.48 (d, 2H, J = 16Hz), 6.90 (s, 2H), 7.08 (d, 4H, J = 8Hz), 7.10 (s, 2H), 7.29 (d, 4H, J = 8Hz), 7.32 (t, 1H, J = 8Hz), 7.54 (s, 2H), 7.65 (d, 1H, J = 8Hz), 7.95 (dd, 1H, J = 8Hz, 2Hz), 8.16 (t, 1H, J = 2Hz).

IR ($cm^{-1}$) $\nu$co 1695

$\nu no_2$ 1530, 1350

FAB mass 711 (M + 1)

Example 28

6-(1-imidazolylmethyl)benzoxazol-2-yl-methyl methyl 1,4-dihydro-2,6-dimethyl-4-(3-nitrophenyl)pyridine-3,5-dicarboxylate

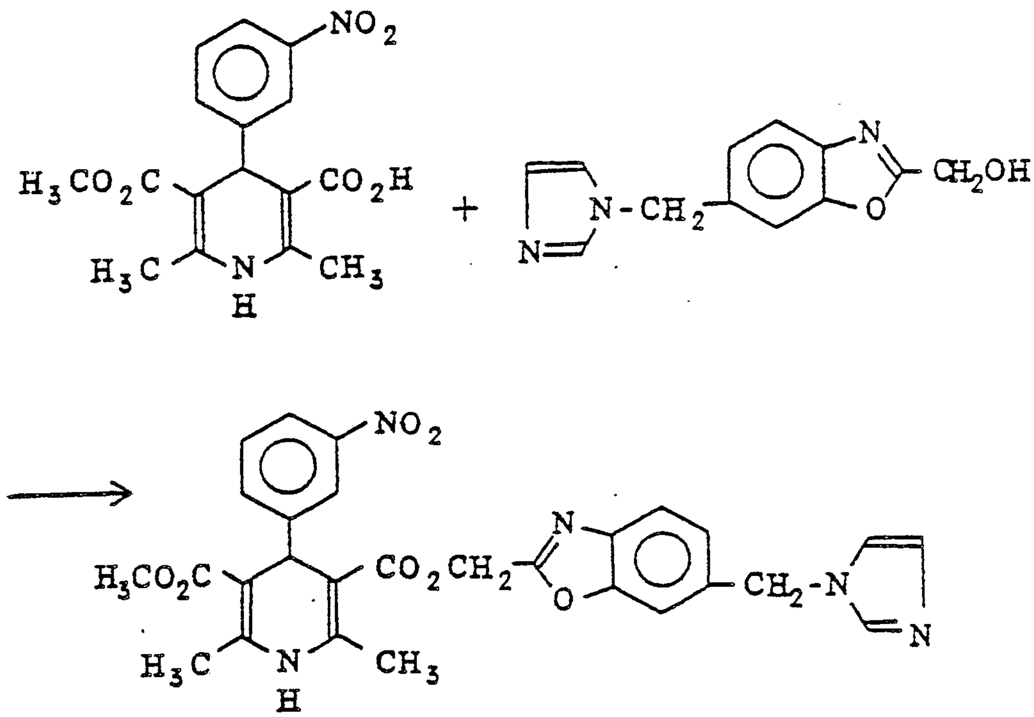

332 mg (1 mM) of 1,4-dihydro-2,6-dimethyl-5-methoxycarbonyl-4-(3-nitrophenyl)pyridine-3-carboxylic acid together with 229 mg (1 mM) of 2-hydroxymethyl-6-(1-imidazolylmethyl)benzoxazole 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 542 mg of the captioned compound. Yield: 99.9% of theory

NMR $\delta CDCl_3$ 2.37 (s, 3H), 2.41 (s, 3H), 3.62 (s, 3H), 5.13 (s, 1H), 5.20 (d, 1H, J=14Hz), 5.26 (s, 2H), 5.40 (d, 1H, J-14Hz), 6.59 (s, 1H), 6.97 (s, 1H), 7.14 (s, 1H), 7.19 (d, 1H, J=8Hz), 7.20 (s, 1H), 7.25 (t, 1H, J=8Hz), 7.60 (d, 1H, J=8Hz), 7.62 (s, 1H), 7.68 (d, 1H, J=8Hz), 7.89 (dd, 1H, J=8Hz, 2Hz), 8.08 (t, 1H, J=2Hz).

IR ($cm^{-1}$) $\nu co$ 1705

$\nu no_2$ 1525, 1345

High mass Molecular formula $C_{28}H_{25}N_5O_7$

| | |
|---|---|
| Found | 543.17657 |
| Calcd. | 543.17536 |

Example 29

(E)-4-{4-(1-imidazolylmethyl)phenyl}-3-buten-1-yl methyl 1,4-dihydro-2,6-dimethyl-4-(3-nitrophenyl)pyridine-3,5-dicarboxylate

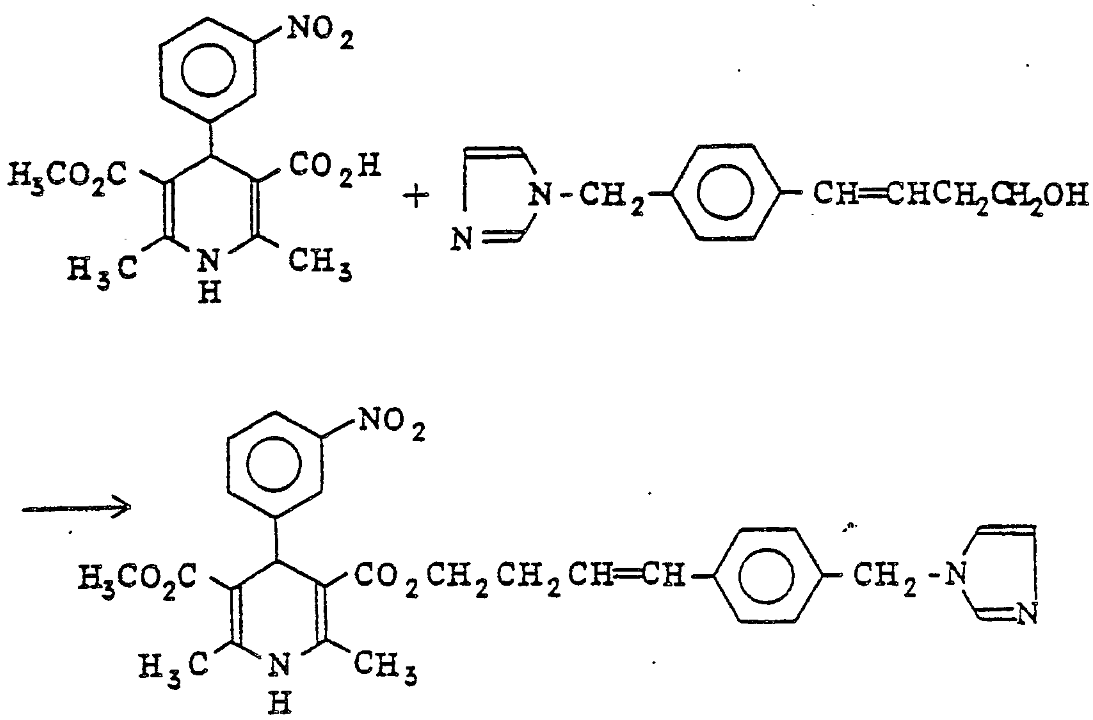

332 mg (1 mM) of 1,4-dihydro-2,6-dimethyl-5-methoxycarbonyl-4-(3-nitrophenyl)pyridine-3-carboxylic acid together with 228 mg (1 mM) of (E)-4-{4-(1-imidazolylmethyl)phenyl}-3-buten-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the resiude was purified by chromatography on a silica gel column to obtain 400 mg of the captioned compound. Yield: 73.8% of theory

Melting point (°C) 178.9 ~ 180.1

NMR $\delta CDCl_3$ 2.35 (s, 3H), 2.36 (s, 3H), 2.51 (dt, 2H, J=6Hz, 6Hz), 3.59 (s, 3H), 4.14 (dt, 1H, J=12Hz, 6Hz), 4.21 (dt, 1H, J=12Hz, 6Hz), 5.09 (s, 3H), 5.84 (s, 1H), 6.07 (dt, 1H, J=16Hz, 6Hz), 6.36 (d, 1H, J=16Hz), 6.91 (s, 1H), 7.08 (d, 2H, J=8Hz), 7.09 (s, 1H), 7.25 (d, 2H, J=8Hz), 7.29 (t, 1H, J=8Hz), 7.55 (s, 1H), 7.61 (d, 1H, J=8Hz), 7.93 (dd, 1H, J=8Hz, 2Hz), 8.09 (t, 1H, J=2Hz).

IR ($cm^{-1}$) $\nu co$ 1700

$\nu no_2$ 1530, 1345

High mass Molecular formula $C_{30}H_{30}N_4O_6$

| | |
|---|---|
| Found | 542.21571 |
| Calcd. | 542.21650 |

Example 30

Isopropyl (E)-3-{4-(1-imidazolylmethyl)phenyl}-2-propen-1-yl 1,4-dihydro-2,6-dimethyl-4-(3-nitrophenyl)-pyridine-3,5-dicarboxylate

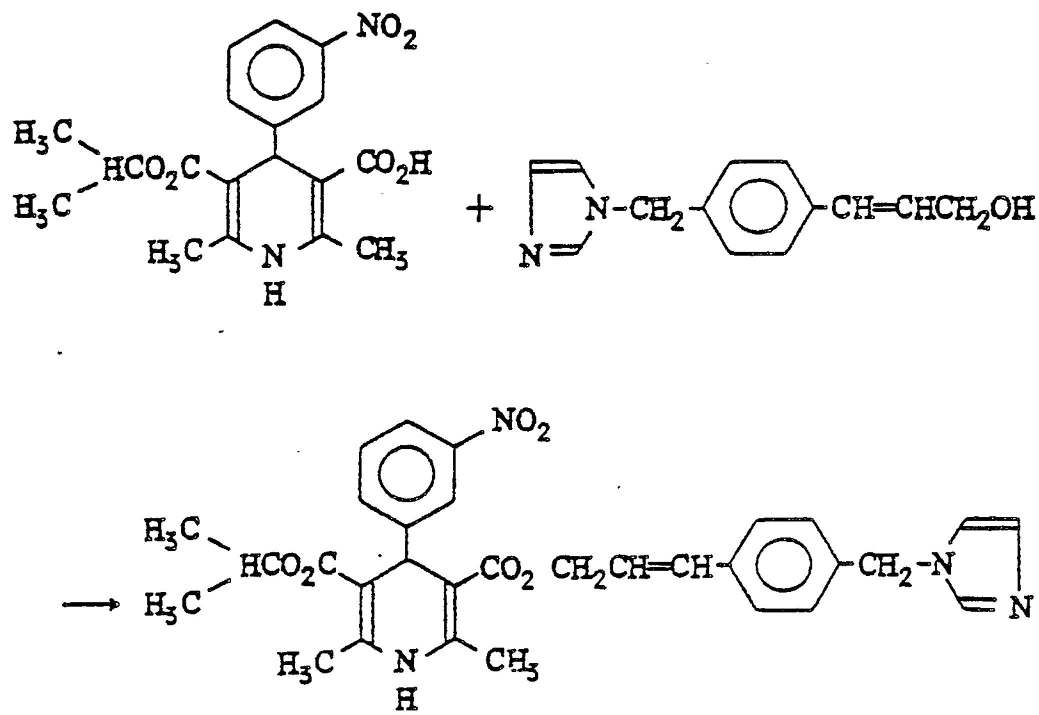

360 mg (1 mM) of 1,4-dihydro-2,6-dimethyl-5-isopropyloxycarbonyl-4-(3-nitrophenyl)pyridine-3-carboxylic acid together with 215 mg (1 mM) of (E)-3-{4-(1-imidazolylmethyl)phenyl}-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 541 mg of the captioned compound. Yield: 97.2% of theory

NMR $\delta CDCl_3$ 1.09 (d, 3H, J=6Hz), 1.25 (d, 3H, J=6Hz), 2.36 (s, 3H), 2.39 (s, 3H), 4.65 (dd, 1H, J=13Hz, 6Hz), 4.75 (dd, 1H, 13Hz, 6Hz), 4.95 (qq, 1H, J=6Hz, 6Hz), 5.11 (s, 3H), 5.80 (s, 1H), 6.20 (dt, 1H, J=16Hz, 6Hz), 6.49 (d, 1H, J=16Hz), 6.91 (s, 1H), 7.09 (s, 1H), 7.11 (d, 2H, J=8Hz), 7.30 (d, 2H, J=8Hz), 7.34 (t, 1H, J=8Hz), 7.55 (s, 1H), 7.64 (d, 1H, J=8Hz), 7.97 (dd, 1H, J=8Hz, 2Hz), 8.14 (t, 1H, J=2Hz).

IR ($cm^{-1}$) $\nu_{co}$ 1700

$\nu_{no_2}$ 1530, 1350

High mass Molecular formula $C_{31}H_{32}N_4O_6$

| | |
|---|---|
| Found | 556.23184 |
| Calcd. | 556.23214 |

Example 31

(E)-4-{4-(1-imidazolylmethyl)phenyl}-3-buten-2-yl methyl 1,4-dihydro-2,6-dimethyl-4-(3-nitrophenyl)pyridine-3,5-dicarboxylate

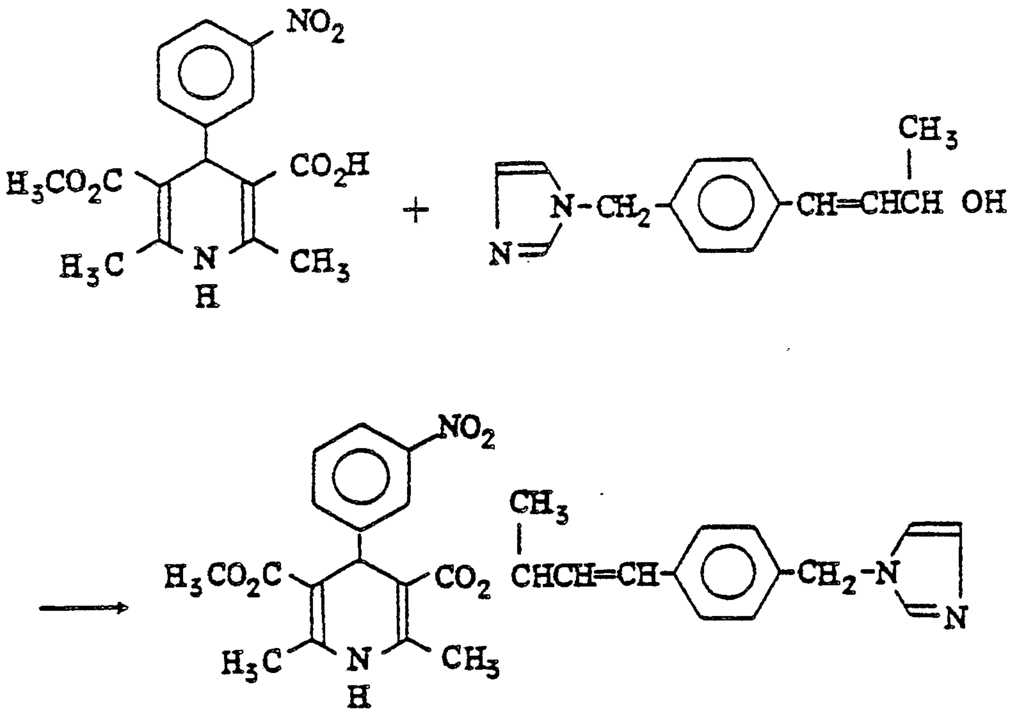

332 mg (1 mM) of 1,4-dihydro-2,6-dimethyl-5-methoxycarbonyl-4-(3-nitrophenyl)pyridine-3-carboxylic acid together with 228 mg (1 mM) of (E)-4-{4-(1-imidazolylmethyl)phenyl}-3-buten-2-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the resiude was purified by chromatography on a silica gel column. Two kinds of diastereomers were eluted by silica gel column chromatography with a eluate consisting of a mixture of hexane, ethyl acetate and methanol in a ratio of 5:5:1. The first eluted moiety is referred to as "Compound a", and the later eluted moiety is referred to as "Compound b".
Yield:

| | |
|---|---|
| "Compound a" | 220 mg |
| "Compound b" | 180 mg |

Compounds a and b have characteristics as shown below.

[Compound a]

Melting point (°C) 179.1 ~ 180.9
NMR $\delta CDCl_3$ 1.43 (d, 3H, J=6Hz), 2.36 (s, 3H), 2.38 (s, 3H), 3.65 (s, 3H), 5.09 (s, 2H), 5.11 (s, 1H), 5.48 (dq, 1H, J=6Hz, 6Hz), 5.85 (s, 1H), 5.99 (dd, 1H, J=16Hz, 6Hz), 6.25 (d, 1H, J=16Hz), 6.90 (s, 1H), 7.07 (d, 2H, J=8Hz), 7.08 (s, 1H), 7.18 (d, 2H, J=8Hz), 7.31 (t, 1H, J=8Hz), 7.55 (s, 1H), 7.64 (d, 1H, J=8Hz), 7.94 (dd, 1H, J=8Hz, 2Hz), 8.13 (t, 1H, J=2Hz)
IR ($cm^{-1}$) $\nu$co 1695
$\nu$no2 1530, 1350
High mass Molecular formula $C_{30}H_{30}N_4O_6$

| | |
|---|---|
| Found | 542.21648 |
| Calcd. | 542.21650 |

[Compound b]

NMR δCDCl₃ 1.27 (d, 3H, J=6Hz), 2.36 (s, 3H), 2.37 (s, 3H), 3.64 (s, 3H), 5.11 (s, 2H), 5.12 (s, 1H), 5.48 (dq, 1H, J=6Hz, 6Hz), 5.89 (s, 1H), 6.22 (dd, 1H, J=16Hz, 6Hz), 6.55 (d, 1H, J=16Hz), 6.90 (s, 1H), 7.10 (s, 1H), 7.11 (d, 2H, J=8Hz), 7.36 (d, 2H, J=8Hz), 7.38 (t, 1H, J=8Hz), 7.56 (s, 1H), 7.64 (d, 1H, J=8Hz), 8.00 (dd, 1H, J=8Hz, 2Hz), 8.14 (t, 1H, J=2Hz)
IR ($cm^{-1}$) $\nu_{co}$ 1695
$\nu_{no_2}$ 1535, 1350
High mass Molecular formula $C_{30}H_{30}N_4O_6$

| | |
|---|---|
| Found | 542.21708 |
| Calcd. | 542.21650 |

Example 32

3-{4-(1-imidazolylmethyl)phenyl-2-propen-1-yl methyl 1,4-dihydro-2,6-dimethyl-4-(3-nitrophenyl)pyridine-3,5-dicarboxylate

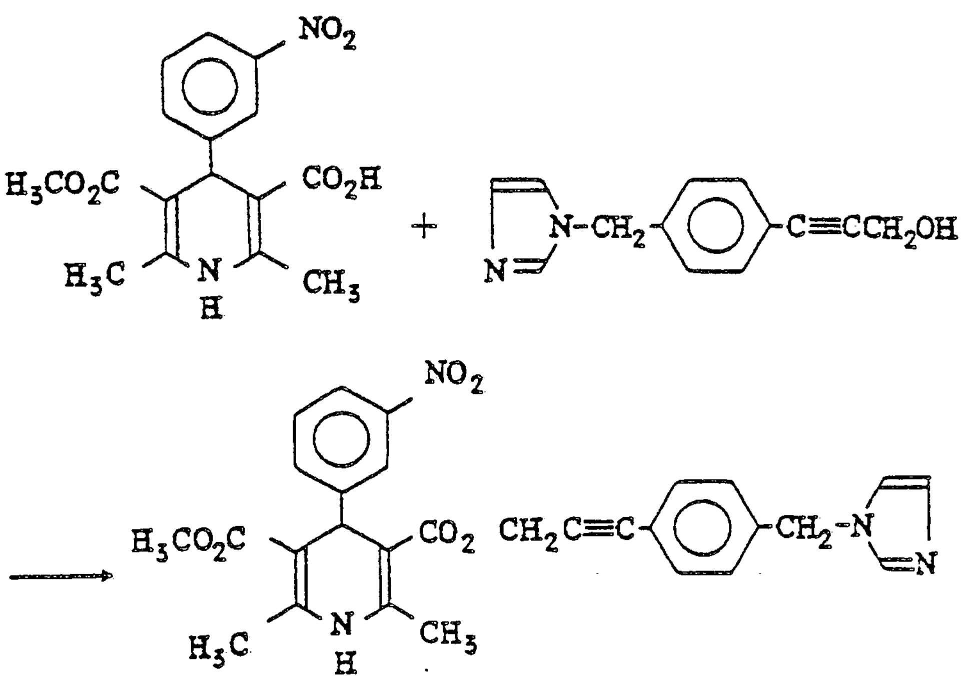

332 mg (1 mM) of 1,4-dihydro-2,6-dimethyl-5-methoxycarbonyl-4-(3-nitrophenyl)pyridine-3-carboxylic acid together with 212 mg (1 mM) of 3-{4-(1-imidazolylmethyl)phenyl}-2-propyn-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 421 mg of the captioned compound. Yield: 80% of theory
Melting point (°C) 143 - 144
NMR δCDCl₃ 2.38 (s, 3H), 2.40 (s, 3H), 3.64 (s, 3H), 4.82 (d, 1H, J=16Hz), 4.91 (d, 1H, J=16Hz), 5.14 (s, 1H), 5.18 (s, 2H), 5.96 (s, 1H), 6.94 (s, 1H), 7.12 (d, 2H, J=8Hz), 7.16 (s, 1H), 7.32 (t, 1H), 7.40 (d, 2H, J=8Hz), 7.68 (dt, 1H, J=8Hz, 2Hz), 7.82 (s, 1H), 7.97 (ddd, 1H, J=8Hz, 2Hz, 1Hz), 8.13 (dd, 1H, J=2Hz, 1Hz)
IR ($cm^{-1}$) $\nu_{co}$ 1700

$\nu_{NO_2}$ 1530, 1352

High mass  Molecular formula $C_{29}H_{26}N_4O_6$

| | |
|---|---|
| Found | 526.18449 |
| Calcd. | 526.18520 |

Example 33

(E)-5-{4-(1-imidazolylmethyl)phenyl}-pent-2-en-4-yne-1-yl methyl 1,4-dihydro-2,6-dimethyl-4-(3-nitrophenyl)-pyridine-3,5-dicarboxylate

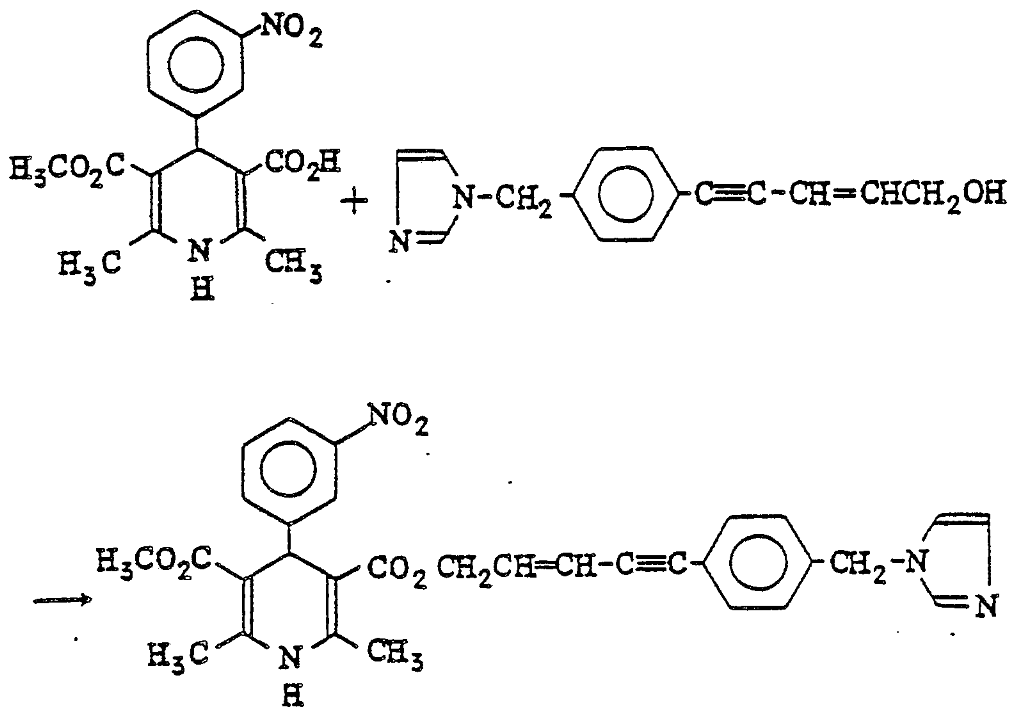

30 mg (0.089 mM) of 1,4-dihydro-2,6-dimethyl-5-methoxycarbonyl-4-(3-nitrophenyl)pyridine-3-carboxylic acid together with 21 mg (0.089 mM) of (E)-5-{4-(1-imidazolylmethyl)phenyl}-pent-2-en-4-yn-1-ol, 22 mg (0.107 mM) of dicyclohexylcarbodiimide and 12 mg (0.098 mM) of 4-N,N-dimethylaminopyridine were dissolved in 3 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 49 mg of the captioned compound. Yield: 87% of theory

Melting point (°C)  177 ~ 178

NMR $\delta CDCl_3$  2.38 (s, 3H), 2.40 (s, 3H), 3.66 (s, 3H), 4.61 (dd, 1H, J=15Hz, 6Hz), 4.68 (dd, 1H, J=15Hz, 6Hz), 5.12 (s, 1H), 5.23 (s, 2H), 5.78 (d, 1H, J=16Hz), 5.85 (s, 1H), 6.21 (dt, 1H, J=16Hz, 6Hz), 6.97 (s, 1H), 7.17 (d, 2H, J=8Hz), 7.22 (s, 1H), 7.39 (t, 1H, J=8Hz), 7.45 (d, 2H, J=8Hz), 7.64 (dt, 1H, J=8Hz, 2Hz), 8.01 (ddd, 1H, J=8Hz, 2Hz, 1Hz), 8.09-8.14 (m, 2H)

IR ($cm^{-1}$)  $\nu_{CO}$ 1690

$\nu_{NO_2}$ 1530, 1350

High mass  Molecular formula $C_{31}H_{28}N_4O_6$

| | |
|---|---|
| Found | 552.20148 |
| Calcd. | 552.20085 |

Example 34

(Z)-3-{4-(1-imidazolylmethyl)phenyl)-2-propen-1-yl methyl 1,4-dihydro-2,6-dimethyl-4-(3-nitrophenyl)-pyridine-3,5-dicarboxylate

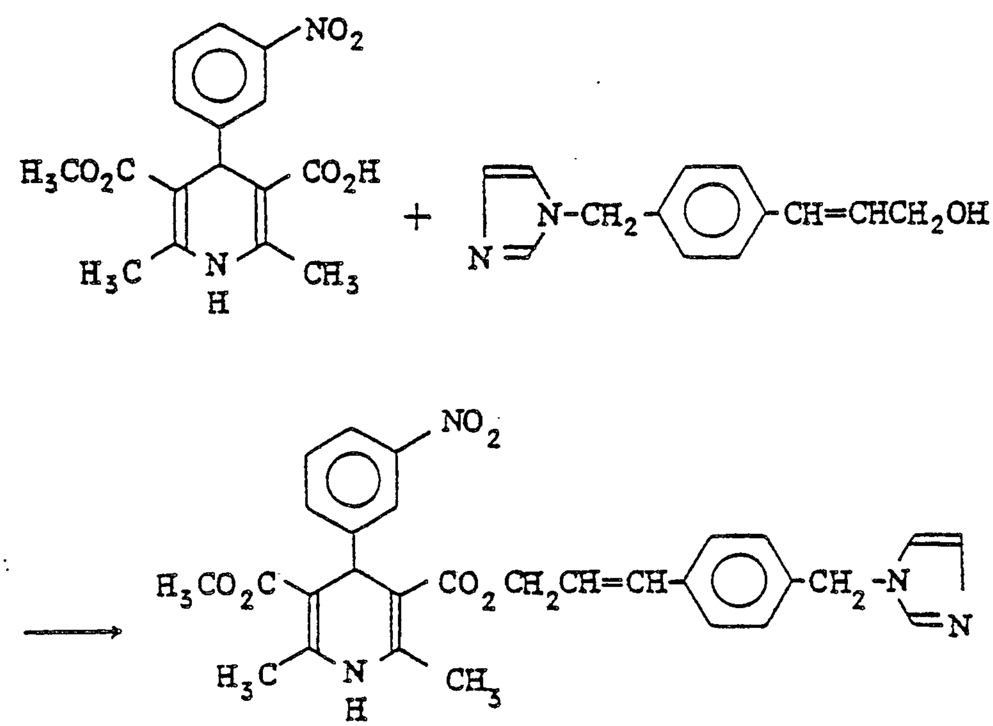

332 mg (1 mM) of 1,4-dihydro-2,6-dimethyl-5-methoxycarbonyl-4-(3-nitrophenyl)pyridine-3-carboxylic acid together with 215 mg (1 mM) of (Z)-3-{4-(1-imidazolylmethyl)phenyl)-2-propen-1-ol, 248 mg (1.2 mM) of dicyclohexylcarbodiimide and 134 mg (1.1 mM) of 4-N,N-dimethylaminopyridine were dissolved in 5 ml of toluene, while heating, and refluxed for six hours. The solution was cooled to room temperatures, and the crystals produced were filtered off. The filtrate was washed with water and dried over anhydrous sodium sulfate. After the solvent was distilled off, the residue was purified by chromatography on a silica gel column to obtain 528 mg of the captioned compound. Yield: 100% of theory

Melting point (°C) 75 ~ 77

NMR $\delta CDCl_3$ 2.37 (s, 6H), 3.64 (s, 3H), 4.77 (d, 2H, J=7Hz), 5.10 (s, 1H), 5.13 (s, 2H), 5.81 (dt, 1H, J=12Hz, 7Hz), 6.21 (s, 1H), 6.60 (d, 1H, J=12Hz), 6.93 (s, 1H), 7.10 (d, 2H, J=9Hz), 7.13 (s, 1H), 7.15 (d, 2H, J=9Hz), 7.35 (t, 1H, J=8.4Hz), 7.63 (ddd, 1H, J=8.4Hz, 2Hz, 1Hz), 7.66 (s, 1H), 7.97 (ddd, 1H, J=8.4Hz, 2Hz, 1Hz), 8.11 (t, 1H, J=2Hz)

IR ($cm^{-1}$) $\nu co$ 1698

$\nu no_2$ 1530, 1350

High mass Molecular formula $C_{29}H_{28}N_4O_6$

| | |
|---|---|
| Found | 528.20189 |
| Calcd. | 528.20085 |

Example 35

2-{4-(1-imidazolylmethyl)benzylideneamino}ethyl methyl 1,4-dihydro-2,6-dimethyl-4-(3-nitrophenyl)pyridine-3,5-dicarboxylate

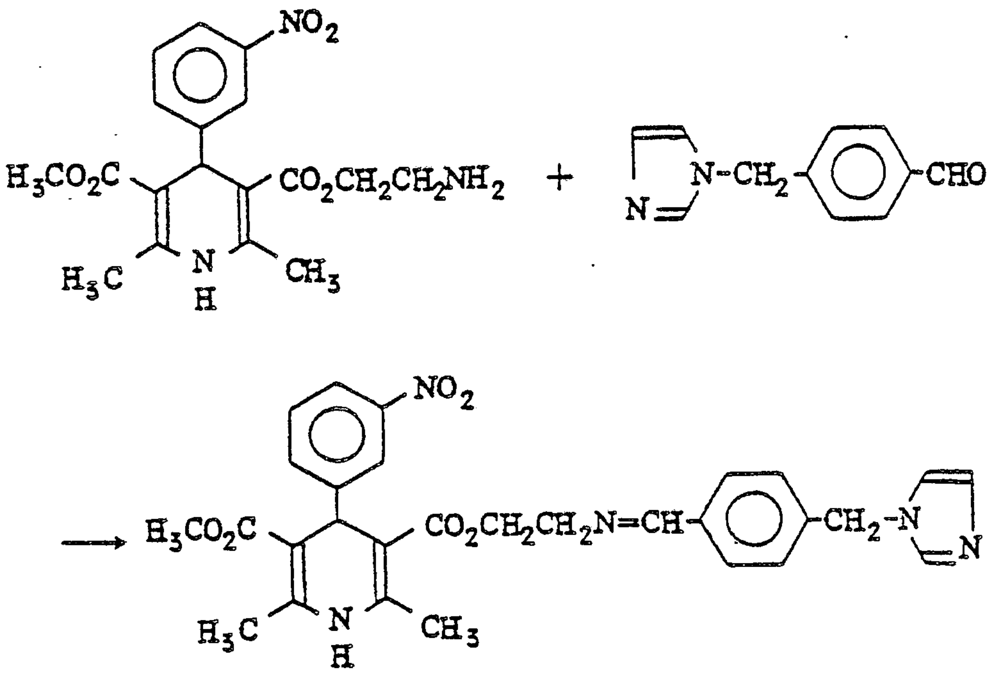

506 mg (1.35 mM) of 2-aminoethyl methyl 1,4-dihydro-2,6-dimethyl-4-(3-nitrophenyl)pyridine-3,5-dicarboxylate was mixed with 251 mg (1.35 mM) of 4-(1-imidazolylmethyl)benzaldehyde and the mixture was stirred for 15 hours at room temperatures to obtain 544 mg of the captioned compound. Yield: 100% of theory

Melting point (°C) 153 (dec.)

NMR $\delta CDCl_3$ 2.32 (s, 3H), 2.33 (s, 3H), 3.55 (s, 3H), 3.80 (t, 2H, J=5Hz), 4.32-4.47 (m, 2H), 5.05 (s, 1H), 5.11 (s, 2H), 5.93 (s, 1H), 6.93 (s, 1H), 7.11 (s, 1H), 7.18 (d, 2H, J=9Hz), 7.24 (t, 1H, J=9Hz), 7.57 (dt, 1H, J=9Hz, 2Hz), 7.66 (d, 2H, J=9Hz), 7.88 (dt, 1H, J=9Hz, 2Hz), 8.23 (t, 1H, J=2Hz), 8.20 (s, 1H)

IR ($cm^{-1}$) $\nu co$ 1698

$\nu no_2$ 1530, 1348

High mass Molecular formula $C_{29}H_{29}N_5O_6$

| | |
|---|---|
| Found | 543.21105 |
| Calcd. | 543.21175 |

Control experiments of the derivatives of the invention were carried out as follows:

1. Experiment for hypotensive activity:

Male spontaneously hypertensive rats (SHR) were used. An arterial blood pressure in conscious SHR was measured through the catheterized abdominal aorta by a pressure transducer (sold by Nihon Kohden K.K. under the tradename of MPU-0.5). Test compounds and reference compounds were injected into the caudal vein. NIFEDIPINE and NICARDIPINE were used as controls. The results are shown in Table 1.

2. Experiment for inhibitory activity on KCl-induced contraction of aorta:

Thoracic aortas were isolated from male rabbits and cut into helical strips. The preparations were mounted vertically in organ baths containing 30 ml of Krebs- Henseleit solution. The tissue solution was maintained at 37° C and bubbled with a mixture of 95% $O_2$ and 5% $CO_2$. After 90 minutes equilibration, a contraction induced by the application cf 30 mM KCl was measured isometrically. Test and reference compounds were added to the bath 30 minutes after KCl treatment. The inhibitory effects of test and reference compounds were determined 60 minutes later. The results are shown in Table 1.

Table 1

| Derivatives of the Invention Example No. | Hypotensive effect - Max 100μg (mm Hg) | Inhibitory effect on KCl-induced contraction 60min $10^{-6}$M (%) |
|---|---|---|
| 1 | 28.8 | 41.1 |
| 2 | 50.0 | 39.7 |
| 3 | 27.5 | 35.3 |
| 4 | 62.5 | 76.4 |
| 5 | 37.5 | 69.4 |
| 6 | 20.8 | 50.0 |
| 7 | 32.5 | 39.5 |
| 8 | 30.0 | 64.0 |
| 9 | 5.0 | 48.2 |
| 10 | 41 | 30.0 |
| 11 | 20 | 30.0 |
| 12 | 40 | 64.5 |
| 13 | 25.7 | 86.7 |

Table 1 (Cont'd)

| Derivatives of the Invention Example No. | Hypotensive effect - Max 100μg (mm Hg) | Inhibitory effect on KCl-induced contraction 60min $10^{-6}$M (%) |
|---|---|---|
| 14 | 36.0 | 25.6 |
| 15 | 25.0 | 21.6 |
| 16 | 34.0 | 27.3 |
| 17 | 30.0 | 55.1 |
| 18 | 22.5 | 88.2 |
| 19 | 25.0 | 68.8 |
| 20 | 37.5 | 68.8 |
| 21 | 15.0 | 37.8 |
| 22 | 38.0 | 11.0 |
| 23 | 44.0 | 21.6 |
| 24 | 35.0 | 74.3 |
| 25 | 12.5 | 88.3 |
| 26 | 30.0 | 74.8 |
| NIFEDIPINE | 55 (30μg) | 87.6 |
| NICARDIPINE | 100 (30μg) | 91.1 |

3. Experiment for inhibitory activity on platelet aggregation in rabbit:

Rabbit platelet rich plasma (PRP) was prepared by centrifugation of the citrated blood at 380 g for 10 minutes at 20°C. Test solution (10 μl) was added to PRP (200 μl) 30 minutes before treatment of aggregation agents (10 μl). The extent of aggregation was expressed with the maximum change in light transmission for PRP and platelet poor plasma was taken as a value of 100%

Aggregating agents (final concentration):

| | |
|---|---|
| Arachidonic acid (AA) | 1 mM |
| Adenosine diphosphate (ADP) | 5 mM |
| Platelet activating factor (PAF) | 10 mg/ml |

The results are shown in Table 2.

Table 2

| Derivatives of the Invention Example No. | Collagen $10^{-4}$ (%) | AA $10^{-4}$ (%) | ADP $10^{-4}$ (%) | PAF $3x10^{-6}$ (%) |
|---|---|---|---|---|
| 1 | 19.5 | 26.2 | 7.3 | 35.4 |
| 2 | 23.5 | 12.6 | 5.5 | 0 |
| 3 | 33.2 | 19.3 | 2.2 | 0 |
| 4 | 30.5 | 30.8 | - 2.2 | 95.6 |
| 5 | 32.3 | 33.4 | 12.0 | 100 |
| 6 | 27.2 | 15.6 | 6.5 | 0 |
| 7 | 30.9 | 17.8 | 4.3 | 0 |
| 8 | 18.9 | 18.8 | 10.8 | 28.9 |
| 9 | 11.1 | 5.9 | 6.5 | 78.8 |

Table 2 (Cont'd)

| Derivatives of the Invention Example No. | Collagen $10^{-4}$ (%) | AA $10^{-4}$ (%) | ADP $10^{-4}$ (%) | PAF $3x10^{-6}$ (%) |
|---|---|---|---|---|
| 10 | 5.5 | 5.0 | 2.1 | 24.4 |
| 11 | 7.1 | 2.9 | 1.3 | 31.1 |
| 12 | 10.3 | 11.4 | 7.3 | 51.8 |
| 13 | 6.3 | 17.4 | 10.9 | 49.6 |
| 14 | 69.6 | 42.0 | 4.3 | 44.7 |
| 15 | 10.5 | 30.2 | 8.8 | 74.1 |
| 16 | 24.7 | 33.7 | 7.1 | 42.4 |
| 17 | 55.0 | 37.8 | 10.5 | 39.7 |
| 18 | 61.8 | 30.4 | 8.8 | 48.6 |
| 19 | 66.3 | 35.5 | 17.2 | 41.5 |
| 20 | 32.3 | 30.4 | 9.5 | 0 |
| 21 | 29.5 | 37.6 | 17.3 | 25.4 |
| 22 | 24.7 | 23.2 | 14.7 | 0 |
| 23 | 29.5 | 25.7 | 2.6 | 0 |
| 24 | 27.5 | 28.2 | 7.8 | 18.3 |
| 25 | 22.5 | 27.8 | 0 | 28.5 |
| 26 | 13.5 | 27.6 | 6.0 | 30.0 |
| NIFEDIPINE | 5.1 | 0.2 | 0 | |
| NICARDIPINE | 3.6 | | 6.9 | |

As can be seen from the results of the experiments, 1,4-dihydropyridine derivatives of the present invention have inhibitory activity on KCl-induced contraction of aorta and inhibitory activity on platelet aggregation together with hypotensive activity.

**Claims**

1. 1,4-Dihydropyridine derivatives of the formula (I):

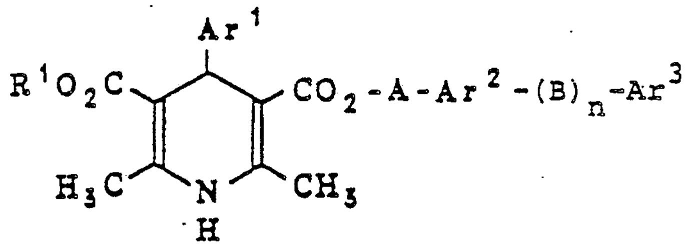

... (I)

wherein $Ar^1$ represents an aromatic hydrocarbon group or heterocyclic group which may have at least one substituent; $R^1$ represents a hydrocarbon group which may have at least one substituent; A and B each represent a hydrocarbon group which may have at least one substituent, in which any of the carbon atoms in the main chain thereof may be replaced by a nitrogen atom or an oxygen atom; $Ar^2$ represents an aromatic hydrocarbon group or heterocyclic group; $Ar^3$ represents a heterocyclic group which may have at least one substituent; and n is 0 or 1.

2. The 1,4-dihydropyridine derivatives as claimed in Claim 1, wherein said aromatic hydrocarbon group represented by $Ar^1$ is selected from the group consisting of a phenyl group and a naphthyl group.

3. The 1,4-dihydropyridine derivatives as claimed in Claim 4, wherein said aromatic group represented by $Ar^1$ is a phenyl group.

4. The 1,4-dihydropyridine derivatives as claimed in Claim 1, wherein said heterocyclic group represented by $Ar^1$ is selected from the group consisting of a pyridyl group, a benzoxazolyl group, a quinolyl group, a furyl group, a thienyl group, an isoquinolyl, a benzthiazolyl group, a benzoxadiazolyl group and a benzthiadizolyl group.

5. The 1,4-dihydropyridine derivatives as claimed in Claim 4, wherein said heterocyclic group represented by $Ar^1$ is selected from the group consisting of a pyridyl group, a benzoxazolyl group, a quinolyl group, a furyl group, and a thienyl group.

6. The 1,4-dihydropyridine derivatives as claimed in Claim 1, wherein the substituent of said aromatic hydrocarbon group or heterocyclic group represented by $Ar^1$ is selected from the group consisting of a nitro group, a trihalomethyl group, a cyano group, a halogen, a phenoxy group, an azido group, an amido group, a lower alkoxyl group having 1 to 4 carbon atoms, a lower alkyl group having 1 to 4 carbon atoms, a lower akylthio group, a lower alkoxy carbonyl group, and a lower akylcarbonyl group.

7. The 1,4-dihydropyridine derivatives as claimed in Claim 6, wherein the substituent of said aromatic hydrocarbon group or heterocyclic group represented by $Ar^1$ is selected from the group consisting of a nitro group, trihalomethyl group, a cyano group, a halogen, and a phenoxy group.

8. The 1,4-dihydropyridine derivatives as claimed in Claim 1, wherein said aromatic hydrocarbon group or heterocyclic group represented by $Ar^1$ is selected from the group consisting of 2-nitrophenyl group, 3-nitrophenyl group, 4-nitrophenyl group, 2-cyanophenyl group, 2,3-dichlorophenyl group, 2-furyl group, 2-thienyl group, 3-thienyl group, 1-naphthyl group, 4-quinolyl group, 3-azidophenyl group, 3-trifluoromethylphenyl group, 4-methoxyphenyl group, 4-methylthiophenyl group, 3-pyridyl group, 4-methylphenyl group and 3-trichloromethylphenyl group.

9. The 1,4-dihydroxypyridine derivatives as claimed in Claim 1, wherein said hydrocarbon group represented by $R^1$ is a straight, branched or cyclic saturated or unsaturated hydrocarbon group having 1 to 6 carbon atoms.

10. The 1,4-dihydroxypyridine derivatives as claimed in Claim 1, wherein said hydrocarbon group represented by $R^1$ is a straight, branched or cyclic saturated or unsaturated hydrocarbon group having 1 to 6 carbon atoms, containing therein an atom selected from the group consisting of oxygen, sulfur and nitrogen.

11. The 1,4-dihydroxypyridine derivatives as claimed in Claim 10, wherein said hydrocarbon group represented by $R^1$ is an alkoxyalkyl group.

12. The 1,4-dihydroxypyridine derivatives as claimed in Claim 1, wherein the substituent of said hydrocrabon group represented by $R^1$ is selected from the group consisting of a halogen, a cyano group, a nitro group, a nitrato group, a hydroxyl group, a phenoxy group, a disubstituted amino group, a phenylthio group, piperidino group, pyrrolidino group, a substituted piperazinyl group, a morpholino group, and a phenyl group, said phenyl group may further have one or two substituents selected from the group

consisting of a halogen, a cyano group, a trihalomethyl group, an azido group, a disubstituted amino group, a lower alkyl group having 1 to 4 carbon atoms, a lower alkoxyl group having 1 to 4 carbon atoms, a lower alkylthio group, a lower alkylcarbonyl group, a lower alkoxycarbonyl group, a carbamoyl group, a halomethyl group, and a hydroxymethyl group.

13. The 1,4-dihydroxypyridine derivatives as claimed in Claim 12, wherein the substituent of said hydrocrabon group represented by $R^1$ is a disubstituted amino group.

14. The 1,4-dihydroxypyridine derivatives as claimed in Claim 1, wherein said hydrocrabon group represented by $R^1$ is a group represented by $-A-Ar^2-(B)_n-Ar^3$.

15. The 1,4-dihydroxypyridine derivatives as claimed in Claim 9, wherein said hydrocrabon group represented by $R^1$ is selected from the group consisting of a methyl group, an ethyl group, a propyl group, a n-butyl group, a n-butyl group, a n-pentyl group, a n-hexyl group, an isopropyl group, an isobutyl group, a cyclopentyl group, a cyclohexyl group, a propenyl group, a 2-butenyl group, 3-butenyl group, a 2-pentynyl group, a 2,4-hexadienyl group, a 2,4-hexadinyly a hexa-2-yn-4-ene group and a hexa-2-en-4-yne group.

16. The 1,4-dihydroxypyridine derivatives as claimed in claim 10, wherein said hydrocarbon group represented by $R^1$ is selected from the group consisting of a methoxyethyl group, an ethoxyethyl group, a methoxypropyl group, an ethoxypropyl group, a methylthioethyl group, an ethylthioethyl group, a methylthiopropyl group, an ethylthiopropyl group, a phenylthioethyl, 2-(N-methylamino)ethyl group, 2-(N,N-dimethylamino)ethyl group, 2-(N-phenyl-N-methylamino) ethyl group, 2-(N-benzyl-N-methylamino)ethyl group, 2-(4-phenylpypiperazin-1-yl)ethyl, 2-{4-(diphenylmethyl)piperazinyl}ethyl, 2-morpholinoethyl group, N-benzylpyrrolidin-3-yl, and N-benzylpiperizin-3-yl.

17. The 1,4-dihydroxypyridine derivatives as claimed in Claim 1, wherein said hydrocarbon group represented by A is a straight, branched or cyclic unsaturated hydrocarbon group in which at least an unsaturated bond thereof is conjugated with said aromatic group hydrocarbon or heterocyclic group represented by $Ar^2$.

18. The 1,4-dihydroxypyridine derivatives as claimed in Claim 17, wherein said hydrocarbon group represented by A is a straight or branched unsaturated hydrocarbon group having 3 to 12 carbon atoms, which may have a substituent selected from the group consisting of a halogen, a phenyl group, a pyridyl group, a thienyl group, a furyl group, a cyano group, an alkylcarbonyl group, and an alkoxylcarbonyl group, and which may contain in the main chain thereof a group selected from the group consisting of $-O-N=CH-$, $-N=N-$, $-N=CH-$, and $-CH=N-$.

19. The 1,4-dihydroxypyridine derivatives as claimed in Claim 17, wherein said hydrocarbon group represented by A is a cyclic unsaturated hydrocarbon group having 4 to 12 carbon atoms, which may have a substituent selected from the group consisting of a halogen, a phenyl group, a pyridyl group, a thienyl group, a furyl group, a cyano group, an alkylcarbonyl group, and alkoxylcarbonyly group, and which may contain in the main chain thereof a group selected from the group consisting of $-O-N=CH-$, $-N=N-$, $-N=CH-$, and $-CH=N-$.

20. The 1,4-dihydroxypyridine derivatives as claimed in claim 18, wherein said straight or branched unsaturated hydrocarbon group represented by A is selected from the group consisting of:

$$-CH_2-CH{=}CH- \quad , \quad -CH_2\!\left(CH{=}CH\right)_2 \quad ,$$

$$-CH_2-C{\equiv}C- \quad , \quad -CH_2\!\left(C{\equiv}C\right)_2 \quad , \quad -CH_2\!\left(CH{=}CH\right)_3 \quad ,$$

$$-\overset{\displaystyle CH_3}{\overset{|}{CH}}-CH{=}CH- \quad , \quad -\overset{\displaystyle CH_3}{\overset{|}{CH}}\!\left(CH{=}CH\right)_2 \quad , \quad -\overset{\displaystyle CH_3}{\overset{|}{CH}}\!\left(CH{=}CH\right)_3 \quad ,$$

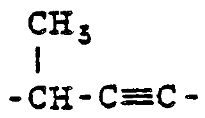 , 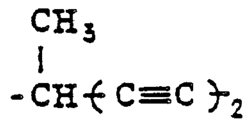 , 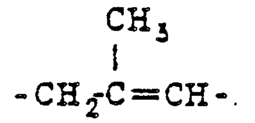 ,

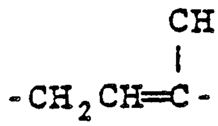 , 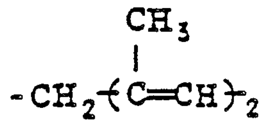 , 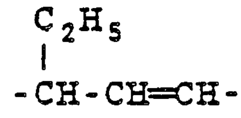 ,

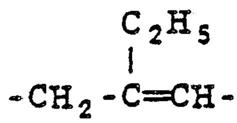 , 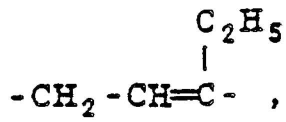 , 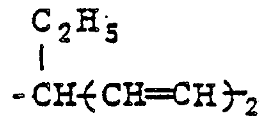 ,

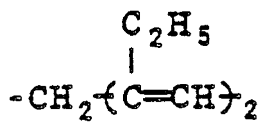 , $-CH_2-CF{=}CH-$ , 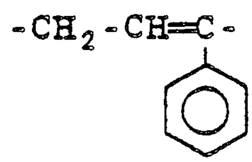 ,

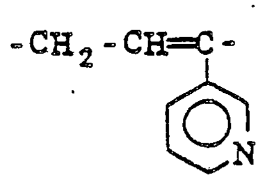 , $-CH_2-CH{=}CH-C{\equiv}C-$ ,

$-CH_2CH_2-CH{=}CH-$ , $-CH_2CH_2-O-N{=}CH-$ ,

$-CH_2CH_2-N{=}CH-$ , $-CH_2CH_2CH{=}N-$ , $-CH_2CH_2-N{=}N-$ , and

$-CH_2-C{\equiv}C-CH{=}CH-$ ,

21. The 1,4-dihydroxypyridine derivatives as claimed in Claim 1, wherein said aromatic group or heterocyclic group represented by $Ar^2$ is a group selected from the group consisting of o-, m- or p-phenylene group, a furandiyl group, a thiophenediyl group, a pyridinediyl group and a pyrroledinyl group, which may further form a 5- or 6-membered condensed ring in combination with said hydrocarbon group represented by A or a part thereof through a member selected from the group consisting of a methylene group, an ethylene group, a vinyl group, oxygen, sulfur and an amino group.

22. The 1,4-dihydroxypyridine derivatives as claimed in Claim 1, wherein said aromatic group or heterocyclic group represented by $Ar^2$ is selected from the group consisting of:

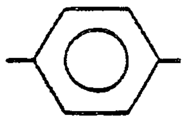 , 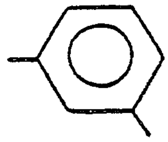 , 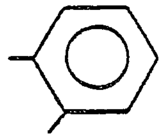 , 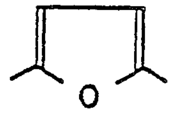 ,

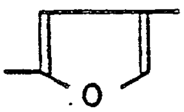 , 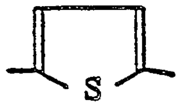 , 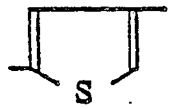 , 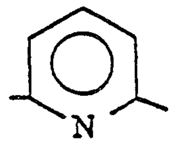 ,

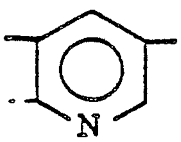 , 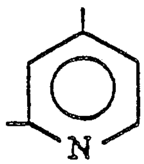 , 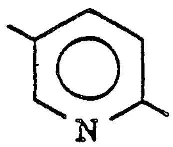 , 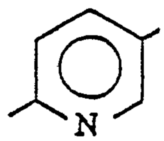 ,

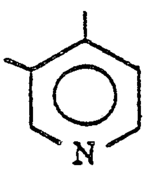 , 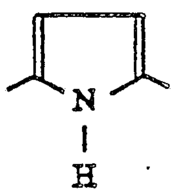 , 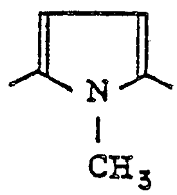 ,

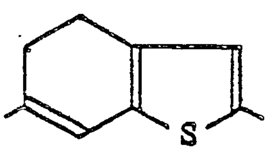 , 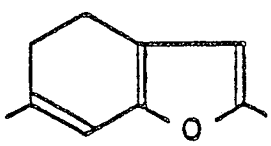 , 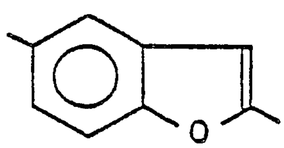 ,

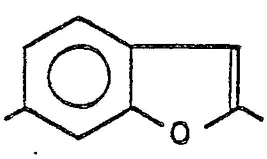 , 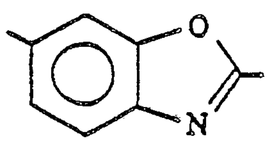 , 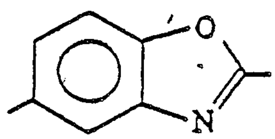 , and

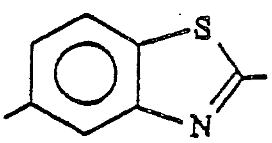 , 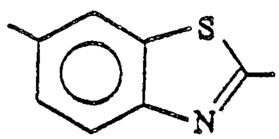 ,

23. The 1,4-dihydroxy pyridine derivatives as claimed in Claim 1, wherein when n is 1, said hydrocarbon group represented by B is an alkylene or alkenylene group having 1 to 3 carbon atoms, which may have a substituent selected from the group consisting of a lower alkyl group having 1 to 4 carbon atoms, an cyclic alkyl group, an aryl group, and an aralkyl group.

24. The 1,4-dihydroxypyridine derivatives as claimed in Claim 23, wherein said alkylene or alkenylene group is selected from the group consisting of a methylene group, an ethylene group, a trimethylene group, an ethylidene group, a benzylidene group a vinylene group, a cylcohexylmethylene group and a 2-phenylethylidene group.

25. The 1,4-dihydroxypyridine derivatives as claimed in Claim 1, wherein said heterocyclic group represented by $Ar^3$ is a 5- to 6-membered heterocyclic ring including at least an atom selected from the group consisting of nitrogen, oxygen and sulfur.

26. The 1,4-dihydroxypyridine derivatives as claimed in Claim 25, wherein said 5- to 6-membered aromatic heterocyclic ring is selected from the group consisting of a 2-pyrrolyl group, a 2-pyridyl group, a 3-pyridyl group, a 4-pyridyl group, an imidazolyl group, a 2-oxazolyl group, 4-oxazolyl group, a 2-thiazolyl group, a triazolyl group, and a tetrazolyl group.

27. The 1,4-dihydroxypyridine derivatives as claimed in Claim 1, wherein said hydrocarbon group represented by B or a part thereof forms a 5- or 6-membered ring in combination with said aromatic hydrocarbon group or heterocyclic group represented by $Ar^2$ or with said heterocyclic group represented by $Ar^3$ through a member selected from the group consisting of a methylene group, an ethylene group, and a vinylene group.

28. The 1,4-dihydroxypyridine derivatives as claimed in Claim 27, wherein said 5- or 6-membered ring is selected from the group consisting of:

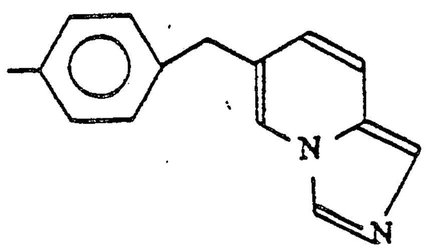 , 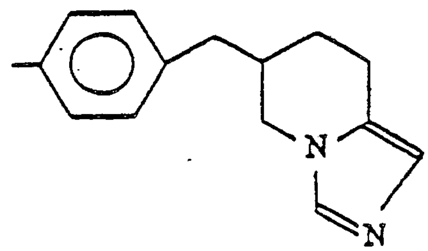 ,